# EUROPEAN PATENT APPLICATION

(11) **EP 1 743 945 A1**
(43) Date of publication of application: **17.01.2007**
(21) Application number: 05736544.7
(22) Date of filing: 26.04.2005
(51) Int. Cl.: C12Q 1/48, A61K 31/7088, A61K 38/00, A61K 38/43, A61K 38/45, A61K 38/55

(54) **SCREENING METHOD**

(30) Priority: 27.04.2004 JP 2004131731
(71) Applicant: Takeda Pharmaceutical Company Limited, Osaka-shi, Osaka 541-0045 (JP)
(72) Inventor: HAZAMA, Masatoshi, TAKEDA PHARM. COMPANY LIMITED, Osaka-shi, Osaka, 5328686 (JP); FUJITANI, Yasushi, TAKEDA PHARM. COMPANY LIMITED, Osaka-shi, Osaka, 5328686 (JP); ICHIJO, Hidenori, Tokyo 1120002 (JP)
(74) Representative: Helbing, Jörg
(86) International application number: PCT/JP2005/008375
(87) International publication number: WO 2005/103288

(57) **Abstract**

The present invention provides a method for screening for a prophylactic or therapeutic drug for apoptosis or an inflammation-associated disease, diabetes and complications thereof or a chronic obstructive pulmonary disease (COPD), namely, a method for screening for a prophylactic or therapeutic drug for apoptosis or an inflammation-associated disease, diabetes and complications thereof or COPD, which includes (1) comparing the activities of a protein having an amino acid sequence the same as or substantially the same as the amino acid sequence shown by SEQ ID NO: 2 or a partial peptide thereof or a salt thereof, between in the presence of a test substance and in the absence of the test substance, or (2) comparing the expressions of a protein having an amino acid sequence the same as or substantially the same as the amino acid sequence shown by SEQ ID NO: 2, a partial peptide thereof and a salt thereof in the cells having the ability to produce the protein, the partial peptide thereof and the salt thereof, between in the presence of a test substance and in the absence of the test substance.

SEQUENCE LISTING

<110>  Takeda Pharmaceutical Company Limited

<120>  Screening Method

<130>  09762

<150>  JP 2004-131731
<151>  2004-04-27

<160>  2

<170>  PatentIn version 3.2

<210>  1
<211>  4125
<212>  DNA
<213>  Homo sapiens


<220>
<221>  CDS
<222>  (1)..(4125)

<400>  1
atg agc acg gag gcg gac gag ggc atc act ttc tct gtg cca ccc ttc       48
Met Ser Thr Glu Ala Asp Glu Gly Ile Thr Phe Ser Val Pro Pro Phe
1               5                   10                  15

gcc ccc tcg ggc ttc tgc acc atc ccc gag ggc ggc atc tgc agg agg       96
Ala Pro Ser Gly Phe Cys Thr Ile Pro Glu Gly Gly Ile Cys Arg Arg
            20                  25                  30

gga gga gcg gcg gcg gtg ggc gag ggc gag gag cac cag ctg cca ccg      144
Gly Gly Ala Ala Ala Val Gly Glu Gly Glu Glu His Gln Leu Pro Pro
        35                  40                  45

ccg ccg ccg ggc agt ttc tgg aac gtg gag agc gcc gct gcc cct ggc      192
Pro Pro Pro Gly Ser Phe Trp Asn Val Glu Ser Ala Ala Ala Pro Gly
    50                  55                  60

atc ggt tgt ccg gcg gcc acc tcc tcg agc agt gcc acc cga ggc cgg      240
Ile Gly Cys Pro Ala Ala Thr Ser Ser Ser Ser Ala Thr Arg Gly Arg
65                  70                  75                  80

ggc agc tct gtt ggc ggg ggc agc cga cgg acc acg gtg gca tat gtg      288
Gly Ser Ser Val Gly Gly Gly Ser Arg Arg Thr Thr Val Ala Tyr Val
                85                  90                  95

atc aac gaa gcg agc caa ggg caa ctg gtg gtg gcc gag agc gag gcc      336
Ile Asn Glu Ala Ser Gln Gly Gln Leu Val Val Ala Glu Ser Glu Ala
            100                 105                 110

ctg cag agc ttg cgg gag gcg tgc gag aca gtg ggc gcc acc ctg gaa      384
Leu Gln Ser Leu Arg Glu Ala Cys Glu Thr Val Gly Ala Thr Leu Glu
        115                 120                 125

acc ctg cat ttt ggg aaa ctc gac ttt gga gaa acc acc gtg ctg gac      432
Thr Leu His Phe Gly Lys Leu Asp Phe Gly Glu Thr Thr Val Leu Asp
    130                 135                 140

cgc ttt tac aat gca gat att gcg gtg gtg gag atg agc gat gcc ttc      480

Arg Phe Tyr Asn Ala Asp Ile Ala Val Val Glu Met Ser Asp Ala Phe
145                 150                 155                 160

cgg cag ccg tcc ttg ttt tac cac ctt ggg gtg aga gaa agt ttc agc      528
Arg Gln Pro Ser Leu Phe Tyr His Leu Gly Val Arg Glu Ser Phe Ser
                165                 170                 175

atg gcc aac aac atc atc ctc tac tgc gat act aac tcg gac tct ctg      576
Met Ala Asn Asn Ile Ile Leu Tyr Cys Asp Thr Asn Ser Asp Ser Leu
            180                 185                 190

cag tca ctg aag gaa atc att tgc cag aag aat act atg tgc act ggg      624
Gln Ser Leu Lys Glu Ile Ile Cys Gln Lys Asn Thr Met Cys Thr Gly
        195                 200                 205

aac tac acc ttt gtt cct tac atg ata act cca cat aac aaa gtc tac      672
Asn Tyr Thr Phe Val Pro Tyr Met Ile Thr Pro His Asn Lys Val Tyr
    210                 215                 220

tgc tgt gac agc agc ttc atg aag ggg ttg aca gag ctc atg caa ccg      720
Cys Cys Asp Ser Ser Phe Met Lys Gly Leu Thr Glu Leu Met Gln Pro
225                 230                 235                 240

aac ttc gag ctg ctt ctt gga ccc atc tgc tta cct ctt gtg gat cgt      768
Asn Phe Glu Leu Leu Leu Gly Pro Ile Cys Leu Pro Leu Val Asp Arg
                245                 250                 255

ttt att caa ctt ttg aag gtg gca caa gca agt tct agc cag tac ttc      816
Phe Ile Gln Leu Leu Lys Val Ala Gln Ala Ser Ser Ser Gln Tyr Phe
            260                 265                 270

cgg gaa tct ata ctc aat gac atc agg aaa gct cgt aat tta tac act      864
Arg Glu Ser Ile Leu Asn Asp Ile Arg Lys Ala Arg Asn Leu Tyr Thr
        275                 280                 285

ggt aaa gaa ttg gca gct gag ttg gca aga att cgg cag cga gta gat      912
Gly Lys Glu Leu Ala Ala Glu Leu Ala Arg Ile Arg Gln Arg Val Asp
    290                 295                 300

aat atc gaa gtc ttg aca gca gat att gtc ata aat ctg tta ctt tcc      960
Asn Ile Glu Val Leu Thr Ala Asp Ile Val Ile Asn Leu Leu Leu Ser
305                 310                 315                 320

tac aga gat atc cag gac tat gat tct att gtg aag ctg gta gag act     1008
Tyr Arg Asp Ile Gln Asp Tyr Asp Ser Ile Val Lys Leu Val Glu Thr
                325                 330                 335

tta gaa aaa ctg cca acc ttt gat ttg gcc tcc cat cac cat gtg aag     1056
Leu Glu Lys Leu Pro Thr Phe Asp Leu Ala Ser His His His Val Lys
            340                 345                 350

ttt cat tat gca ttt gca ctg aat agg aga aat ctc cct ggt gac aga     1104
Phe His Tyr Ala Phe Ala Leu Asn Arg Arg Asn Leu Pro Gly Asp Arg
        355                 360                 365

gca aaa gct ctt gat att atg att ccc atg gtg caa agc gaa gga caa     1152
Ala Lys Ala Leu Asp Ile Met Ile Pro Met Val Gln Ser Glu Gly Gln
    370                 375                 380

gtt gct tca gat atg tat tgc cta gtt ggt cga atc tac aaa gat atg     1200
Val Ala Ser Asp Met Tyr Cys Leu Val Gly Arg Ile Tyr Lys Asp Met
385                 390                 395                 400

ttt ttg gac tct aat ttc acg gac act gaa agc aga gac cat gga gct      1248
Phe Leu Asp Ser Asn Phe Thr Asp Thr Glu Ser Arg Asp His Gly Ala
                405                 410                 415

tct tgg ttc aaa aag gca ttt gaa tct gag cca aca cta cag tca gga      1296
Ser Trp Phe Lys Lys Ala Phe Glu Ser Glu Pro Thr Leu Gln Ser Gly
            420                 425                 430

att aat tat gcg gtc ctc ctc ctg gca gct gga cac cag ttt gaa tct      1344
Ile Asn Tyr Ala Val Leu Leu Leu Ala Ala Gly His Gln Phe Glu Ser
        435                 440                 445

tcc ttt gag ctc cgg aaa gtt ggg gtg aag cta agt agt ctt ctt ggt      1392
Ser Phe Glu Leu Arg Lys Val Gly Val Lys Leu Ser Ser Leu Leu Gly
    450                 455                 460

aaa aag gga aac ttg gaa aaa ctc cag agc tac tgg gaa gtt gga ttt      1440
Lys Lys Gly Asn Leu Glu Lys Leu Gln Ser Tyr Trp Glu Val Gly Phe
465                 470                 475                 480

ttt ctg ggg gcc agc gtc cta gcc aat gac cac atg aga gtc att caa      1488
Phe Leu Gly Ala Ser Val Leu Ala Asn Asp His Met Arg Val Ile Gln
                485                 490                 495

gca tct gaa aag ctt ttt aaa ctg aag aca cca gca tgg tac ctc aag      1536
Ala Ser Glu Lys Leu Phe Lys Leu Lys Thr Pro Ala Trp Tyr Leu Lys
            500                 505                 510

tct att gta gag aca att ttg ata tat aag cat ttt gtg aaa ctg acc      1584
Ser Ile Val Glu Thr Ile Leu Ile Tyr Lys His Phe Val Lys Leu Thr
        515                 520                 525

aca gaa cag cct gtg gcc aag caa gaa ctt gtg gac ttt tgg atg gat      1632
Thr Glu Gln Pro Val Ala Lys Gln Glu Leu Val Asp Phe Trp Met Asp
    530                 535                 540

ttc ctg gtc gag gcc aca aag aca gat gtt act gtg gtt agg ttt cca      1680
Phe Leu Val Glu Ala Thr Lys Thr Asp Val Thr Val Val Arg Phe Pro
545                 550                 555                 560

gta tta ata tta gaa cca acc aaa atc tat caa cct tct tat ttg tct      1728
Val Leu Ile Leu Glu Pro Thr Lys Ile Tyr Gln Pro Ser Tyr Leu Ser
                565                 570                 575

atc aac aat gaa gtt gag gaa aag aca atc tct att tgg cac gtg ctt      1776
Ile Asn Asn Glu Val Glu Glu Lys Thr Ile Ser Ile Trp His Val Leu
            580                 585                 590

cct gat gac aag aaa ggt ata cat gag tgg aat ttt agt gcc tct tct      1824
Pro Asp Asp Lys Lys Gly Ile His Glu Trp Asn Phe Ser Ala Ser Ser
        595                 600                 605

gtc agg gga gtg agt att tct aaa ttt gaa gaa aga tgc tgc ttt ctt      1872
Val Arg Gly Val Ser Ile Ser Lys Phe Glu Glu Arg Cys Cys Phe Leu
    610                 615                 620

tat gtg ctt cac aat tct gat gat ttc caa atc tat ttc tgt aca gaa      1920
Tyr Val Leu His Asn Ser Asp Asp Phe Gln Ile Tyr Phe Cys Thr Glu
625                 630                 635                 640

ctt cat tgt aaa aag ttt ttt gag atg gtg aac acc att acc gaa gag      1968
Leu His Cys Lys Lys Phe Phe Glu Met Val Asn Thr Ile Thr Glu Glu
                645                 650                 655

aag ggg aga agc aca gag gaa gga gac tgt gaa agt gac ttg ctg gag     2016
Lys Gly Arg Ser Thr Glu Glu Gly Asp Cys Glu Ser Asp Leu Leu Glu
            660                 665                 670

tat gac tat gaa tat gat gaa aat ggt gac aga gtc gtt tta gga aaa     2064
Tyr Asp Tyr Glu Tyr Asp Glu Asn Gly Asp Arg Val Val Leu Gly Lys
        675                 680                 685

ggc act tat ggg ata gtc tac gca ggt cgg gac ttg agc aac caa gtc     2112
Gly Thr Tyr Gly Ile Val Tyr Ala Gly Arg Asp Leu Ser Asn Gln Val
    690                 695                 700

aga att gct att aag gaa atc cca gag aga gac agc aga tac tct cag     2160
Arg Ile Ala Ile Lys Glu Ile Pro Glu Arg Asp Ser Arg Tyr Ser Gln
705                 710                 715                 720

ccc ctg cat gaa gaa ata gca ttg cat aaa cac ctg aag cac aaa aat     2208
Pro Leu His Glu Glu Ile Ala Leu His Lys His Leu Lys His Lys Asn
                725                 730                 735

att gtc cag tat ctg ggc tct ttc agt gag aat ggt ttc att aaa atc     2256
Ile Val Gln Tyr Leu Gly Ser Phe Ser Glu Asn Gly Phe Ile Lys Ile
            740                 745                 750

ttc atg gag cag gtc cct gga gga agt ctt tat gct ctc ctt cgt tcc     2304
Phe Met Glu Gln Val Pro Gly Gly Ser Leu Tyr Ala Leu Leu Arg Ser
        755                 760                 765

aaa tgg ggt cca tta aaa gac aat gag caa aca att ggc ttt tat aca     2352
Lys Trp Gly Pro Leu Lys Asp Asn Glu Gln Thr Ile Gly Phe Tyr Thr
    770                 775                 780

aag caa ata ctg gaa gga tta aaa tat ctc cat gac aat cag ata gtt     2400
Lys Gln Ile Leu Glu Gly Leu Lys Tyr Leu His Asp Asn Gln Ile Val
785                 790                 795                 800

cac cgg gac ata aag ggt gac aat gtg ttg att aat acc tac agt ggt     2448
His Arg Asp Ile Lys Gly Asp Asn Val Leu Ile Asn Thr Tyr Ser Gly
                805                 810                 815

gtt ctc aag atc tct gac ttc gga aca tca aag agg ctt gct ggc ata     2496
Val Leu Lys Ile Ser Asp Phe Gly Thr Ser Lys Arg Leu Ala Gly Ile
            820                 825                 830

aac ccc tgt act gaa act ttt act ggt acc ctc cag tat atg gca cca     2544
Asn Pro Cys Thr Glu Thr Phe Thr Gly Thr Leu Gln Tyr Met Ala Pro
        835                 840                 845

gaa ata ata gat aaa gga cca aga ggc tac gga aaa gca gca gac atc     2592
Glu Ile Ile Asp Lys Gly Pro Arg Gly Tyr Gly Lys Ala Ala Asp Ile
    850                 855                 860

tgg tct ctg ggc tgt aca atc att gaa atg gcc aca gga aaa ccc cca     2640
Trp Ser Leu Gly Cys Thr Ile Ile Glu Met Ala Thr Gly Lys Pro Pro
865                 870                 875                 880

ttt tat gaa ctg gga gaa cca caa gca gct atg ttc aag gtg gga atg     2688
Phe Tyr Glu Leu Gly Glu Pro Gln Ala Ala Met Phe Lys Val Gly Met
                885                 890                 895

ttt aaa gtc cac cct gag atc cca gag tcc atg tct gca gag gcc aag     2736
Phe Lys Val His Pro Glu Ile Pro Glu Ser Met Ser Ala Glu Ala Lys

900                 905                 910

gca ttc ata ctg aaa tgt ttt gaa cca gat cct gac aag aga gcc tgt      2784
Ala Phe Ile Leu Lys Cys Phe Glu Pro Asp Pro Asp Lys Arg Ala Cys
        915                 920                 925

gct aac gac ttg ctt gtt gat gag ttt tta aaa gtt tca agc aaa aag      2832
Ala Asn Asp Leu Leu Val Asp Glu Phe Leu Lys Val Ser Ser Lys Lys
    930                 935                 940

aaa aag aca caa cct aag ctt tca gct ctt tca gct gga tca aat gca      2880
Lys Lys Thr Gln Pro Lys Leu Ser Ala Leu Ser Ala Gly Ser Asn Ala
945                 950                 955                 960

gaa tat ctc agg agt ata tcc ttg ccg gta cct gtg ctg gtg gag gac      2928
Glu Tyr Leu Arg Ser Ile Ser Leu Pro Val Pro Val Leu Val Glu Asp
                965                 970                 975

acc agc agc agc agt gag tac ggc tca gtt tca ccc gac acg gag ttg      2976
Thr Ser Ser Ser Ser Glu Tyr Gly Ser Val Ser Pro Asp Thr Glu Leu
            980                 985                 990

aaa gtg gac ccc ttc tct ttc aaa  aca aga gcc aag tcc  tgc gga gaa   3024
Lys Val Asp Pro Phe Ser Phe Lys  Thr Arg Ala Lys Ser  Cys Gly Glu
        995                 1000                1005

aga gat  gtc aag gga att cgg  aca ctc ttt ttg ggc  att cca gat      3069
Arg Asp  Val Lys Gly Ile Arg  Thr Leu Phe Leu Gly  Ile Pro Asp
    1010                1015                1020

gag aat  ttt gaa gat cac agt  gct cct cct tcc cct  gaa gaa aaa      3114
Glu Asn  Phe Glu Asp His Ser  Ala Pro Pro Ser Pro  Glu Glu Lys
    1025                1030                1035

gat tct  gga ttc ttc atg ctg  agg aag gac agt gag  agg cga gct      3159
Asp Ser  Gly Phe Phe Met Leu  Arg Lys Asp Ser Glu  Arg Arg Ala
    1040                1045                1050

acc ctt  cac agg atc ctg acg  gaa gac caa gac aaa  att gtg aga      3204
Thr Leu  His Arg Ile Leu Thr  Glu Asp Gln Asp Lys  Ile Val Arg
    1055                1060                1065

aac cta  atg gaa tct tta gct  cag ggg gct gaa gaa  ccg aaa cta      3249
Asn Leu  Met Glu Ser Leu Ala  Gln Gly Ala Glu Glu  Pro Lys Leu
    1070                1075                1080

aaa tgg  gaa cac atc aca acc  ctc att gca agc ctc  aga gaa ttt      3294
Lys Trp  Glu His Ile Thr Thr  Leu Ile Ala Ser Leu  Arg Glu Phe
    1085                1090                1095

gtg aga  tcc act gac cga aaa  atc ata gcc acc aca  ctg tca aag      3339
Val Arg  Ser Thr Asp Arg Lys  Ile Ile Ala Thr Thr  Leu Ser Lys
    1100                1105                1110

ctg aaa  ctg gag ctg gac ttc  gac agc cat ggc att  agc caa gtc      3384
Leu Lys  Leu Glu Leu Asp Phe  Asp Ser His Gly Ile  Ser Gln Val
    1115                1120                1125

cag gtg  gta ctc ttt ggt ttt  caa gat gct gtc aat  aaa gtt ctt      3429
Gln Val  Val Leu Phe Gly Phe  Gln Asp Ala Val Asn  Lys Val Leu
    1130                1135                1140

cgg aat  cat aac atc aag ccg  cac tgg atg ttt gcc  tta gac agt      3474

Arg Asn His Asn Ile Lys Pro His Trp Met Phe Ala Leu Asp Ser
    1145                1150                1155

atc att cgg aag gcg gta cag aca gcc att acc atc ctg gtt cca      3519
Ile Ile Arg Lys Ala Val Gln Thr Ala Ile Thr Ile Leu Val Pro
    1160                1165                1170

gaa cta agg cca cat ttc agc ctt gca tct gag agt gat act gct      3564
Glu Leu Arg Pro His Phe Ser Leu Ala Ser Glu Ser Asp Thr Ala
    1175                1180                1185

gat caa gaa gac ttg gat gta gaa gat gac cat gag gaa cag cct      3609
Asp Gln Glu Asp Leu Asp Val Glu Asp Asp His Glu Glu Gln Pro
    1190                1195                1200

tca aat caa act gtc cga aga cct cag gct gtc att gaa gat gct      3654
Ser Asn Gln Thr Val Arg Arg Pro Gln Ala Val Ile Glu Asp Ala
    1205                1210                1215

gtg gct acc tca ggc gtg agc acg ctc agt tct act gtg tct cat      3699
Val Ala Thr Ser Gly Val Ser Thr Leu Ser Ser Thr Val Ser His
    1220                1225                1230

gat tcc cag agt gct cac cgg tca ctg aat gta cag ctt gga agg      3744
Asp Ser Gln Ser Ala His Arg Ser Leu Asn Val Gln Leu Gly Arg
    1235                1240                1245

atg aaa ata gaa acc aat aga tta ctg gaa gaa ttg gtt cgg aaa      3789
Met Lys Ile Glu Thr Asn Arg Leu Leu Glu Glu Leu Val Arg Lys
    1250                1255                1260

gag aaa gaa tta caa gca ctc ctt cat cga gct att gaa gaa aaa      3834
Glu Lys Glu Leu Gln Ala Leu Leu His Arg Ala Ile Glu Glu Lys
    1265                1270                1275

gac caa gaa att aaa cac ctg aag ctt aag tcc caa ccc ata gaa      3879
Asp Gln Glu Ile Lys His Leu Lys Leu Lys Ser Gln Pro Ile Glu
    1280                1285                1290

att cct gaa ttg cct gta ttt cat cta aat tct tct ggc aca aat      3924
Ile Pro Glu Leu Pro Val Phe His Leu Asn Ser Ser Gly Thr Asn
    1295                1300                1305

att gaa gat tct gaa ctt acc gac tgg ctg aga gtg aat gga gct      3969
Ile Glu Asp Ser Glu Leu Thr Asp Trp Leu Arg Val Asn Gly Ala
    1310                1315                1320

gat gaa gac act ata agc cgg ttt ttg gct gaa gat tat aca cta      4014
Asp Glu Asp Thr Ile Ser Arg Phe Leu Ala Glu Asp Tyr Thr Leu
    1325                1330                1335

ttg gat gtt ctc tac tat gtt aca cgt gat gac tta aaa tgc ttg      4059
Leu Asp Val Leu Tyr Tyr Val Thr Arg Asp Asp Leu Lys Cys Leu
    1340                1345                1350

aga cta agg gga ggg atg ctg tgc aca ctg tgg aag gct atc att      4104
Arg Leu Arg Gly Gly Met Leu Cys Thr Leu Trp Lys Ala Ile Ile
    1355                1360                1365

gac ttt cga aac aaa cag act                                      4125
Asp Phe Arg Asn Lys Gln Thr
    1370                1375

<210>  2
<211>  1375
<212>  PRT
<213>  Homo sapiens

<400>  2

Met Ser Thr Glu Ala Asp Glu Gly Ile Thr Phe Ser Val Pro Pro Phe
1               5                   10                  15

Ala Pro Ser Gly Phe Cys Thr Ile Pro Glu Gly Gly Ile Cys Arg Arg
            20                  25                  30

Gly Gly Ala Ala Ala Val Gly Glu Gly Glu Glu His Gln Leu Pro Pro
        35                  40                  45

Pro Pro Pro Gly Ser Phe Trp Asn Val Glu Ser Ala Ala Ala Pro Gly
    50                  55                  60

Ile Gly Cys Pro Ala Ala Thr Ser Ser Ser Ser Ala Thr Arg Gly Arg
65                  70                  75                  80

Gly Ser Ser Val Gly Gly Gly Ser Arg Arg Thr Thr Val Ala Tyr Val
                85                  90                  95

Ile Asn Glu Ala Ser Gln Gly Gln Leu Val Val Ala Glu Ser Glu Ala
            100                 105                 110

Leu Gln Ser Leu Arg Glu Ala Cys Glu Thr Val Gly Ala Thr Leu Glu
        115                 120                 125

Thr Leu His Phe Gly Lys Leu Asp Phe Gly Glu Thr Thr Val Leu Asp
    130                 135                 140

Arg Phe Tyr Asn Ala Asp Ile Ala Val Val Glu Met Ser Asp Ala Phe
145                 150                 155                 160

Arg Gln Pro Ser Leu Phe Tyr His Leu Gly Val Arg Glu Ser Phe Ser
                165                 170                 175

Met Ala Asn Asn Ile Ile Leu Tyr Cys Asp Thr Asn Ser Asp Ser Leu
            180                 185                 190

Gln Ser Leu Lys Glu Ile Ile Cys Gln Lys Asn Thr Met Cys Thr Gly
        195                 200                 205

Asn Tyr Thr Phe Val Pro Tyr Met Ile Thr Pro His Asn Lys Val Tyr
    210                 215                 220

Cys Cys Asp Ser Ser Phe Met Lys Gly Leu Thr Glu Leu Met Gln Pro
225                 230                 235                 240

Asn Phe Glu Leu Leu Leu Gly Pro Ile Cys Leu Pro Leu Val Asp Arg
                245                 250                 255

Phe Ile Gln Leu Leu Lys Val Ala Gln Ala Ser Ser Ser Gln Tyr Phe
            260                 265                 270

Arg Glu Ser Ile Leu Asn Asp Ile Arg Lys Ala Arg Asn Leu Tyr Thr
        275                 280                 285

Gly Lys Glu Leu Ala Ala Glu Leu Ala Arg Ile Arg Gln Arg Val Asp
    290                 295                 300

Asn Ile Glu Val Leu Thr Ala Asp Ile Val Ile Asn Leu Leu Leu Ser
305                 310                 315                 320

Tyr Arg Asp Ile Gln Asp Tyr Asp Ser Ile Val Lys Leu Val Glu Thr
                325                 330                 335

Leu Glu Lys Leu Pro Thr Phe Asp Leu Ala Ser His His His Val Lys
            340                 345                 350

Phe His Tyr Ala Phe Ala Leu Asn Arg Arg Asn Leu Pro Gly Asp Arg
        355                 360                 365

Ala Lys Ala Leu Asp Ile Met Ile Pro Met Val Gln Ser Glu Gly Gln
    370                 375                 380

Val Ala Ser Asp Met Tyr Cys Leu Val Gly Arg Ile Tyr Lys Asp Met
385                 390                 395                 400

Phe Leu Asp Ser Asn Phe Thr Asp Thr Glu Ser Arg Asp His Gly Ala
                405                 410                 415

Ser Trp Phe Lys Lys Ala Phe Glu Ser Glu Pro Thr Leu Gln Ser Gly
            420                 425                 430

Ile Asn Tyr Ala Val Leu Leu Leu Ala Ala Gly His Gln Phe Glu Ser
        435                 440                 445

Ser Phe Glu Leu Arg Lys Val Gly Val Lys Leu Ser Ser Leu Leu Gly
    450                 455                 460

Lys Lys Gly Asn Leu Glu Lys Leu Gln Ser Tyr Trp Glu Val Gly Phe
465                 470                 475                 480

Phe Leu Gly Ala Ser Val Leu Ala Asn Asp His Met Arg Val Ile Gln
                485                 490                 495

Ala Ser Glu Lys Leu Phe Lys Leu Lys Thr Pro Ala Trp Tyr Leu Lys
            500                 505                 510

Ser Ile Val Glu Thr Ile Leu Ile Tyr Lys His Phe Val Lys Leu Thr
        515                 520                 525

Thr Glu Gln Pro Val Ala Lys Gln Glu Leu Val Asp Phe Trp Met Asp
    530                 535                 540

Phe Leu Val Glu Ala Thr Lys Thr Asp Val Thr Val Val Arg Phe Pro
545                 550                 555                 560

Val Leu Ile Leu Glu Pro Thr Lys Ile Tyr Gln Pro Ser Tyr Leu Ser
                565                 570                 575

Ile Asn Asn Glu Val Glu Glu Lys Thr Ile Ser Ile Trp His Val Leu
            580                 585                 590

Pro Asp Asp Lys Lys Gly Ile His Glu Trp Asn Phe Ser Ala Ser Ser
        595                 600                 605

Val Arg Gly Val Ser Ile Ser Lys Phe Glu Glu Arg Cys Cys Phe Leu
    610                 615                 620

Tyr Val Leu His Asn Ser Asp Asp Phe Gln Ile Tyr Phe Cys Thr Glu
625                 630                 635                 640

Leu His Cys Lys Lys Phe Phe Glu Met Val Asn Thr Ile Thr Glu Glu
                645                 650                 655

Lys Gly Arg Ser Thr Glu Glu Gly Asp Cys Glu Ser Asp Leu Leu Glu
            660                 665                 670

Tyr Asp Tyr Glu Tyr Asp Glu Asn Gly Asp Arg Val Val Leu Gly Lys
        675                 680                 685

Gly Thr Tyr Gly Ile Val Tyr Ala Gly Arg Asp Leu Ser Asn Gln Val
    690                 695                 700

Arg Ile Ala Ile Lys Glu Ile Pro Glu Arg Asp Ser Arg Tyr Ser Gln
705                 710                 715                 720

Pro Leu His Glu Glu Ile Ala Leu His Lys His Leu Lys His Lys Asn

                725                 730                 735

Ile Val Gln Tyr Leu Gly Ser Phe Ser Glu Asn Gly Phe Ile Lys Ile
            740                 745                 750

Phe Met Glu Gln Val Pro Gly Gly Ser Leu Tyr Ala Leu Leu Arg Ser
        755                 760                 765

Lys Trp Gly Pro Leu Lys Asp Asn Glu Gln Thr Ile Gly Phe Tyr Thr
    770                 775                 780

Lys Gln Ile Leu Glu Gly Leu Lys Tyr Leu His Asp Asn Gln Ile Val
785                 790                 795                 800

His Arg Asp Ile Lys Gly Asp Asn Val Leu Ile Asn Thr Tyr Ser Gly
                805                 810                 815

Val Leu Lys Ile Ser Asp Phe Gly Thr Ser Lys Arg Leu Ala Gly Ile
            820                 825                 830

Asn Pro Cys Thr Glu Thr Phe Thr Gly Thr Leu Gln Tyr Met Ala Pro
        835                 840                 845

Glu Ile Ile Asp Lys Gly Pro Arg Gly Tyr Gly Lys Ala Ala Asp Ile
    850                 855                 860

Trp Ser Leu Gly Cys Thr Ile Ile Glu Met Ala Thr Gly Lys Pro Pro
865                 870                 875                 880

Phe Tyr Glu Leu Gly Glu Pro Gln Ala Ala Met Phe Lys Val Gly Met
                885                 890                 895

Phe Lys Val His Pro Glu Ile Pro Glu Ser Met Ser Ala Glu Ala Lys
            900                 905                 910

Ala Phe Ile Leu Lys Cys Phe Glu Pro Asp Pro Asp Lys Arg Ala Cys
        915                 920                 925

Ala Asn Asp Leu Leu Val Asp Glu Phe Leu Lys Val Ser Ser Lys Lys
    930                 935                 940

Lys Lys Thr Gln Pro Lys Leu Ser Ala Leu Ser Ala Gly Ser Asn Ala
945                 950                 955                 960

Glu Tyr Leu Arg Ser Ile Ser Leu Pro Val Pro Val Leu Val Glu Asp
                965                 970                 975

Thr Ser Ser Ser Ser Glu Tyr Gly Ser Val Ser Pro Asp Thr Glu Leu
            980                 985                 990

Lys Val Asp Pro Phe Ser Phe Lys  Thr Arg Ala Lys Ser  Cys Gly Glu
        995                 1000                 1005

Arg Asp  Val Lys Gly Ile Arg  Thr Leu Phe Leu Gly  Ile Pro Asp
    1010                 1015                 1020

Glu Asn  Phe Glu Asp His Ser  Ala Pro Pro Ser Pro  Glu Glu Lys
    1025                 1030                 1035

Asp Ser  Gly Phe Phe Met Leu  Arg Lys Asp Ser Glu  Arg Arg Ala
    1040                 1045                 1050

Thr Leu  His Arg Ile Leu Thr  Glu Asp Gln Asp Lys  Ile Val Arg
    1055                 1060                 1065

Asn Leu  Met Glu Ser Leu Ala  Gln Gly Ala Glu Glu  Pro Lys Leu
    1070                 1075                 1080

Lys Trp  Glu His Ile Thr Thr  Leu Ile Ala Ser Leu  Arg Glu Phe
    1085                 1090                 1095

Val Arg  Ser Thr Asp Arg Lys  Ile Ile Ala Thr Thr  Leu Ser Lys
    1100                 1105                 1110

Leu Lys  Leu Glu Leu Asp Phe  Asp Ser His Gly Ile  Ser Gln Val
    1115                 1120                 1125

Gln Val  Val Leu Phe Gly Phe  Gln Asp Ala Val Asn  Lys Val Leu
    1130                 1135                 1140

Arg Asn  His Asn Ile Lys Pro  His Trp Met Phe Ala  Leu Asp Ser
    1145                 1150                 1155

Ile Ile  Arg Lys Ala Val Gln  Thr Ala Ile Thr Ile  Leu Val Pro
    1160                 1165                 1170

Glu Leu  Arg Pro His Phe Ser  Leu Ala Ser Glu Ser  Asp Thr Ala
    1175                 1180                 1185

Asp Gln  Glu Asp Leu Asp Val  Glu Asp Asp His Glu  Glu Gln Pro
    1190                 1195                 1200

Ser Asn  Gln Thr Val Arg Arg  Pro Gln Ala Val Ile  Glu Asp Ala
    1205                 1210                 1215

Val Ala Thr Ser Gly Val Ser Thr Leu Ser Ser Thr Val Ser His
    1220                1225                1230

Asp Ser Gln Ser Ala His Arg Ser Leu Asn Val Gln Leu Gly Arg
    1235                1240                1245

Met Lys Ile Glu Thr Asn Arg Leu Leu Glu Glu Leu Val Arg Lys
    1250                1255                1260

Glu Lys Glu Leu Gln Ala Leu Leu His Arg Ala Ile Glu Glu Lys
    1265                1270                1275

Asp Gln Glu Ile Lys His Leu Lys Leu Lys Ser Gln Pro Ile Glu
    1280                1285                1290

Ile Pro Glu Leu Pro Val Phe His Leu Asn Ser Ser Gly Thr Asn
    1295                1300                1305

Ile Glu Asp Ser Glu Leu Thr Asp Trp Leu Arg Val Asn Gly Ala
    1310                1315                1320

Asp Glu Asp Thr Ile Ser Arg Phe Leu Ala Glu Asp Tyr Thr Leu
    1325                1330                1335

Leu Asp Val Leu Tyr Tyr Val Thr Arg Asp Asp Leu Lys Cys Leu
    1340                1345                1350

Arg Leu Arg Gly Gly Met Leu Cys Thr Leu Trp Lys Ala Ile Ile
    1355                1360                1365

Asp Phe Arg Asn Lys Gln Thr
    1370                1375

## Description

### Field of the Invention

The present invention relates to novel use of apoptosis signal-regulating kinase 1 (ASK1), a nucleic acid encoding same and an antibody thereto, particularly, use of ASK1 or a nucleic acid encoding same for screening for a pharmaceutical agent effective for the prophylaxis or treatment of apoptosis or an inflammation-associated diseases (e.g., diabetes and complications thereof, or chronic obstructive pulmonary disease) and the like.

### Background Art

The mitogen-activated protein (MAP) kinase cascade is a signal transduction mechanism through which MAP kinase kinase kinase (MAPKKK) activated by a physicochemical stress or an inflammatory cytokine such as tumor necrosis factor-α (TNF-α) or interleukin-1 (IL-1) sequentially activates MAP kinase kinase (MAPKK) and MAP kinase (MAPK), and cells, in response to these stimuli, exhibit phenotypes such as survival, proliferation, differentiation and death (apoptosis). c-Jun N-terminal kinase (JNK) and p38 MAP kinase (p38) are known as MAPKs that play a part of the role in the signal transduction pathway to induce apoptosis (see, for example, non-patent reference 1).

JNK and p38 are activated by MKK4/MKK7 and MKK3/MKK6, respectively, which are MAPKKs. These MAPKKs are activated by MAPKKK known as ASK1 (patent reference 1, non-patent reference 2). In addition to ASK1, many MAPKKKs have been reported, and ASK1 is characterized by the capability of inducing apoptosis of cells through signal transduction via the activation of JNK and/or p38. Recently, ASK1 activation has been suggested to be involved in cell differentiation such as keratinocyte differentiation and PC12 cell axon elongation as well, and ASK1 has been shown to play an important role, not only in apoptosis, but also in the control of cell fate.

Since ASK1 is an important molecule that influences the subsequent fate of cells as described above, its activation/inactivation is considered to be involved by various factors and undergoes complicated control. For example, protein phosphatase 5 (PP5) is considered to restore activated ASK1 to an inactivated state by binding directly to ASK1 under stimulation with H₂O₂ and dephosphorylating threonine (non-patent reference 3). Furthermore, it has also been reported that thioredoxin, a redox control factor, acts as an ASK1 activation inhibitor as constitutively bound to the N-terminal domain of ASK1 in the absence of oxidation stress and leaves ASK1 upon exposure to oxidation stress and hence causes the activation of ASK1 (non-patent reference 4), and that the 14-3-3 protein inhibits the activation of ASK1 by binding to the C-terminal domain of ASK1 (non-patent reference 5).

As mentioned above, since ASK1 can induce apoptosis of cell (particularly immortalized cell) through JNK/p38 pathway, the protein is suggested to be effective for inhibition of the formation/metastasis of malignant tumor (patent reference 1).

On the other hand, when the cell of ASK1 knockout (KO) mouse is treated with an endoplasmic reticulum stress inducer, apoptosis is significantly inhibited as compared to the cell of wild-type mouse, and when the cell of KO mouse is stimulated with LPS, cytokine·chemokine production is significantly suppressed as compared to the cell of wild-type mouse. Thus, ASK1 is closely related to apoptosis induction and cytokine·chemokine production due to endoplasmic reticulum stress. Accordingly, it is suggested that ASK1 inhibitory substances such as the above-mentioned thioredoxin, 14-3-3 protein and the like, ASK1 dominant-negative mutants, ASK1 antisense oligonucleotides and the like are effective for the prophylaxis or treatment of endoplasmic reticulum stress-associated diseases such as neurodegenerative diseases (e.g., polyglutamine disease etc.) and the like, and immune diseases (patent references 2, 3).
[patent reference 1] JP-A-10-93
[patent reference 2] WO 02/38179
[patent reference 3] WO 03/00826
[non-patent reference 1] Science, 270: 1326 (1995)
[non-patent reference 2] Science, 275: 90-94 (1997)
[non-patent reference 3] EMBO Journal, 20: 6028-6036 (2001)
[non-patent reference 4] EMBO Journal, 17: 2596-2606 (1998)
[non-patent reference 5] Proceedings of National Academy of Sciences, USA, 96: 8511-8515 (1999)

### Disclosure of the Invention

As mentioned above, the biological significance of ASK1 and the relationship thereof with various diseases have been gradually clarified, and use of ASK1 as a prophylactic or therapeutic drug for such diseases has been proposed. However, a method for searching for a prophylactic or therapeutic drug for the above-mentioned diseases wherein ASK1 is the drug discovery target has not been reported yet. Accordingly, a first object of the present invention is to provide a screening method for a prophylactic or therapeutic drug for various diseases, which uses ASK1.

The relationship between the diseases other than those mentioned above and ASK1 remains mostly unclarified (for example, the relationship with diabetes and complications thereof, or chronic obstructive pulmonary disease (COPD) has not been reported). Accordingly, a second object of the present invention is to newly find an unknown relationship between ASK1 and certain diseases, and provide a novel method for the prophylaxis or treatment of such diseases based thereon.

When a hyperglycemic state is maintained for a long time in diabetes, complications such as diabetic neuropathy, diabetic retinopathy, diabetic nephropathy and the like are developed. For example, while diabetic nephropathy is treated with angiotensin converting enzyme (ACE) inhibitors and the like, since such drugs affect renal hemodynamics, they cannot be used for patients with severe kidney hypofunction. As a therapeutic drug for diabetic neuropathy, moreover, aldose reductase inhibitors are known. However, since they sometimes cause side effects such as skin rash, diarrhea, liver·kidney dysfunction and the like, their use outside our country is not observed. Hence, the development of a safe and effective prophylactic or therapeutic drug for diabetic complications has been awaited.

Accordingly, a third object of the present invention is to provide a novel prophylactic or therapeutic drug for diabetes and complications thereof, and a screening method therefor.

Chronic obstructive pulmonary disease (COPD) is currently ranked the 4th of the cause of death by the statistics of the World Health Organization (WHO) (5 million or more patients of not less than 40 years old are considered to be present in Japan), and a further increase in the number of patients in the future is predicted. As therapeutic drugs for COPD, however, bronchodilators such as anticholinergic drugs, β2 stimulating agents, theophylline preparations and the like, expectorant, steroids for acute exacerbation, and the like are used only for a palliative therapy, and the development of a therapeutic drug aiming at a basic pathological improvement is desired.

Accordingly, a fourth object of the present invention is to provide a novel prophylactic or therapeutic drug for COPD, and a screening method therefor.

The present inventors have conducted intensive studies in an attempt to achieve the aforementioned objects and found that, in a diabetes model prepared by administering streptozocin to ASK1 knockout mouse, the progress of neuropathy is inhibited as compared to wild-type mouse treated similarly, in ASK1 knockout mouse, increase in the blood glucose level under high-fat diet is significantly suppressed as compared to wild-type mouse, and, in diabetic nephropathy model (*db*/*db*) mouse, the expression level of ASK1 in the kidney increases as compared to normal mouse. These results suggest that ASK1 is deeply involved in the onset or progress of diabetes and complications thereof, and inhibition of the expression or activity of ASK1 is effective for the prophylaxis and treatment of diabetes and complications thereof.

Moreover, the present inventors have found that, in COPD model prepared by exposing ASK1 knockout mouse to tobacco smoke, inflammatory changes in the lung can be suppressed as compared to wild-type mouse treated similarly. Moreover, they have found that the expression level of activated ASK1 in the lung has increased by exposing wild-type mouse to tobacco smoke.

The present inventor further studied based on these findings and completed the present invention.

Accordingly, the present invention provides
[1] a method for screening for a substance for the prophylaxis or treatment of apoptosis or an inflammation-associated disease, which comprises using a protein having an amino acid sequence the same as or substantially the same as the amino acid sequence shown by SEQ ID NO: 2 or a partial peptide thereof or a salt thereof,
[2] a method for screening for a substance for the prophylaxis or treatment of diabetes or complications thereof, which comprises using a protein having an amino acid sequence the same as or substantially the same as the amino acid sequence shown by SEQ ID NO: 2 or a partial peptide thereof or a salt thereof,
[3] a method for screening for a substance for the prophylaxis or treatment of a chronic obstructive pulmonary disease, which comprises using a protein having an amino acid sequence the same as or substantially the same as the amino acid sequence shown by SEQ ID NO: 2 or a partial peptide thereof or a salt thereof,
[4] the screening method of any one of the above-mentioned [1]-[3], which comprises comparing the activity of a protein having an amino acid sequence the same as or substantially the same as the amino acid sequence shown by SEQ ID NO: 2 or a partial peptide thereof or a salt thereof, between that in the presence of a test substance and that in the absence of the test substance,
[5] the screening method of the above-mentioned [4], which comprises culturing a cell having the ability to produce said protein or a partial peptide thereof or a salt thereof in the presence of a test substance and in the absence of the test substance, and comparing the activity of the protein or a partial peptide thereof or a salt thereof under the both conditions,
[6] the screening method of any one of the above-mentioned [1]-[3], which comprises using a protein having the amino acid sequence shown by SEQ ID NO: 2 or a partial peptide thereof or a salt thereof,
[7] the screening method of the above-mentioned [2], wherein the complication is diabetic nephropathy or diabetic neuropathy,
[8] a method for screening for a substance for the prophylaxis or treatment of apoptosis or an inflammation-associated disease, which comprises comparing, between that in the presence of a test substance and that in the absence of the test substance, the expression of a protein having an amino acid sequence the same as or substantially the same as the amino acid sequence shown by SEQ ID NO: 2 or a partial peptide thereof or a salt thereof, in a cell having the ability to produce the protein or a partial peptide thereof or a salt thereof,
[9] a method for screening for a substance for the prophylaxis or treatment of diabetes or complications thereof, which comprises comparing, between that in the presence of a test substance and that in the absence of the test substance, the expression of a protein having an amino acid sequence the same as or substantially the same as the amino acid sequence shown by SEQ ID NO: 2 or a partial peptide thereof or a salt thereof, in a cell having the ability to produce the protein or a partial peptide thereof or a salt thereof,
[10] a method for screening for a substance for the prophylaxis or treatment of a chronic obstructive pulmonary disease, which comprises comparing, between that in the presence of a test substance and that in the absence of the test substance, the expression of a protein having an amino acid sequence the same as or substantially the same as the amino acid sequence shown by SEQ ID NO: 2 or a partial peptide thereof or a salt thereof, in a cell having the ability to produce the protein or a partial peptide thereof or a salt thereof,
[11] the screening method of any one of the above-mentioned [8] to [10], which comprises using a nucleic acid having a base sequence encoding a protein having an amino acid sequence the same as or substantially the same as the amino acid sequence shown by SEQ ID NO: 2 or a part thereof, or an antibody against a protein having an amino acid sequence the same as or substantially the same as the amino acid sequence shown by SEQ ID NO: 2 or a partial peptide thereof or a salt thereof,
[12] the screening method of any one of the above-mentioned [8]-[10], wherein the cell has an ability to produce a protein having the amino acid sequence shown by SEQ ID NO: 2 or a partial peptide thereof or a salt thereof,
[13] the screening method of the above-mentioned [9], wherein the complication is diabetic nephropathy or diabetic neuropathy,
[14] an agent for the prophylaxis or treatment of apoptosis or an inflammation-associated disease, which comprises an antibody against a protein having an amino acid sequence the same as or substantially the same as the amino acid sequence shown by SEQ ID NO: 2 or a partial peptide thereof or a salt thereof,
[15] a method for the prophylaxis or treatment of apoptosis or an inflammation-associated disease in a mammal, which comprises administering, to the mammal, an effective amount of an antibody against a protein having an amino acid sequence the same as or substantially the same as the amino acid sequence shown by SEQ ID NO: 2 or a partial peptide thereof or a salt thereof,
[16] use of an antibody against a protein having an amino acid sequence the same as or substantially the same as the amino acid sequence shown by SEQ ID NO: 2 or a partial peptide thereof or a salt thereof, for the production of an agent for the prophylaxis or treatment of apoptosis or an inflammation-associated disease,
[17] an agent for the prophylaxis or treatment of diabetes or complications thereof, which comprises an antibody against a protein having an amino acid sequence the same as or substantially the same as the amino acid sequence shown by SEQ ID NO: 2 or a partial peptide thereof or a salt thereof,
[18] a method for the prophylaxis or treatment of diabetes or complications thereof in a mammal, which comprises administering, to the mammal, an effective amount of an antibody against a protein having an amino acid sequence the same as or substantially the same as the amino acid sequence shown by SEQ ID NO: 2 or a partial peptide thereof or a salt thereof,
[19] use of an antibody against a protein having an amino acid sequence the same as or substantially the same as the amino acid sequence shown by SEQ ID NO: 2 or a partial peptide thereof or a salt thereof, for the production of an agent for the prophylaxis or treatment of diabetes or complications thereof,
[20] the agent of the above-mentioned [17], wherein the complications is diabetic nephropathy or diabetic neuropathy,
[21] an agent for the prophylaxis or treatment of a chronic obstructive pulmonary disease, which comprises an antibody against a protein having an amino acid sequence the same as or substantially the same as the amino acid sequence shown by SEQ ID NO: 2 or a partial peptide thereof or a salt thereof,
[22] a method for the prophylaxis or treatment of a chronic obstructive pulmonary disease in a mammal, which comprises administering, to the mammal, an effective amount of an antibody against a protein having an amino acid sequence the same as or substantially the same as the amino acid sequence shown by SEQ ID NO: 2 or a partial peptide thereof or a salt thereof,
[23] use of an antibody against a protein having an amino acid sequence the same as or substantially the same as the amino acid sequence shown by SEQ ID NO: 2 or a partial peptide thereof or a salt thereof, for the production of an agent for the prophylaxis or treatment of a chronic obstructive pulmonary disease,
[24] an agent for the prophylaxis or treatment of type 2 diabetes or diabetic complications, which comprises a nucleic acid having a base sequence complementary to a base sequence encoding a protein having an amino acid sequence the same as or substantially the same as the amino acid sequence shown by SEQ ID NO: 2 or a part thereof,
[25] a method for the prophylaxis or treatment of type 2 diabetes or diabetic complications in a mammal, which comprises administering, to the mammal, an effective amount of a nucleic acid having a base sequence complementary to a base sequence encoding a protein having an amino acid sequence the same as or substantially the same as the amino acid sequence shown by SEQ ID NO: 2 or a part thereof,
[26] use of a nucleic acid having a base sequence complementary to a base sequence encoding a protein having an amino acid sequence the same as or substantially the same as the amino acid sequence shown by SEQ ID NO: 2 or a part thereof, for the production of an agent for the prophylaxis or treatment of type 2 diabetes or diabetic complications,
[27] the agent of the above-mentioned [24], wherein the diabetic complication is diabetic nephropathy or diabetic neuropathy,
[28] an agent for the prophylaxis or treatment of a chronic obstructive pulmonary disease, which comprises a nucleic acid having a base sequence complementary to a base sequence encoding a protein having an amino acid sequence the same as or substantially the same as the amino acid sequence shown by SEQ ID NO: 2 or a part thereof,
[29] a method for the prophylaxis or treatment of a chronic obstructive pulmonary disease in a mammal, which comprises administering, to the mammal, an effective amount of a nucleic acid having a base sequence complementary to a base sequence encoding a protein having an amino acid sequence the same as or substantially the same as the amino acid sequence shown by SEQ ID NO: 2 or a part thereof,
[30] use of a nucleic acid having a base sequence complementary to a base sequence encoding a protein having an amino acid sequence the same as or substantially the same as the amino acid sequence shown by SEQ ID NO: 2 or a part thereof, for the production of an agent for the prophylaxis or treatment of a chronic obstructive pulmonary disease,
[31] a reagent for diagnosing apoptosis or an inflammation-associated disease, which comprises an antibody against a protein having an amino acid sequence the same as or substantially the same as the amino acid sequence shown by SEQ ID NO: 2 or a partial peptide thereof or a salt thereof,
[32] a method for diagnosing apoptosis or an inflammation-associated disease in a mammal, which comprises using an antibody against a protein having an amino acid sequence the same as or substantially the same as the amino acid sequence shown by SEQ ID NO: 2 or a partial peptide thereof or a salt thereof,
[33] use of an antibody against a protein having an amino acid sequence the same as or substantially the same as the amino acid sequence shown by SEQ ID NO: 2 or a partial peptide thereof or a salt thereof, for the production of a reagent for diagnosing apoptosis or an inflammation-associated disease,
[34] a reagent for diagnosing diabetes or complications thereof, which comprises an antibody against a protein having an amino acid sequence the same as or substantially the same as the amino acid sequence shown by SEQ ID NO: 2 or a partial peptide thereof or a salt thereof,
[35] a method for diagnosing diabetes or complications thereof in a mammal, which comprises using an antibody against a protein having an amino acid sequence the same as or substantially the same as the amino acid sequence shown by SEQ ID NO: 2 or a partial peptide thereof or a salt thereof,
[36] use of an antibody against a protein having an amino acid sequence the same as or substantially the same as the amino acid sequence shown by SEQ ID NO: 2 or a partial peptide thereof or a salt thereof, for the production of a reagent for diagnosing diabetes or complications thereof,
[37] the diagnostic reagent of the above-mentioned [34], wherein the complication is diabetic nephropathy or diabetic neuropathy,
[38] a reagent for diagnosing a chronic obstructive pulmonary disease, which comprises an antibody against a protein having an amino acid sequence the same as or substantially the same as the amino acid sequence shown by SEQ ID NO: 2 or a partial peptide thereof or a salt thereof,
[39] a method for diagnosing a chronic obstructive pulmonary disease in a mammal, which comprises using an antibody against a protein having an amino acid sequence the same as or substantially the same as the amino acid sequence shown by SEQ ID NO: 2 or a partial peptide thereof or a salt thereof,
[40] use of an antibody against a protein having an amino acid sequence the same as or substantially the same as the amino acid sequence shown by SEQ ID NO: 2 or a partial peptide thereof or a salt thereof, for the production of a reagent for diagnosing a chronic obstructive pulmonary disease,
[41] a reagent for diagnosing apoptosis or an inflammation-associated disease, which comprises a nucleic acid having a base sequence encoding a protein having an amino acid sequence the same as or substantially the same as the amino acid sequence shown by SEQ ID NO: 2 or a part thereof,
[42] a method for diagnosing apoptosis or an inflammation-associated disease in a mammal, which comprises administering, to the mammal, an effective amount of a nucleic acid having a base sequence encoding a protein having an amino acid sequence the same as or substantially the same as the amino acid sequence shown by SEQ ID NO: 2 or a part thereof,
[43] use of a nucleic acid having a base sequence encoding a protein having an amino acid sequence the same as or substantially the same as the amino acid sequence shown by SEQ ID NO: 2 or a part thereof, for the production of a reagent for diagnosing apoptosis or an inflammation-associated disease,
[44] a reagent for diagnosing diabetes or complications thereof, which comprises a nucleic acid having a base sequence encoding a protein having an amino acid sequence the same as or substantially the same as the amino acid sequence shown by SEQ ID NO: 2 or a part thereof,
[45] a method for diagnosing diabetes or complications thereof in a mammal, which comprises administering, to the mammal, an effective amount of a nucleic acid having a base sequence encoding a protein having an amino acid sequence the same as or substantially the same as the amino acid sequence shown by SEQ ID NO: 2 or a part thereof,
[46] use of a nucleic acid having a base sequence encoding a protein having an amino acid sequence the same as or substantially the same as the amino acid sequence shown by SEQ ID NO: 2 or a part thereof, for the production of a reagent for diagnosing diabetes or complications thereof,
[47] the reagent of the above-mentioned [44], wherein the complication is diabetic nephropathy or diabetic neuropathy,
[48] a reagent for diagnosing a chronic obstructive pulmonary disease, which comprises a nucleic acid having a base sequence encoding a protein having an amino acid sequence the same as or substantially the same as the amino acid sequence shown by SEQ ID NO: 2 or a part thereof,
[49] a method for diagnosing a chronic obstructive pulmonary disease in a mammal, which comprises administering, to the mammal, an effective amount of a nucleic acid having a base sequence encoding a protein having an amino acid sequence the same as or substantially the same as the amino acid sequence shown by SEQ ID NO: 2 or a part thereof,
[50] use of a nucleic acid having a base sequence encoding a protein having an amino acid sequence the same as or substantially the same as the amino acid sequence shown by SEQ ID NO: 2 or a part thereof, for the production of a reagent for diagnosing a chronic obstructive pulmonary disease,
[51] an agent for the prophylaxis or treatment of type 2 diabetes or diabetic complications (excluding diabetic neuropathy), which comprises an ASK1 inhibitory substance,
[52] a method for the prophylaxis or treatment of type 2 diabetes or diabetic complications (excluding diabetic neuropathy) in a mammal, which comprises administering, to the mammal, an effective amount of an ASK1 inhibitory substance,
[53] use of an ASK1 inhibitory substance for the production of an agent for the prophylaxis or treatment of type 2 diabetes or diabetic complications (excluding diabetic neuropathy),
[54] the agent of the above-mentioned [51], wherein the ASK1 inhibitory substance is one or more compounds selected from the group consisting of a dominant-negative mutant of ASK1, glutathione S-transferase, neph, 14-3-3, thioredoxin and HSP72,
[55] the agent of the above-mentioned [51], wherein the diabetic complication is diabetic nephropathy,
[56] an agent for the prophylaxis or treatment of a chronic obstructive pulmonary disease, which comprises an ASK1 inhibitory substance,
[57] a method for the prophylaxis or treatment of a chronic obstructive pulmonary disease, which comprises administering, to the mammal, an effective amount of an ASK1 inhibitory substance, and
[58] use of an ASK1 inhibitory substance for the production of an agent for the prophylaxis or treatment of a chronic obstructive pulmonary disease.

The screening method of the present invention affords an effect of quickly and conveniently selecting a candidate compound effective for the prophylaxis or treatment of apoptosis or an inflammation-associated disease by determining the ASK1 expression or activity regulating action of the test compound.

### Brief Description of the Drawings

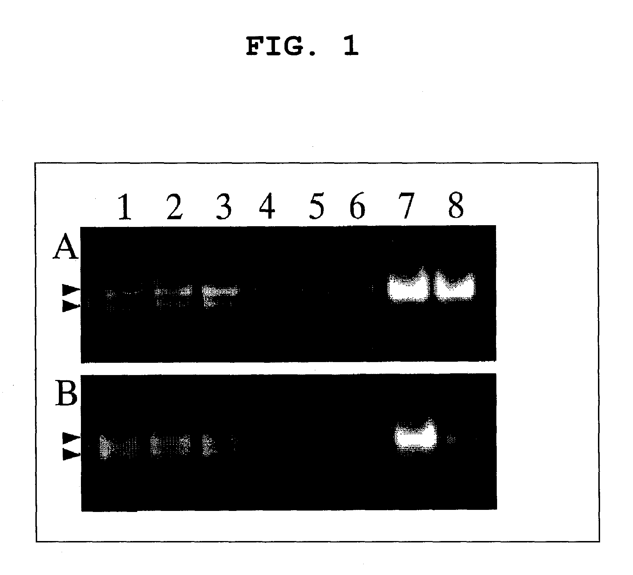

Fig. 1 shows the expression level (panel A) and activity (autophosphorylation activity)(panel B) of ASK1 in a kidney tissue of C57BL/KsJ db/db mouse and C57BL/KsJ db/+m mouse, wherein lanes 1-3: C57BL/KsJ db/db mouse kidney tissue; lanes 4-6: C57BL/KsJ db/+m mouse kidney tissue; lane 7: HEK293 cell (H₂O₂ addition); lane 8: HEK293 cell (H₂O₂ non-addition)

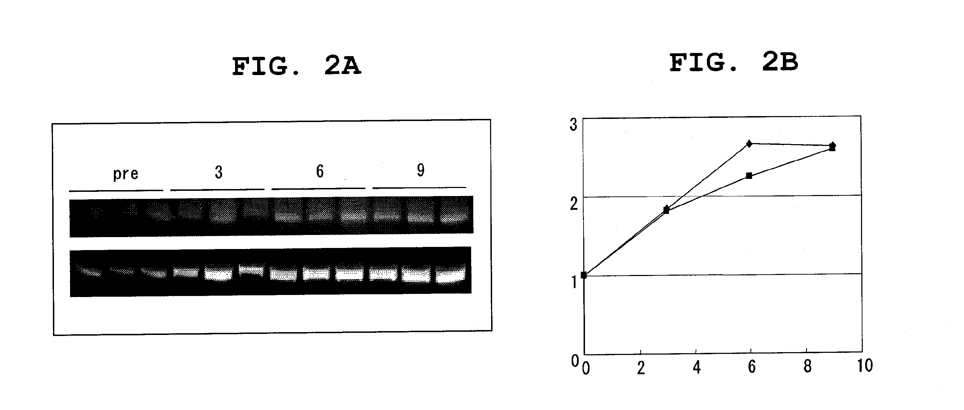

Fig. 2 shows the expression level and activity (autophosphorylation activity) of ASK1 in a lung tissue of tobacco smoke exposure COPD model. Fig. 2A shows western blotting images of phosphorylated ASK1 and ASK1 (upper panel: phosphorylated ASK1, lower panel: ASK1, each numeric value is time (hr) after tobacco smoke exposure) and Fig. 2B shows the results of plotting (-◆-: phosphorylated ASK1, -■-: ASK1) of the band after quantification (value relative to band intensity before exposure (pre) as 1), relative to time (hr) after tobacco smoke exposure.

### Best Mode for Embodiment of the Invention

The ASK1 used in the present invention is a protein that comprises the same or substantially the same amino acid sequence as the amino acid sequence shown by SEQ ID NO:2.

The ASK1 may be a protein derived from a cell (e.g., hepatocyte, splenocyte, nerve cell, glial cell, pancreatic β cell, myeloid cell, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding precursor cell, stem cell or cancer cell thereof, and the like) of a warm-blooded animal (for example, human, mouse, rat, guinea pig, hamster, rabbit, sheep, goat, swine, bovine, horse, bird, cat, dog, monkey, chimpanzee and the like), or any tissue where such cells are present (e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle (e.g., skeletal muscle, smooth muscle), lung, gastrointestinal tract (e.g., large intestine and small intestine), blood vessel, heart, thymus, spleen, submandibular gland, peripheral blood, prostate, testicle, ovary, placenta, uterus, bone, joint, adipose tissue, and the like) and may also be a chemically synthesized protein or a protein synthesized using a cell-free translation system. Alternatively, the ASK1 may be a recombinant protein produced by a transformant introduced with a nucleic acid having the base sequence that encodes the above-described amino acid sequence.

As "substantially the same amino acid sequence" as the amino acid sequence shown by SEQ ID NO:2, an amino acid sequence having a homology of about 70% or more, preferably about 80% or more, more preferably about 90% or more, particularly preferably about 95% or more, and most preferably about 98% or more, to the amino acid sequence shown by SEQ ID NO:2 and the like can be mentioned. As used herein, the "homology" means the ratio (%) in an optimal alignment (preferably, the algorithm can consider introduction of gap into one or both of sequences for optimal alignment) of the same amino acid and analogous amino acid residue relative to the overlapping whole amino acid residue, when two amino acid sequences are aligned using a mathematical algorithm known in the technique field. The "analogous amino acid" means amino acid analogous in physicochemical properties and, for example, amino acids classified into the same group such as aromatic amino acid (Phe, Trp, Tyr), aliphatic amino acid (Ala, Leu, Ile, Val), polar amino acid (Gln, Asn), basic amino acid (Lys, Arg, His), acidic amino acid (Glu, Asp), amino acid having a hydroxyl group (Ser, Thr), amino acid having a small side chain (Gly, Ala, Ser, Thr, Met) and the like can be mentioned. Substitution with such analogous amino acid is predicted to cause no change in the phenotype of protein (i.e., conservative amino acid substitution). Specific examples of the conservative amino acid substitution are well known in the technique field and described in various literatures (e.g., see Bowie et al., Science, 247: 1306-1310 (1990)).

The homology of amino acid sequence in the present specification can be calculated using the homology calculation algorithm NCBI BLAST (National Center for Biotechnology Information Basic Local Alignment Search Tool) under the following conditions (expectancy = 10; allowing gap; matrix=BLOSUM62;filtering=OFF). As other algorithm for determining the homology of amino acid sequence, for example, the algorithm described in Karlin et al., Proc. Natl. Acad. Sci. USA, 90: 5873-5877 (1993) [the algorithm is incorporated in NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in GCG software package], the algorithm described in Myers and Miller, CABIOS, 4: 11-17 (1988) [the algorithm is incorporated in ALIGN program (version 2.0) which is a part of the CGC sequence alignment software package], the algorithm described in Pearson et al., Proc. Natl. Acad. Sci. USA, 85: 2444-2448 (1988) [the algorithm is incorporated in the FASTA program in GCG software package] and the like can be mentioned, and they can be preferably used in a similar way.

More preferably, the "substantially the same amino acid sequence" is an amino acid sequence having a homology of not less than about 70%, preferably not less than about 80%, more preferably not less than about 90%, particularly preferably not less than about 95%, most preferably not less than about 98% with the amino acid sequence shown by SEQ ID NO: 2.

As examples of "the protein having substantially the same amino acid sequence" as the amino acid sequence shown by SEQ ID NO:2, a protein that comprises substantially the same amino acid sequence as the aforementioned amino acid sequence shown by SEQ ID NO:2, and that has substantially the same quality of activity as a protein that comprises the amino acid sequence shown by SEQ ID NO:2, and the like are preferred.

As examples of "substantially the same quality of activity", activity to promote the autophosphorylation of ASK1 or phosphorylation (activation) of kinase group located at the downstream of cascade thereof (e.g., MKK4/MKK7, MKK3/ MKK6, JNK, p38 and the like), activity to induce cell apoptosis, and the like can be mentioned. Substantially the same quality means that the properties thereof are qualitatively (e.g., physiologically or pharmacologically) equivalent to each other. Accordingly, it is preferable that the proteins be equivalent to each other in terms of activities such as to promote the activation of the ASK1 cascade, but quantitative factors such as the extent of these activities and the molecular weights of the proteins may be different (for example, differences within the range of about 0.01 to 100 times, preferably about 0.1 to 10 times, more preferably 0.5 to 2 times, with respect to activity, can be mentioned).

The autophosphorylation activity of ASK1 or ASK1 cascade activation promoting activity can be measured according to a known method, for example, detecting autophosphorylation of ASK1 or phosphorylation of a substance that can be a substrate of ASK1, such as kinase group (e.g., MKK4/MKK7, MKK3/MKK6, JNK, p38 and the like) located at the downstream of the its cascade and the like, using a labeled phosphoric acid group donor, and the like, and the apoptosis induction activity can be measured by the measurement of cell deth-induction rate, morphological observation of cell, detection of DNA fragmentation and the like.

The ASK1 to be used in the present invention includes, for example, a protein having (1) an amino acid sequence shown by SEQ ID NO: 2, wherein one or more (e.g., about 1-300, preferably about 1-100, more preferably about 1-50, more preferably about 1-10, most preferably several (1-5)) amino acids have been deleted, (2) an amino acid sequence shown by SEQ ID NO: 2, wherein one or more (e.g., about 1-300, preferably about 1-100, more preferably about 1-50, more preferably about 1-10, most preferably several (1-5)) amino acids have been added, (3) an amino acid sequence shown by SEQ ID NO: 2, wherein one or more (e.g., about 1-300, preferably about 1-100, more preferably about 1-50, more preferably about 1-10, most preferably several (1-5)) amino acids have been inserted, (4) an amino acid sequence shown by SEQ ID NO: 2, wherein one or more (e.g., about 1-300, preferably about 1-100, more preferably about 1-50, more preferably about 1-10, most preferably several (1-5)) amino acids have been substituted by other amino acid(s), or (5) an amino acid sequence which is a combination thereof.

When the amino acid sequence is inserted, deleted or substituted as mentioned above, the position of the insertion, deletion or substitution is not particularly limited as long as the activity of ASK1 [e.g., promoting activity of autophosphorylation of ASK1 or phosphorylation (activation) of kinase group located at the downstream thereof (e.g., MKK4/MKK7, MKK3/MKK6, JNK, p38 and the like), cell apoptosis induction activity and the like] can be maintained.

The ASK1 to be used in the present invention preferably includes human ASK1 (GenBank Accession NO:BAA12684) having the amino acid sequence shown by SEQ ID NO: 2 or its allele variant [e.g., GenBank Accession NO:NP_005914 (an amino acid sequence shown by SEQ ID NO: 2, wherein one amino acid is deleted (ΔA₉₆₀) and two amino acids are substituted (Y₇₆₃S, I₁₃₀₉T)) etc.], or ortholog in other warm-blooded animal (e.g., mouse, rat, guinea pig, hamster, rabbit, sheep, goat, swine, bovine, horse, bird, cat, dog, monkey, chimpanzee and the like) [e.g., as mouse ortholog, GenBank Accession No:BAA23648 (having about 92% homology with the amino acid sequence shown by SEQ ID NO: 2) etc.].

With respect to the proteins and peptide mentioned herein, the left end is the N-terminal (amino terminal) and the right end is the C-terminal (carboxyl terminal) in accordance with the conventional peptide marking. For the ASK1, including a protein that comprises the amino acid sequence shown by SEQ ID NO:2, the C-terminal may be any of a carboxyl group (-COOH), a carboxylate (-COO⁻), an amide (-CONH₂), and an ester (-COOR).

Here, as R in the ester, a C₁₋₆ alkyl group such as methyl, ethyl, n-propyl, isopropyl and n-butyl; a C₃₋₈ cycloalkyl group such as cyclopentyl and cyclohexyl; a C₆₋₁₂ aryl group such as phenyl and α-naphthyl; a phenyl-C₁₋₂ alkyl group such as benzyl and phenethyl; a C₇₋₁₄ aralkyl group such as an α-naphthyl-C₁₋₂ alkyl group such as α-naphthylmethyl; a pivaloyloxymethyl group; and the like can be used.

When ASK1 has a carboxyl group (or a carboxylate) in addition to that on the C-terminal, one in which the carboxyl group is amidated or esterified is also included in the ASK1 of the present invention. In this case, as the ester, the above-described C-terminal ester and the like, for example, can be used.

Furthermore, the ASK1 also includes a protein wherein the amino group of the N-terminal amino acid residue thereof (e.g., methionine residue) is protected by a protecting group (for example, a C₁₋₆ acyl group such as a C₁₋₆ alkanoyl group such as a formyl group or an acetyl group, and the like), a protein wherein the N-terminal glutamine residue, which is produced by cleavage in vivo, has been converted to pyroglutamic acid, a protein wherein a substituent (for example, -OH, -SH, an amino group, an imidazole group, an indole group, a guadinino group and the like) on an amino acid side chain in the molecule is protected by an appropriate protecting group (for example, a C₁₋₆ acyl group such as a C₁₋₆ alkanoyl group such as a formyl group or an acetyl group, and the like), a conjugated protein such as what is called a glycoprotein, which has a sugar chain bound thereto, and the like.

The partial peptide of ASK1 to be used in the present invention has the above-mentioned partial amino acid sequence of ASK1 and substantially the same quality of activity as ASK1. As used herein, the "substantially the same quality of activity" is as defined above. In addition, "substantially the same quality of activity" can be measured in the same manner as mentioned above. In the present specification, the partial peptide is hereinafter to be referred to as "active peptide of the present invention".

The active peptide of the present invention is not particularly limited as long as it has the above-mentioned properties and, for example, a peptide having an amino acid sequence the same or substantially the same as a partial amino acid sequence consisting of not less than about 250 amino acids, preferably not less than about 500 amino acids, more preferably not less than about 1000 amino acids, from the amino acid sequence shown by SEQ ID NO: 2, and the like can be mentioned. The partial amino acid sequence may be at the N-terminal side sequence or C-terminal side sequence of ASK1, or may be an internal sequence. Alternatively, it may be an intermittent combination of such partial sequences.
Preferably, the active peptide of the present invention is a partial peptide of ASK1 having at least a serine/threonine-protein kinase catalytic domain (e.g., in the case of human ASK1, a partial amino acid sequence of amino acids 678-936 from the amino acid sequence shown by SEQ ID NO: 2) having a protein phosphorylation activity.

The partial peptide of ASK1 includes one functional as an (antagonist) inhibitor of ASK1 or the above-mentioned active peptide of the present invention. As such partial peptide, for example, one having an affinity for MKK4/MKK7 or MKK3/MKK6, which is a substrate protein of ASK1, but incapable of activating MAPKK and the like can be mentioned. Specifically, for example, a partial peptide partly or entirely defective in the above-mentioned serine/threonine·protein kinase catalytic domain and the like can be mentioned. As the kinase catalytic domain of ASK1, for example, the above-mentioned partial amino acid sequence can be mentioned for human ASK1.

In the present specification, this partial peptide is hereinafter referred to as "the inhibitory peptide of the present invention". Accordingly, the inhibitory peptide of the present invention is a peptide that has the same or substantially the same amino acid sequence as a part of the amino acid sequence shown by SEQ ID NO:2, and that does not activate kinase group located at the downstream of ASK1 signal cascade.

With respect to the partial peptide of ASK1 of the present invention (encompassing both of the active peptide of the present invention and the inhibitory peptide of the present invention; hereinafter, such case is to be also referred to as "the partial peptide of the present invention"), the C-terminal may be any of a carboxyl group (-COOH), a carboxylate (-COO⁻) , an amide (-CONH₂), and an ester (-COOR). Here, as R in the ester, the same as those mentioned for ASK1 above can be mentioned. When these peptides have a carboxyl group (or a carboxylate) in addition to that on the C-terminal, one in which the carboxyl group is amidated or esterified is also included in the partial peptide of the present invention. In this case, as the ester, the above-described C-terminal ester and the like, for example, can be used.

Furthermore, the partial peptide of the present invention also includes a peptide wherein the amino group of the N-terminal methionine residue is protected by a protecting group, a peptide wherein Gln, which is produced by cleavage on the N-terminal side in vivo, has been converted to pyroglutamic acid, a peptide wherein a substituent on an amino acid side chain in the molecule is protected by an appropriate protecting group, a conjugated peptide such as what is called a glycopeptide, which has a sugar chain bound thereto, and the like, as with the above-described ASK1.

As the salt of the ASK1 of the present invention or a partial peptide thereof, a physiologically acceptable salt with an acid or a base can be mentioned, with preference given to a physiologically acceptable acid addition salt. Useful salts include, for example, salts with inorganic acids (for example, hydrochloric acid, phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The ASK1 of the present invention or a salt thereof can be produced from cells or a tissue of the aforementioned warm-blooded animal by a method of protein purification known *per se*. Specifically, ASK1 or a salt thereof can be produced by homogenizing a tissue or cells of a warm-blooded animal, and separating and purifying the soluble fraction by a chromatography such as reversed-phase chromatography, ion exchange chromatography or affinity chromatography, and the like.

The ASK1 or a partial peptide thereof can also be produced according to a publicly known peptide synthesis process.

The peptide synthesis process may be any of, for example, a solid phase synthesis process and a liquid phase synthesis process. A desired protein can be produced by condensing a partial peptide or amino acids capable of constituting ASK1 with the remaining portion, and removing the protecting group if any in the resultant product.

Here, the condensation and the removal of the protecting group are conducted according to methods known *per se,* for example, methods described in [1] to [5] below.
[1] M. Bodanszky and M.A. Ondetti: Peptide Synthesis, Interscience Publishers, New York (1966)
[2] Schroeder and Luebke: The Peptide, Academic Press, New York (1965)
[3] Nobuo Izumiya, et al.: Peptide Gosei-no-Kiso to Jikken, published by Maruzen Co. (1975);
[4] Haruaki Yajima and Shunpei Sakakibara: Seikagaku Jikken Koza 1, Tanpakushitsu no Kagaku IV, 205 (1977)
[5] Haruaki Yajima, ed.: Zoku Iyakuhin no Kaihatsu, Vol. 14, Peptide Synthesis, published by Hirokawa Shoten.

The ASK1 or a partial peptide thereof thus obtained can be isolated and purified by a publicly known method of purification. Here, as examples of the method of purification, solvent extraction, distillation, column chromatography, liquid chromatography, recrystallization, a combination thereof, and the like can be mentioned.

When the ASK1 or a partial peptide thereof obtained by the above-described method is a free form, the free form can be converted to an appropriate salt by a publicly known method or a method based thereon; conversely, when the ASK1 or a partial peptide thereof is obtained in the form of a salt, the salt can be converted to a free form or another salt by a publicly known method or a method based thereon.

For the synthesis of the ASK1 or a partial peptide thereof, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such a resin, an amino acid having an appropriately protected α-amino group and side chain functional group is condensed on the resin in accordance with the sequence of the desired protein or peptide according to various methods of condensation known *per se.* At the end of the reaction, the protein (peptide) is cleaved from the resin, various protecting groups are removed simultaneously, and a reaction to form an intramolecular disulfide bond is carried out in a highly diluted solution to obtain the desired protein (peptide) or an amide thereof.

For the above-described condensation of protected amino acids, various activation reagents useful for protein synthesis can be used, with preference given to a carbodiimide. As the carbodiimide, DCC, N,N'-diisopropylcarbodiimide, N-ethyl-N'-(3-dimethylaminoprolyl)carbodiimide and the like can be used. For the activation using these carbodiimides, the protected amino acid, along with a racemation-suppressing additive (for example, HOBt, HOOBt), may be added directly to the resin, or the protected amino acid may be activated in advance as a symmetric acid anhydride, or HOBt ester or HOOBt ester and then added to the resin.

A solvent used for activation of protected amino acids and condensation of protected amino acids with a resin can be appropriately selected from among solvents that are known to be usable for protein condensation reactions. Examples of such useful solvents include acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethyl sulfoxide; amines such as pyridine; ethers such as dioxane and tetrahydrofuran; nitriles such as acetonitrile and propionitrile; esters such as methyl acetate and ethyl acetate; suitable mixtures thereof; and the like. Reaction temperature is appropriately selected from the range that is known to be usable in protein binding reactions, and is normally from the range of about -20°C to about 50°C. An activated amino acid derivative is normally used from 1.5 to 4 times in excess. When the condensation is insufficient as the result of the test using a ninhydrin reaction, sufficient condensation can be carried out by repeating the condensation reaction without elimination of the protecting group. If the condensation is insufficient even though the condensation reaction is repeated, unreacted amino acids can be acetylated by using acetic anhydride or acetylimidazole.

A protecting method and a protecting group of a functional group that should not been involved in the reaction of raw materials, a method of removing the protecting group, a method of activating a functional group involved in the reaction, and the like can be appropriately selected from among publicly known groups or publicly known means.

As the protecting group for the amino group of the starting material, for example, Z, Boc, tertiary pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc and the like can be used.

The carboxyl group can be protected by, for example, alkyl esterification (for example, linear, branched or cyclic alkyl esterification with methyl, ethyl, propyl, butyl, tertiary butyl, cyclopentyl, cyclohexyl, cycloheptyl, cyclooctyl, 2-adamantyl, and the like), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, tertiary butoxycarbonyl hydrazidation, trityl hydrazidation, and the like.

The hydroxyl group of serine can be protected by, for example, esterification or etherification. As the group suitable for this esterification, for example, lower alkanoyl groups such as an acetyl group, aroyl groups such as a benzoyl group, and groups derived from carbonic acid such as benzyloxycarbonyl group, ethoxycarbonyl group and the like can be used. In addition, as examples of the group suitable for etherification, for example, a benzyl group, a tetrahydropyranyl group, a t-butyl group and the like can be mentioned.

As the protecting group for the phenolic hydroxyl group of tyrosine, for example, Bzl, Cl₂-Bzl, 2-nitrobenzyl, Br-Z, tertiary butyl and the like can be used.

As the protecting group for the imidazole of histidine, for example, Tos, 4-methoxy-2,3,6-trimethylbenzenesulfonyl, DNP, benzyloxymethyl, Bum, Boc, Trt, Fmoc and the like can be used.

As the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like, for example, can be used. The elimination reaction by the above-described acid treatment is generally carried out at a temperature of about -20°C to about 40°C; the acid treatment is efficiently conducted by adding a cation scavenger such as, for example, anisole, phenol, thioanisole, m-cresol, p-cresol, dimethyl sulfide, 1,4-butanedithiol or 1,2-ethanedithiol. Also, a 2,4-dinitrophenyl group used as a protecting group for the imidazole of histidine is removed by thiophenol treatment; a formyl group used as a protecting group for the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

As the raw material having an activated carboxyl group, for example, a corresponding acid anhydride, an azide, an activated ester [an ester with an alcohol (for example, pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like can be used. As the raw material having an activated amino group, for example, a corresponding phosphoric amide can be used.

As another method for obtaining an amide of a protein (peptide), for example, a method comprising protecting the α-carboxyl group of each C-terminal amino acid of partial peptide constituting a protein (peptide) by amidation, extending peptide chain to the amino group side in a desired chain length (amino acid to be joined with C-terminal amino acid of adjacent partial peptide). Producing a peptide only without α-amino-protecting group of N-terminal amino acid of C-terminal side peptide chain, and a peptide only without carboxyl group-protecting group of C-terminal amino acid of N-terminal side peptide chain, and condensing these peptides in the above-mentioned mixed solvent can be mentioned. The details of the condensation reaction are as mentioned above. After purification of the protected protein (protected peptide) obtained by condensation, the protecting group is eliminated by the above-mentioned method to give a desired crude protein (crude peptide). The crude protein (crude peptide) is purified by various known purification means, and the main fraction is lyophilized to give an amide of the desired protein (peptide).

An ester of the protein (peptide) can be obtained, for example, by condensing the α-carboxyl group of C-terminal amino acid with a desired alcohol to give an amino acid ester, and treating the ester in the same manner as in the above-mentioned amide.

The partial peptide of the present invention or a salt thereof can also be produced by cleaving ASK1 or a salt thereof with an appropriate peptidase.

Moreover, ASK1 or a partial peptide thereof of the present invention can also be produced by culturing a transformant having the nucleic acid encoding same, and separating and purifying ASK1 or a partial peptide thereof from the obtained culture.

The nucleic acid encoding ASK1 or a partial peptide thereof may be a DNA or an RNA, or DNA/RNA chimera. DNA is preferable. The nucleic acid may be a double stranded or a single strand. In the case of a double stranded, it may be a double stranded DNA, a double stranded RNA or a DNA:RNA hybrid. In the case of a single strand, it may be a sense strand (i.e., coding strand) or an antisense chain (i.e., noncoding strand).

As the DNA encoding ASK1 or a partial peptide thereof, genomic DNA, cDNA derived from any cell [for example, splenocyte, nerve cell, glial cell, pancreatic β cells, myeloid cell, mesangial cell, Langerhans' cell, epidermal cell, epithelial cell, endothelial cell, fibroblast, fibrocyte, myocytes, adipocyte, immune cell (e.g., macrophage, T cell, B cell, natural killer cell, mast cell, neutrophil, basophil, eosinophils, monocyte), megakaryocyte, synovial cell, chondrocytes, bone cell, osteoblast, osteoclast, mammary cell, hepatocyte or interstitial cell, or a corresponding precursor cell, stem cell, cancer cell and the like, blood cells] of warm-blooded animal (e.g., human, mouse, rat, guinea pig, hamster, rabbit, sheep, goat, swine, bovine, horse, bird, cat, dog, monkey, chimpanzee and the like), or any tissue where such cells are present [e.g., brain or any portion of brain (e.g., olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, lateral lobe, putamen, caudate nucleus, callosum, substantia nigra), spinal cord, hypophysis, stomach, pancreas, kidney, liver, gonad, thyroid, gallbladder, bone marrow, adrenal gland, skin, muscle (e.g., skeletal muscle, smooth muscle), lung, gastrointestinal tract(e.g., large intestine, small intestine), blood vessel, heart, thymus, spleen, submandibular gland, peripheral blood, peripheral blood cell, prostate, testicle, ovary, placenta, uterus, bone, joint, adipose tissue and the like], synthetic DNA and the like can be mentioned. A genomic DNA and cDNA encoding ASK1 or a partial peptide thereof can also be directly amplified by Polymerase Chain Reaction (hereinafter to be abbreviated as "PCR method") or Reverse Transcriptase-PCR (hereinafter to be abbreviated as "RT-PCR method"), using genomic DNA fraction or whole RNA or mRNA fraction prepared from the above-mentioned cell·tissue as a template. Alternatively, genomic DNA or cDNA encoding ASK1 or a partial peptide thereof can also be cloned by colony or plaque hybridization method, PCR method and the like, from the genomic DNA library or cDNA library prepared by inserting, into a suitable vector, a fragment of genomic DNA and whole RNA or mRNA prepared from the above-mentioned cell·tissue. The vector to be used for the library may be any of bacteriophage, plasmid, cosmid, phagemid and the like.

As examples of the DNA that encodes the ASK1, a DNA that has the base sequence shown by SEQ ID NO:1, a DNA that has a base sequence hybridizing to the base sequence shown by SEQ ID NO:1 under high stringent conditions, and that encodes the aforementioned protein having substantially the same quality of activity (e.g., activity to promote the activation of the ASK1 cascade, activity to induce apoptosis, and the like) as a protein that has the amino acid sequence shown by SEQ ID NO:2, and the like can be mentioned.

As the DNA capable of hybridizing to the base sequence shown by SEQ ID NO:1 under high stringent conditions, a DNA that has a base sequence having a homology of about 60% or more, preferably about 70% or more, more preferably about 80% or more, and particularly preferably about 90% or more, to the base sequence shown by SEQ ID NO:1, and the like, for example, can be used.

The homology of the base sequence in the present specification can be calculated using the homology calculation algorithm NCBI BLAST (National Center for Biotechnology Information Basic Local Alignment Search Tool) and under the following conditions (expectancy =10; allowing gap; filtering=ON; match score=1; mismatch score=-3). As other algorithm with which to determine the homology of the base sequence, the homology calculation algorithm of the above-mentioned amino acid sequence can be preferably used in the same manner.

Hybridization can be conducted according to a method known *per se* or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached to the library. Hybridization can preferably be conducted under high stringent conditions.

As the high stringent conditions, for example, the conditions of sodium salt concentration of about 19 - about 40 mM, preferably about 19 - about 20 mM, a temperature of about 50°C - about 70°C, preferably about 60°C - about 65°C, and the like can be mentioned. Particularly, a sodium salt concentration of about 19 mM and a temperature of about 65°C are preferable. Those of ordinary skill in the art can easily adjust to desired stringency by appropriately changing the salt concentration of hybridization solution, hybridization reaction temperature, probe concentration, probe length, number of mismatch, hybridization reaction time, salt concentration of washing, washing temperature and the like.

The DNA encoding ASK1 is preferably human ASK1 DNA (GenBank Accession NO: D84476) having the base sequence shown by SEQ ID NO: 1, or its allele variant (e.g., GenBank Accession NO: NM_005923), or ortholog [e.g., as mouse ortholog, GenBank Accession NO: AB006787 (having about 86% homology with the base sequence shown by SEQ ID NO: 1) and the like can be mentioned] of other warm-blooded animal (e.g., mouse, rat, guinea pig, hamster, rabbit, sheep, goat, swine, bovine, horse, bird, cat, dog, monkey, chimpanzee and the like).

The DNA that encodes the partial peptide of the present invention may be any one that has the base sequence that encodes the same or substantially the same amino acid sequence as a part of the amino acid sequence shown by SEQ ID NO:2. The DNA may be any of a genomic DNA, a cDNA derived from the above-described cell or tissue, and a synthetic DNA.

Specifically, as the DNA that encodes the partial peptide of the present invention,
(1) a partial base sequence of a DNA having the base sequence shown by SEQ ID NO:1,
(2) a DNA having a base sequence hybridizing to a DNA having the base sequence shown by SEQ ID NO:1 under high stringent conditions, and that encodes a peptide having:
   (a) substantially the same quality of activity (e.g., activity to promote the activation of the ASK1 cascade, activity to induce apoptosis, and the like) as that of a protein that has the amino acid sequence encoded by the DNA or
   (b) activity to inhibit the activity of a protein that has the amino acid sequence encoded by the DNA (e.g., activity to inhibit the activation of the ASK1 cascade, activity to suppress apoptosis, and the like) and the like, for example, can be used.

As the DNA capable of hybridizing to the DNA having the base sequence shown by SEQ ID NO:1 under high stringent conditions, a DNA that has a base sequence having a homology of about 60% or more, preferably about 70% or more, more preferably about 80% or more, and particularly preferably about 90% or more, to the corresponding portion in the base sequence, and the like, for example, can be used.

The DNA that encodes the ASK1 or a partial peptide thereof can be cloned by amplifying it by the PCR method using a synthetic DNA primer having a portion of the base sequence that encodes the protein or peptide, or by hybridizing DNA incorporated in an appropriate expression vector to a labeled DNA fragment or synthetic DNA that encodes a portion or the entire region of the protein of the present invention. Hybridization can be conducted according to, for example, a method described in Molecular Cloning, 2nd edition (ibidem) and the like. When a commercially available library is used, hybridization can be conducted according to the method described in the instruction manual attached to the library.

The base sequence of DNA can be converted according to a method known *per se,* such as the ODA-LA PCR method, the Gapped duplex method, the Kunkel method and the like, or a method based thereon, using a publicly known kit, for example, Mutan™-super Express Km (Takara Shuzo Co., Ltd.), Mutan™-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

The protein or peptide can be produced by transforming a host with an expression vector containing a DNA encoding the above-mentioned ASK1 or a partial peptide thereof and cultivating the obtained transformant.

An expression vector containing a DNA encoding ASK1 or a partial peptide thereof can be produced, for example, by cleaving out an object DNA fragment from the DNA encoding ASK and connecting the DNA fragment with the downstream of a promoter in a suitable expression vector.

Useful expression vectors include plasmids derived from E. coli (e.g., pBR322, pBR325, pUC12, pUC13); plasmids derived from *Bacillus* subtilis (e.g., pUB110, pTP5, pC194); plasmids derived from yeast (e.g., pSH19, pSH15); bacteriophages such as λ phage; animal viruses such as retrovirus, vaccinia virus and baculovirus; pAl-11, pXT1, pRc/CMV, pRc/RSV, pcDNAI/Neo and the like.

The promoter may be any promoter, as long as it is appropriate for the host used to express the gene.

For example, when the host is an animal cell, the SRα promoter, the SV40 promoter, the LTR promoter, the CMV (cytomegalovirus) promoter, the HSV-TK promoter and the like are used. Of these, the CMV promoter, the SRα promoter and the like are preferred.

When the host is a bacterium of the genus *Escherichia,* the trp promoter, the lac promoter, the recA promoter, the λP_{L} promoter, the lpp promoter, the T7 promoter and the like are preferred.

When the host is a bacterium of the genus *Bacillus,* the SPO1 promoter, the SPO2 promoter, the penP promoter and the like are preferred.

When the host is yeast, the PHO5 promoter, the PGK promoter, the GAP promoter, the ADH promoter and the like are preferred.

When the host is an insect cell, the polyhedrin prompter, the P10 promoter and the like are preferred.

Useful expression vectors include, in addition to the above, expression vectors that optionally comprises an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance), and the like can be mentioned. In particular, when a dhfr gene defective Chinese hamster cell is used and the dhfr gene is used as the selection marker, a target gene can also be selected using a thymidine-free medium.

Where necessary, a base sequence encoding (signal codon) a signal sequence suitable for the host may be added to the 5' end side of DNA encoding ASK1 or a partial peptide thereof. When the host is the genus *Escherichia,* PhoA·signal sequence, OmpA·signal sequence and the like are used; when the host is the genus Bacillus, α-amylase·signal sequence, subtilisin·signal sequence and the like are used; when the host is yeast, MFα· signal sequence, SUC2·signal sequence and the like are used; and when the host is an animal cell, insulin·signal sequence, α-interferon·signal sequence, antibody molecule·signal sequence and the like are used.

As useful examples of the host, a bacterium of the genus *Escherichia,* a bacterium of the genus *Bacillus,* yeast, an insect cell, an insect, an animal cell, and the like can be mentioned.

As useful examples of the bacterium of the genus *Escherichia, Escherichia* coli K12 DH1 *(*Proc. Natl. Acad. Sci. U.S.A., Vol. 60, 160 (1968)), JM103 *(*Nucleic Acids Research, Vol. 9, 309 (1981)), JA221 *(*Journal of Molecular Biology, Vol. 120, 517 (1978)), HB101 *(*Journal of Molecular Biology, Vol. 41, 459 (1969)), C600 *(*Genetics, Vol. 39, 440 (1954)), and the like can be mentioned.

As useful examples of the bacterium of the genus *Bacillus, Bacillus* subtilis MI114 *(*Gene, Vol. 24, 255 (1983)), 207-21 *(*Journal of Biochemistry, Vol. 95, 87 (1984)) and the like can be mentioned.

As useful examples of the yeast, *Saccharomyces cerevisiae* AH22, AH22R⁻, NA87-11A, DKD-5D and 20B-12, *Schizosaccharomyces pombe* NCYC1913, and NCYC2036, *Pichia pastoris* KM71 and the like can be mentioned.

As useful examples of the insect cell, *Spodoptera frugiperda* cell (Sf cell), MG1 cell derived from the mid-intestine of *Trichoplusia ni,* High Five^{™} cell derived from an egg of *Trichoplusia ni,* cell derived from *Mamestra brassicae,* cell derived from *Estigmena acrea,* and the like can be mentioned when the virus is AcNPV. When the virus is BmNPV, useful insect cells include *Bombyx mori* N cell (BmN cell) and the like. As useful examples of the Sf cell, Sf9 cell (ATCC CRL1711), Sf21 cell (both in Vaughn, J. L. et al., In Vivo, 13, 213-217 (1977), and the like can be mentioned.

As useful examples of the insect, a larva of Bombyx mori (Maeda et al., Nature, 315, 592 (1985)), and the like can be mentioned.

As useful examples of the animal cell, monkey cell COS-7, Vero, Chinese hamster cell CHO (hereafter abbreviated as CHO cell), dhfr gene defective Chinese hamster cell CHO (hereafter abbreviated as CHO (dhfr⁻) cell), mouse L cell, mouse AtT-20, mouse myeloma cell, rat GH3, human FL cell and the like can be mentioned.

Transformation can be carried out according to the kind of host in accordance with a publicly known method.

A bacterium of the genus *Escherichia* can be transformed, for example, in accordance with a method described in Proc. Natl. Acad. Sci. U.S.A., 69, 2110 (1972), Gene, 17, 107 (1982), and the like.

A bacterium of the genus *Bacillus* can be transformed, for example, according to a method described in Molecular and General Genetics, 168, 111 (1979), and the like.

Yeast can be transformed, for example, in accordance with a method described in Methods in Enzymology, 194, 182-187 (1991), Proc. Natl. Acad. Sci. USA, 75, 1929 (1978), and the like.

An insect cell and an insect can be transformed, for example, according to a method described in Bio/Technology, 6, 47-55 (1988), and the like.

An animal cell can be transformed, for example, in accordance with a method described in Saibo Kogaku, extra issue 8, Shin Saibo Kogaku Jikken Protocol, 263-267 (1995), published by Shujunsha, or virology, 52, 456 (1973).

Cultivation of a transformant can be carried out according to the kind of host in accordance with a publicly known method.

For example, when a transformant whose host is a bacterium of the genus *Escherichia* or the genus *Bacillus* is cultivated, the culture medium is preferably a liquid medium. Also, the medium preferably comprises a carbon source, a nitrogen source, an inorganic substance, and the like, necessary for the growth of the transformant. Here, as examples of the carbon source, glucose, dextrin, soluble starch, sucrose, and the like can be mentioned; as examples of the nitrogen source, inorganic and organic substances such as an ammonium salt, a nitrate salt, corn steep liquor, peptone, casein, meat extract, soybean cake, potato extract, and the like can be mentioned; as examples of the inorganic substance, calcium chloride, sodium dihydrogen phosphate, magnesium chloride, and the like can be mentioned. In addition, the medium may be supplemented with yeast extract, vitamins, growth promoting factor, and the like. Preferably, the pH of the medium is about 5 to 8.

Examples of the medium used to cultivate a transformant whose host is a bacterium of the genus *Escherichia* include a M9 medium supplemented with glucose and a Casamino acid (Miller, Journal of Experiments in Molecular Genetics, 431-433, Cold Spring Harbor Laboratory, New York, 1972). As required, in order to increase promoter efficiency, a chemical such as 3β-indolylacrylic acid may be added to the medium.

Cultivation of a transformant whose host is a bacterium of the genus *Escherichia* is normally carried out at about 15°C to 43°C for about 3 to 24 hours. As necessary, the culture may be aerated or agitated.

Cultivation of a transformant whose host is a bacterium of the genus *Bacillus* is normally carried out at about 30°C to 40°C for about 6 to 24 hours. As necessary, the culture may be aerated or agitated.

As examples of the medium for cultivating a transformant whose host is yeast, Burkholder's minimum medium [Bostian, K.L. et al., Proc. Natl. Acad. Sci. USA, 77, 4505 (1980)] and SD medium supplemented with 0.5% casamino acid [Bitter, G.A. et al., Proc. Natl. Acad. Sci. USA, 81, 5330 (1984)] can be mentioned. The medium's pH is preferably about 5 to 8. Cultivation is normally carried out at about 20°C to 35°C for about 24 to 72 hours. As necessary, the culture may be aerated or agitated.

Useful medium for cultivating a transformant whose host is an insect cell or an insect include, for example, Grace's Insect Medium [Grace, T.C.C., Nature, 195, 788 (1962)] supplemented with additives such as inactivated 10% bovine serum as appropriate. The medium's pH is preferably about 6.2 to 6.4. Cultivation is normally carried out at about 27°C for about 3 to 5 days. As necessary, the culture may be aerated or agitated.

Useful medium for cultivating a transformant whose host is an animal cell include, for example, MEM medium supplemented with about 5 to 20% fetal bovine serum [Science, 122, 501(1952)], DMEM medium [Virology, 8, 396(1959)], RPMI 1640 medium [The Journal of the American Medical Association, 199, 519(1967)], 199 medium [Proceeding of the Society for the Biological Medicine, 73, 1(1950)] and the like. The medium's pH is preferably about 6 to 8. Cultivation is normally carried out at about 30°C to 40°C for about 15 to 60 hours. As necessary, the culture may be aerated or agitated.

As described above, the ASK1 or a partial peptide thereof can be produced in or outside the cells of the transformant.

The ASK1 or a partial peptide thereof can be separated and purified from the culture obtained by cultivating the aforementioned transformant according to a method known *per se*.

For example, when the ASK1 or a partial peptide thereof is extracted from cultivated bacteria or cells, a method is used as appropriate wherein the bacteria or cells are recovered from the culture by a known means, suspended in an appropriate buffer solution, and disrupted by means of sonication, lysozyme and/or freeze-thawing and the like, after which a crude extract of soluble protein is obtained by centrifugation or filtration. The buffer solution may contain a protein denaturant such as urea or guanidine hydrochloride and a surfactant such as Triton X-100™.

Isolation and purification of the ASK1 or a partial peptide thereof contained in the thus-obtained soluble fraction can be conducted according to a method know *per se.* Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

When the thus-obtained ASK1 or a partial peptide thereof is a free form, the free form can be converted to a salt by a method known *per se* or a method based thereon; when the protein or the peptide is obtained as a salt, the salt can be converted to a free form or another salt by a method known per se or a method based thereon.

Note that the ASK1 or a partial peptide thereof produced by the transformant can also be optionally modified by the action of an appropriate protein-modifying enzyme, before or after purification, or can have a polypeptide thereof removed partially. As such, useful protein-modifying enzymes include, for example, trypsin, chymotrypsin, arginyl endopeptidase, protein kinase, glycosidase and the like.

The presence of the thus-obtained ASK1 or a partial peptide thereof can be confirmed by enzyme immunoassay, Western blotting and the like employing a specific antibody.

Furthermore, the ASK1 or a partial peptide thereof can also be synthesized by in vitro translation using a cell-free protein translation system that comprises a rabbit reticulocyte lysate, wheat germ lysate, *Escherichia coli* lysate and the like, with RNA corresponding to the above-described DNA that encodes ABP1 or a partial peptide thereof as the template. Alternatively, the ASK1 or a partial peptide thereof can be synthesized using a cell-free transcription/translation system containing RNA polymerase, with the DNA that encodes the ASK1 or a partial peptide thereof as the template. As the cell-free protein (transcription/) translation system, commercially available ones can be used, a method known *per se,* and specifically, an *Escherichia* coli extract can also be prepared by the method described in Pratt J. M. et al., Transcription and Tranlation, 179-209, Hames B. D. & Higgins S. J. eds., IRL Press, Oxford (1984) and the like. As the commercially available cell lysates, as those derived from *Escherichia* coli, *E. coli* S30 extract system (manufactured by Promega), RTS 500 Rapid Tranlation System (manufactured by Roche) and the like can be mentioned, as those derived from rabbit reticulocyte, Rabbit Reticulocyte Lysate System (manufactured by Promega) and the like can be mentioned, and as those derived from wheat germ, PROTEIOS^{™} (manufactured by TOYOBO) and the like can be mentioned. Of these, ones using a wheat germ lysate are preferable. As the production method of wheat germ lysate, for example, the methods described in Johnston F.B. et al., Nature, 179: 160-161 (1957) or Erickson A.H. et al., Meth. Enzymol., 96: 38-50 (1996) and the like can be used.

As the system or apparatus for protein synthesis, batch method (Pratt, J.M. et al. (1984), mentioned above), continuous cell-free protein synthesis system (Spirin A.S. et al., Science, 242: 1162-1164 (1988)) wherein amino acid, energy source and the like are continuously supplied to the reaction system, dialysis (Kikawa et al., The 21st Annual Meeting of the Molecule Biology Society of Japan, WID6), or overlay method (manual of PROTEIOS^{™} Wheat germ cell-free protein synthesis core kit: manufactured by TOYOBO) and the like can be mentioned. Moreover, a method (JP-A-2000-333673) wherein template RNA, amino acid, energy source and the like are supplied to a synthesis reaction system as necessary and a synthesized substance and decomposed product are discharged as necessary, and the like can be used.

The nucleic acid containing "a base sequence encoding a protein having an amino acid sequence the same as or substantially the same as the amino acid sequence shown by SEQ ID NO: 2 or a part thereof", or "a base sequence complementary to the base sequence or a part thereof" is used in the sense including not only a nucleic acid having the aforementioned ASK1 or a partial peptide thereof, but also a base sequence having a mismatching codon reading frame.

A nucleic acid having a base sequence complementary to the target region of the object nucleic acid, namely, nucleic acid capable of hybridizing to the object nucleic acid can be said to be "antisense" to the object nucleic acid. On the other hand, a nucleic acid having a base sequence having homology with the target region of the object nucleic acid can be said to be "sense" to the object nucleic acid. Here, "having homology" or "complementary to" means having homology or complementarity of not less than about 70%, preferably not less than about 80%, more preferably not less than about 90%, most preferably not less than about 95%, between base sequences.

A nucleic acid having a base sequence complementary to a base sequence encoding ASK1 or a part thereof (hereinafter to be also referred to as "antisense nucleic acid of the present invention"), can be designed and synthesized on the basis of information on the cloned or determined base sequence of the nucleic acid encoding ASK1. Such a nucleic acid is capable of inhibiting the replication or expression of the gene that encodes ASK1. Hence, the antisense nucleic acid of the present invention is capable of hybridizing to the RNA transcribed from the gene that encodes ASK1, and capable of inhibiting the synthesis (processing) or function (translation into protein) of mRNA.

The target region of the antisense nucleic acid of the present invention is not subject to limitation as to the length thereof, as long as hybridization of the antisense nucleic acid results in the inhibition of the translation of ASK1 protein, and can be the entire sequence or a partial sequence of the mRNA that encodes the protein; a partial sequence of about 15 bases for the shortest, and the entire sequence of the mRNA or initial transcription product for the longest, can be mentioned. Considering the ease of synthesis and the issue of antigenicity, an oligonucleotide consisting of about 15 to about 30 bases is preferred, which, however, is not to be construed as limiting. Specifically, although the 5'-end hairpin loop, the 5'-end 6-base-pair repeat, the 5'-end untranslated region, the translation initiation codon, the protein-coding region, the translation stop codon, the 3'-end untranslated region, the 3'-end palindrome region, and the 3'-end hairpin loop of the nucleic acid that encodes ASK1 can be selected as the target region, any region encoding ASK1 within the gene can be selected as the target. For example, it is also preferable that the intron portion of the gene be the target region.

Furthermore, an antisense nucleic acid of the present invention may be capable of not only hybridizing to the mRNA or the initial transcription product that encodes ASK1 to inhibit the translation to protein, but also binding to an ASK1-encoding gene that is a double-stranded DNA to form a triple strand (triplex) and inhibit the transcription of RNA.

As the antisense nucleic acid, a deoxyribonucleotide containing 2-deoxy-D-ribose, a ribonucleotide containing D-ribose, another type of nucleotide that is an N-glycoside of the purine or pyrimidine base, or another polymer having a non-nucleotide backbone (for example, commercially available protein nucleic acids and synthetic sequence specific nucleic acid polymers) or another polymer having a special bond (however, this polymer contains a nucleotide having a configuration that allows base pairing or base attachment as found in DNA and RNA) and the like can be mentioned. These may be double-stranded DNAs, single-stranded DNAs, double-stranded RNAs or single-stranded RNAs, or DNA:RNA hybrids, and may also be non-modified polynucleotides (or non-modified oligonucleotides), those having a known modification added thereto, for example, those with a label known in the relevant field, those with a cap, those methylated, those having 1 or more naturally occurring nucleotides substituted by analogues, those modified with an intramolecular nucleotide, for example, those having a non-charge bond (for example, methylphosphonate, phosphotriester, phosphoramidate, carbamate and the like), those having a charged bond or a sulfur-containing bond (for example, phosphorothioate, phosphorodithioate and the like), for example, those having a side chain group of a protein (nuclease, nuclease inhibitor, toxin, antibody, signal peptide, poly-L-lysine and the like), a sugar (for example, monosaccharide and the like) and the like, those having an intercalating compound (for example, acridine, psoralen and the like), those containing a chelate compound (for example, metals, radioactive metals, boron, oxidizing metals and the like), or those containing an alkylating agent, those having a modified bond (for example, α anomer type nucleic acid and the like). Here, "nucleoside", "nucleotide" and "nucleic acid" may include not only those containing the purine and pyrimidine bases, but also those containing another modified heterocycle type base. These modified products may contain a methylated purine and pyrimidine, an acylated purine and pyrimidine, or another heterocycle. The modified nucleotide and the modified nucleotide may also have their sugar portion modified by, for example, substitution of 1 or more hydroxyl groups by a halogen, an aliphatic group and the like, or conversion to a functional group such as an ether or an amine.

Preferably, the antisense nucleic acid is optionally modified RNA or DNA. As specific examples of the modified nucleic acid (RNA, DNA), sulfur derivatives and thiophosphate derivatives of nucleic acids, and those resistant to the decomposition like polynucleosideamide or oligonucleosideamide can be mentioned, which, however, are not to be construed as limiting. The antisense nucleic acid of the present invention can preferably be designed to accomplish one of the following purposes: to make the antisense nucleic acid more stable in the cell, to increase the cell permeability of the antisense nucleic acid, to increase the affinity for the desired sense strand, and to reduce the toxicity, if any, of the antisense nucleic acid. Many such modifications are known in the relevant field, and are disclosed in, for example, J. Kawakami et al., Pharm Tech Japan, Vol. 8, pp. 247, 1992; Vol. 8, pp. 395, 1992; S. T. Crooke et al. ed., Antisense Research and Applications, CRC Press, 1993 and the like.

The antisense nucleic acid may be altered, or may contain an modified sugar, base or bond, and can be supplied in a special form like liposome or microspheres, can be applied for gene therapy, and can be given in an adduct form. As such an adduct form used, a polycation derivative like polylysine, which acts to neutralize the charge of the phosphate backbone, and a hydrophobic one like a lipid that enhances the interaction with cell membrane or increases nucleic acid uptake (for example, phospholipid, cholesterol and the like) can be mentioned. As lipids preferred for addition, cholesterol and derivatives thereof (for example, cholesterylchloroformate, cholic acid and the like) can be mentioned. These can be attached to the 3' end or the 5' end of nucleic acid, and can be attached via a base, a sugar or an intramolecular nucleoside bond. As other groups, a capping group specifically arranged at the 3' end or 5' end of nucleic acid to prevent degradation by a nuclease such as exonuclease or RNase can be mentioned. As such a capping group, hydroxyl group protecting groups known in the relevant field, including glycols such as polyethylene glycol and tetraethylene glycol, can be mentioned, which, however, are not to be construed as limiting.

A ribozyme capable of specifically cleaving the mRNA or the initial transcription product that encodes ASK1 within the coding region (including the intron portion in the case of the initial transcription product) can also be encompassed in the antisense nucleic acid of the present invention. "Ribozyme" refers to RNA possessing an enzyme activity to cleave a nucleic acid, and is herein understood to be used as a concept encompassing DNA, as long as sequence-specific nucleic acid cleavage activity is possessed, since it has recently been found that oligo DNA having the base sequence of the enzyme activity portion also possesses nucleic acid cleavage activity. One of the most versatile ribozymes is self-splicing RNA found in infectious RNAs such as viroid and virusoid, and the hammerhead type, the hairpin type and the like are known. The hammerhead type exhibits enzyme activity with about 40 bases in length, and it is possible to specifically cleave the target mRNA by making several bases at both ends adjoining to the hammerhead structure portion (about 10 bases in total) to be a sequence complementary to the desired cleavage site of the mRNA. Since this type of ribozymes has RNA only as the substrate, they offer an additional advantage of non-attack of genomic DNA. Provided that the ASK1 mRNA takes a double-stranded structure by itself, the target sequence can be made single-stranded, using a hybrid ribozyme prepared by joining an RNA motif derived from a viral nucleic acid that can specifically bind to RNA helicase [Proc. Natl. Acad. Sci. USA, 98(10): 5572-5577 (2001)]. Furthermore, when the ribozyme is used in the form of an expression vector containing the DNA that encodes it, the ribozyme may be a hybrid ribozyme prepared by further joining a sequence modified from the tRNA to promote the migration of the transcription product to cytoplasm *[*Nucleic Acids Res., 29(13): 2780-2788 (2001)].

A double stranded oligo RNA (siRNA) having a base sequence complementary to mRNA encoding ASK1 or a partial sequence (including the intron portion in the case of the initial transcription product) within the coding region of the initial transcription product can be included in the antisense nucleic acid of the present invention. It has been long known that a phenomenon called RNA interference (RNAi) wherein, when a short double stranded RNA is introduced into a cell, mRNA complementary to one chain of RNA is decomposed occurs in nematode, insect, plant and the like. Ever since this phenomenon is confirmed to also occur in a mammalian cell *[*Nature, 411(6836): 494-498 (2001)], it has been widely used as an alternative technique of ribozyme. SiRNA can be appropriately designed using a commercially available software (e.g., RNAi Designer; Invitrogen) based on the base sequence information of the target mRNA.

The antisense oligonucleotide and ribozyme of the present invention can be prepared by determining the target region of the mRNA or initial transcription product on the basis of information on the cDNA sequence or genomic DNA sequence that encodes the ASK1, and synthesizing a sequence complementary thereto using a commercially available DNA/RNA synthesizer (Applied Biosystems, Beckman Instruments, and the like). siRNA having an RNAi activity can be prepared by synthesizing each of a sense strand and an antisense strand using a DNA/RNA synthesizer, denaturing the strands in an appropriate annealing buffer solution at, for example, about 90 to about 95°C for about 1 minute, and then annealing the strands at about 30 to about 70°C for about 1 to about 8 hours. It is also possible to prepare a longer double-stranded polynucleotide by synthesizing complementary oligonucleotide strands in alternative overlaps, annealing the strands, and ligating the strands using ligase.

The gene expression inhibitory activity of the antisense nucleic acid of the present invention can be examined using a transformant containing nucleic acid encoding ASK1, a gene expression system encoding the ASK1 in vivo or in vitro, or an in vivo or in vitro translation system of ASK1. The nucleic acid can be applied to a cell by various methods known *per se.*

The present invention also provides an antibody (hereinafter sometimes to be abbreviated as "the antibody of the present invention") to ASK1 or a partial peptide thereof. The antibody of the present invention may be a monoclonal antibody or a polyclonal antibody, as long as it has specific affinity for the protein or peptide. The antibody of the present invention can be produced according to a production method of antibody or antiserum known *per se,* using ASK1 or a partial peptide thereof as an antigen.

### [Preparation of monoclonal antibody]

### Preparation of monoclonal antibody-producing cells

The ASK1 or a partial peptide thereof, as is or along with a carrier or a diluent, is administered to a mammal at a site permitting antibody production by administration. To increase antibody productivity in this administration, complete Freund's adjuvant and incomplete Freund's adjuvant may be administered. The administration is normally conducted every 2 to 6 weeks, in a total of about 2 to 10 times. As examples of the mammal used, monkey, rabbit, dog, guinea pig, mouse, rat, sheep, goat, and the like can be mentioned, and a mouse and a rat are preferably used.

For example, a monoclonal antibody-producing hybridoma can be prepared by selecting an individual with an antibody titer from among antigen-immunized mammals, for example, mice, recovering the spleen or a lymph node 2-5 days after final immunization, and fusing an antibody-producing cell contained therein with an allogeneic or heterogeneous myeloma cell. A measurement of antibody titer in the antiserum can be conducted by, for example, reacting the labeled ASK1 described below and an antiserum, and thereafter measuring the activity of the labeling agent bound to the antibody. The fusion procedure can be performed according to a known method, for example, the method of Köhler and Milstein [Nature, 256, 495 (1975)]. As examples of a fusogen, polyethylene glycol (PEG), Sendai virus and the like can be mentioned, and PEG is preferably used.

As examples of the myeloma cell, mammalian myeloma cells such as NS-1, P3U1, SP2/O and AP-1 can be mentioned, and P3U1 is preferably used. A preferable ratio of the number of antibody-producing cells (splenocytes) and number of myeloma cells used is about 1:1 to 20:1; cell fusion can be efficiently performed by adding a PEG (preferably PEG1000 to PEG6000) at concentrations of about 10 to 80%, and conducting incubation at 20 to 40°C, preferably at 30 to 37°C, for 1 to 10 minutes.

A monoclonal antibody-producing hybridoma can be screened for by, for example, a method wherein the hybridoma culture supernatant is added to a solid phase (e.g., microplate) having an antigen adsorbed thereto directly or along with a carrier, an anti-immunoglobulin antibody (when the cell used for cell fusion is a mouse cell, an anti-mouse immunoglobulin antibody is used) or protein A labeled with a radioactive substance, an enzyme or the like is then added, and the monoclonal antibody bound to the solid phase is detected; a method wherein the hybridoma culture supernatant is added to a solid phase having an anti-immunoglobulin antibody or protein A adsorbed thereto, ASK1 labeled with a radioactive substance, an enzyme or the like is added, and the monoclonal antibody bound to the solid phase is detected; and the like.

Selection of a monoclonal antibody can be conducted according to a method known *per se* or a method based thereon. Selection of a monoclonal antibody can normally be conducted using an animal cell culture medium supplemented with HAT (hypoxanthine, aminopterin, thymidine). As the medium for selection and breeding of a monoclonal antibody, any medium can be used, as long as the hybridoma can grow therein. For example, an RPMI 1640 medium containing 1 to 20%, preferably 10 to 20%, fetal bovine serum, a GIT medium (Wako Pure Chemical Industries, Ltd.) containing 1 to 10% fetal bovine serum or a serum-free medium for hybridoma culture (SFM-101, Nissui Pharmaceutical Co., Ltd.) and the like can be used. Cultivation temperature is normally 20 to 40°C, preferably about 37°C. Cultivation time is normally 5 days to 3 weeks, preferably 1 week to 2 weeks. Cultivation can normally be conducted under 5% carbonic acid gas. The antibody titer of the hybridoma culture supernatant can be measured in the same manner as the above-described measurement of the antibody titer in the antiserum.

The thus-obtained monoclonal antibody can be separated and purified according to a method known *per se,* for example, a method of immunoglobulin separation and purification [e.g., salting-out method, alcohol precipitation method, isoelectric point precipitation method, electrophoresis method, adsorption and desorption method using an ion exchanger (e.g., DEAE), ultracentrifugation method, gel filtration method, specific purification method wherein only the antibody is recovered using an antigen-binding solid phase or an active adsorbent such as protein A or protein G, and its binding is dissociated to yield the antibody].

### [Preparation of polyclonal antibody]

A polyclonal antibody against ASK1 or a partial peptide thereof can be produced according to a method known *per se.* For example, the polyclonal antibody can be produced by immunizing a mammal with an immunogen (ASK1 or a partial peptide thereof) as is or a complex thereof with a carrier protein in the same manner as the above-described method of monoclonal antibody production, recovering the antibody-containing product of the present invention from the immunized animal, and separating and purifying the antibody.

Regarding the complex of an immunogen and carrier protein used to immunize a mammal, any kind of carrier protein can be crosslinked at any mixing ratio of carrier and hapten, as long as an antibody against the carrier-crosslinked immunized hapten is efficiently produced; for example, a method wherein bovine serum albumin, bovine thyroglobulin, hemocyanin or the like is coupled at a ratio of about 0.1 to 20, preferably about 1 to 5, parts by weight per 1 part by weight of hapten, can be used.

For coupling of a hapten and a carrier, various condensing agents, for example, active ester reagents containing glutaraldehyde, carbodiimide, a maleimide active ester, a thiol group or a dithiopyridyl group, and the like can be used.

The condensation product, as is or along with a carrier or a diluent, is administered to a mammal at a site permitting antibody production. To increase antibody productivity in this administration, complete Freund's adjuvant and incomplete Freund's adjuvant may be administered. The administration is normally conducted every 2 to 6 weeks, in a total of about 3 to 10 times.

A polyclonal antibody can be recovered from blood, ascites fluid and the like, preferably blood, of a mammal immunized by the above-described method.

A measurement of the polyclonal antibody titer in the antiserum can be conducted in the same manner as the above-described measurement of antibody titer in the antiserum. Separation and purification of the polyclonal antibody can be conducted according to the same immunoglobulin separation and purification method as the above-described monoclonal antibody separation and purification.

When the partial peptide of the present invention is used as an antigen, its position on ASK1 is not particularly limited and, for example, an oligopeptide having a partial amino acid sequence of the region well preserved between various warm-blooded animals can be mentioned. Specifically, any partial peptide in a kinase catalytic domain (having about 99% amino acid homology; about 92% amino acid homology for the whole) highly preserved between human and mouse, and the like are preferably shown as examples.

The above-mentioned (1) ASK1 or active peptide of the present invention, (2) a nucleic acid having a base sequence encoding ASK1 or a part thereof, (3) the antibody of the present invention, (4) antisense nucleic acid of the present invention, (5) inhibitory peptide of the present invention has, for example, the following use.

### (1) screening for a substance for the prophylaxis or treatment of apoptosis or an inflammation-associated disease

As mentioned above, ASK1 promotes apoptosis induction and production of inflammatory cytokine. Such fact indicates that a substance capable of regulating (promoting or inhibiting) the expression or activity of ASK1 is effective for the prophylaxis or treatment of apoptosis or an inflammation-associated disease. Here, the "apoptosis or an inflammation-associated disease" means a disease or pathology caused by insufficient apoptosis induction or inflammatory cytokine production or abnormal promotion thereof, or such condition occurring in consequence thereof. As the diseases associated with abnormal promotion of apoptosis induction or inflammatory cytokine production, for example, viral infections (e.g., AIDS, influenza, fever of unknown origin and the like), endocrine diseases (e.g., hormone deficiency, cytokine deficiency and the like), hematological diseases (e.g., hemocytopenia, renal anemia and the like), organ hypoplasia (e.g., thyroid atrophy, cleft palate and the like), organ graft rejection, graft-versus-host disease, immune deficiency, neurodegenerative diseases (e.g., polyglutamine disease, Alzheimer's disease, Parkinson's disease, amyotrophic lateral sclerosis, prion disease, cerebellar degeneration and the like), ischemic heart diseases (e.g., angina pectoris, myocardial infarction and the like), radiation injuries, ultraviolet injuries (e.g., sunburns and the like), poisoning diseases (e.g., renal tubular cell injury by heavy metals, liver cell injury by alcohol, and the like), nutritional disorders (e.g., thymus atrophy due to vitamin or trace element deficiency, and the like), inflammatory diseases (e.g., acute pancreatitis, arthritis, periodontal disease, colitis and the like), ischemic neuropathy, vascular diseases (e.g., arteriosclerosis and the like), respiratory diseases (e.g., interstitial pneumonia, pulmonary fibrosis and the like), articular diseases (e.g., arthritic deformans and the like) and the like, can be mentioned. As the diseases associated with insufficient apoptosis induction or inflammatory cytokine production, cancers (e.g., leukemia, esophageal cancer, gastric cancer, colon cancer, rectal cancer, lung cancer, liver cancer, kidney cancer, breast cancer, uterine cancer, ovarian cancer, prostatic cancer, melanoma, myeloma, osteosarcoma, brain tumor and the like), autoimmune diseases (e.g., systemic lupus erythematosus, scleroderma, rheumatoid arthritis, Sjogren's syndrome, multiple sclerosis, insulin-dependent diabetes, psoriasis, ulcerative colitis, idiopathic thrombocytopenic purpura, Crohn's disease, glomerulonephritis and the like), viral infections (e.g., hemorrhagic fever, T-cell leukemia, Kaposi sarcoma, infectious mononucleosis, lymphoma, epipharyngeal cancer, cervical cancer, skin cancer, hepatitis, liver cancer and the like), endocrine diseases (e.g., hyperhormonal disease, hypercytokine disease and the like), hematological diseases (e.g., hemocytosis, B-cell lymphoma, polycythemia and the like), organ hyperplasia (e.g., hermaphroditism, undescended testis, teratoma, nephroblastoma, polycystic kidney, cardiac/aortic malformations, syndactyly and the like), post-angioplastic restenosis, recurrence after cancer resection, and the like can be mentioned.

As shown in the below-mentioned Examples, inactivation of ASK1 gene decreases the sensitivity of blood glucose increase due to high-fat diet and also suppresses progression of neuropathy in diabetes model. In addition, ASK1 expression in the kidney increases in diabetic nephropathy model. Accordingly, a substance capable of inhibiting the expression or activity of ASK1 (hereinafter sometimes to be comprehensively referred to as "ASK1 inhibitory substance") is particularly effective for the prophylaxis or treatment of diabetes and complications thereof (e.g., diabetic nephropathy, diabetic neuropathy etc.). Since inactivation of ASK1 gene inhibits changes in inflammation in the lung due to tobacco smoke exposure, an ASK1 inhibitory substance is particularly effective for the prophylaxis or treatment of COPD.

Accordingly, the present invention provides a method for screening for a substance for the prophylaxis or treatment of apoptosis or an inflammation-associated disease, diabetes or complications thereof, or COPD, by the use of ASK1 or active peptide of the present invention or a salt thereof (hereinafter sometimes to be comprehensively referred to as "ASK1s").

More specifically, the present invention provides a method for screening for a substance for the prophylaxis or treatment of apoptosis or an inflammation-associated disease, diabetes or complications thereof, or COPD, which comprises
(a) comparing the activity of ASK1s between in the presence of a test substance and in the absence of the test substance.
   The above-mentioned screening method can be largely divided into
   (a-1) a method comprising comparing the isolated ASK1s activity between in the presence of a test substance and in the absence of the test substance, and
   (a-2) a method comprising culturing a cell having the ability to produce ASK1s in the presence of a test substance and in the absence of the test substance and comparing the activity of ASK1s under the both conditions.

ASK1 used for the screening method of the above-mentioned (a-1) or active peptide of the present invention or a salt thereof can be isolated and purified by the above-mentioned method.

As a cell having the ability to produce ASK1 or a salt thereof used for the screening method of the above-mentioned (a-2), a human or other warm-blooded animal cell naturally expressing same or a biological sample (e.g., blood, tissue, organ etc.) containing same can be used without particular limitation. Preferred is one inducing ASK1 expression responsive to the physical or chemical stimuli due to oxidation stress and the like and, for example, HeLa cell, HEK293 cell and the like can be mentioned. In the case of a blood, tissue, organ and the like derived from a non-human animal, it may be isolated from a living organism and cultured, or a test substance may be administered to a living organism and, after a lapse of a give time, its biological sample may be isolated.

As a cell having the ability to produce ASK1 or the active peptide of the present invention or a salt thereof, various transformants prepared by the above-mentioned engineering method can be mentioned.

As examples of the test substance, a protein, a peptide, a non-peptide compound, a synthetic compound, a fermentation product, a cell extract, a plant extract, an animal tissue extract and the like can be mentioned, and these substances may be novel or publicly known.

In the screening method of the above-mentioned (a-1), the ASK1s activity can be measured by detected by phosphorylation of a substrate protein or peptide. Since ASK1 has autophosphorylation activity, addition of other substrate to the reaction system is not essential as long as phosphorylation of ASK1s itself can be detected. Where desired, for example, MAPKK (e.g., MKK4/MKK7, MKK3/MKK6 and the like), which is a physiological substrate protein of ASK1, a fragment (e.g., ASK1 fragment containing a serine residue shown by amino acid NO 83 from the amino acid sequence shown by SEQ ID NO: 2 etc.) containing a target phosphorylation site of ASK1 or the aforementioned substrate protein, or various synthetic peptides known as substrates for assay of serine/threonine kinase and the like can also be used.

The phosphorylation reaction can be carried out, for example, dissolving an ASK1s reference standard product isolated and purified by the aforementioned method in an appropriate buffer [e.g., phosphate buffer, tris-hydrochloric acid buffer (pH about 4 - about 10, desirably pH about 6 - about 8) etc. (as necessary, surfactant such as Triton X-100, CHAPS, Tween-80™, digitonin, deoxycholate and the like, or a protease inhibitor such as PMSF, leupeptin, bacitracin, aprotinin, E-64 (manufactured by Institute for Protein Research), pepstatin and the like may be added)], adding a test substance (or without addition), further adding a phosphoric acid group donor (e.g., ATP etc.) and, as necessary, a substrate protein (peptide), and incubating generally at about 20 to about 40°C, preferably about 30 to about 37°C for about 30 min-several hours.

The phosphorylation can be measured, for example, by reacting a labeled phosphoric acid group donor (e.g., [γ⁻³²P]ATP etc.), and detecting the amount of labeling of ASK1s and/or other substrate protein (peptide) (e.g., when [γ-³²P]ATP is used, the reaction mixture is electrophoresed (e.g., SDS-PAGE) and ³²P uptake of protein (peptide) on the gel is measured by autoradiography). Alternatively, the reaction mixture is contacted with a material (e.g., DE81 membrane, cellulose membrane, PVDF membrane etc.) capable of absorbing a protein (peptide), and the amount of label bonded to the material can be measured. In addition, phosphorylation of the substrate can be detected using changes in the total electric charge or molecular weight as an index. Examples of the former include a method comprising carrying out a phosphorylation reaction using, as a substrate, a synthetic peptide affording a net electric charge of +1, electrophoresing the reaction mixture and detecting the amount of labeling of the peptide moved to the anode side (since the net electric charge of the phosphorylated substrate is -1, the peptide moves to the anode side) and the like can be mentioned. Examples of the latter include a method comprising using a mass analysis, a method using surface plasmon resonance and the like.

When an antibody specific to phosphorylated substrate is available, phosphorylation of a substrate protein (peptide) can also be detected using an antigen-antibody reaction. An antibody against a phosphorylated product of ASK1, MKK4, MKK3 and the like is commercially available (e.g., available from Cell Signaling Technology, New England Biolabs. etc.). For detection of phosphorylation using an anti-phosphorylated protein (peptide) antibody, various immunological measurement methods similar to the screening method to be mentioned below (c) can be used.

The activity of ASK1s in the screening method of the above-mentioned (a-2) can be measured by detecting phosphorylation of a substrate protein (peptide) as in the screening method of the above-mentioned (a-1), as well as by measuring the induction rate of apoptosis or cell death.

When phosphorylation of a substrate protein (peptide) is used as an index, MAPK such as JNK, p38 and the like located further downstream of MKK4, MKK3 can also be utilized as a substrate protein. The phosphorylation reaction can be carried out, for example, by suspending a cell having the ability to produce ASK1s in an appropriate medium (e.g., the aforementioned various media for animal cells) or a buffer [e.g., phosphate buffer, tris-hydrochloride buffer (pH about 4 - about 10, desirably pH about 6 - about 8) etc. (where necessary, surfactant such as Triton X-100, CHAPS, Tween-80™, digitonin, deoxycholate and the like, or a protease inhibitor such as PMSF, leupeptin, bacitracin, aprotinin, E-64 (manufactured by Institute for Protein Research), pepstatin and the like can also be added)], adding a test substance (or without addition), further adding a phosphoric acid group donor (e.g., ATP etc.) and adding, as necessary, a substrate protein (peptide), and incubating generally at about 20°C - about 40°C, preferably about 30°C - about 37°C for about 30 min-several hours.

When ASK1s is localized in a cell, phosphorylation can be measured by suspending the cell in a suitable buffer, disrupting the cell by sonication, freeze-thawing and the like, and treating the obtained cell disrupt solution according to a method similar to the screening method described in the above-mentioned (a-1). When ASK1s is extracellularly secreted, phosphorylation can be measured by treating a cell culture supernatant by a method similar to the screening method described in the above-mentioned (a-1). Where necessary, ASK1s is separated from a disrupt solution or a culture supernatant and purified using, for example, the antibody of the present invention and the like and measured.

When apoptosis (cell death) induction is used as an index, a cell having the ability to produce ASK1s is suspended in an appropriate medium or buffer, a test substance is added (or without addition), a stimuli of oxidization stress (e.g., H₂O₂ addition) and the like is added, the cells are incubated generally at about 20°C - about 40°C, preferably about 30°C - about 37°C for about 6 - about 72 hr, and apoptosis (cell death) induction rate is measured.

Apoptosis (cell death) can be examined, for example, by detecting the membrane blebbing, cell size miniaturization, chromatin condensation, DNA fragmentation and the like by morphological observation using an optical microscope, a phase contrast microscope, a fluorescence microscope and the like. For example, cells are cultured in a multi-well plate, cells separated from the plate or cells showing membrane blebbing are counted under a microscope, and the rate (%) of dead cells in the total cell in the field is calculated. Observation is performed in the number field and the average value thereof is taken as the cell deth-induction rate. In addition, the cell deth-induction rate can be calculated by staining dead cell with a dye such as trypan blue, erythrocin B, negrosin, eosin Y, fluorescent diacetate, acridineorange, ethidium bromide and the like, and counting under a microscope. Moreover, DNA is stained with a fluorescent dye such as DAPI, Hoechst 33342 and the like, and the cells undergoing chromatin condensation can be counted under a fluorescence microscope. Alternatively, dUTP labeled with biotin, fluorescent dye and the like is added to the 3' end of fragmented DNA using terminal·deoxynucleotidyl·transferase (TdT) (TUNEL method), and the number of highly stained cell is counted using an optical microscope, a fluorescence microscope and the like, whereby the apoptosis induction rate can also be calculated.

Moreover, the number of miniaturized or fragmented cells is counted by measuring the cell size distribution using a particle size measuring apparatus (e.g., Coulter multisizer etc.), whereby the apoptosis induction rate can be calculated. Alternatively, living dead cells can be separated and the cell deth-induction rate can also be calculated by detecting miniaturization of cell size or fragmentation into apoptotic body using flow cytometry (FACS).

Furthermore, apoptosis can be biochemically detected by extracting chromosome DNA from a cell by a conventional method, and measuring the level of DNA fragmentation by gel electrophoresis using a densitometer and the like. Alternatively, utilizing reduction of 3-(4,5-dimethyl-thiazol-2-yl)-2,5-diphenyl tetrazolium bromide (MTT) to formazan by living cells, the cell deth-induction rate can be calculated using a microplate reader by measuring a decrease of absorbance at 570-630 nm.

In the screening method of the above-mentioned (a), a substance having an enhanced ASK1 activity can be selected as an "ASK1 activity promoting substance", and a substance having a decreased activity can be selected as an "ASK1 activity inhibitory substance". An ASK1 activity promoting substance can be used as a prophylactic or therapeutic agent for a disease associated with insufficient apoptosis induction or inflammatory cytokine production [such as cancers (e.g., leukemia, esophageal cancer, gastric cancer, colon cancer, rectal cancer, lung cancer, liver cancer, kidney cancer, breast cancer, uterine cancer, ovarian cancer, prostatic cancer, melanoma, myeloma, osteosarcoma, brain tumor and the like), autoimmune diseases (e.g., systemic lupus erythematosus, scleroderma, rheumatoid arthritis, Sjogren's syndrome, multiple sclerosis, insulin-dependent diabetes, psoriasis, ulcerative colitis, idiopathic thrombocytopenic purpura, Crohn's disease, glomerulonephritis and the like), viral infections (e.g., hemorrhagic fever, T-cell leukemia, Kaposi sarcoma, infectious mononucleosis, lymphoma, epipharyngeal cancer, cervical cancer, skin cancer, hepatitis, liver cancer and the like), endocrine diseases (e.g., hyperhormonal disease, hypercytokine disease and the like), hematological diseases (e.g., hemocytosis, B-cell lymphoma, polycythemia and the like), organ hyperplasia (e.g., hermaphroditism, undescended testis, teratoma, nephroblastoma, polycystic kidney, cardiac/aortic malformations, syndactyly and the like), post-angioplastic restenosis, recurrence after cancer resection, and the like].

On the other hand, an ASK1 activity inhibitory substance can be used as a prophylactic or therapeutic agent for a disease associated with abnormal promotion of apoptosis induction or inflammatory cytokine production [such as viral infections (e.g., AIDS, influenza, fever of unknown origin and the like), endocrine diseases (e.g., hormone deficiency, cytokine deficiency and the like), hematological diseases (e.g., hemocytopenia, renal anemia and the like), organ hypoplasia (e.g., thyroid atrophy, cleft palate and the like), organ graft rejection, graft-versus-host disease, immune deficiency, neurodegenerative diseases (e.g., polyglutamine disease, Alzheimer's disease, Parkinson's disease, amyotrophic lateral sclerosis, prion disease, cerebellar degeneration and the like), ischemic heart diseases (e.g., angina pectoris, myocardial infarction and the like), radiation injuries, ultraviolet injuries (e.g., sunburns and the like), poisoning diseases (e.g., renal tubular cell injury by heavy metals, liver cell injury by alcohol, and the like), nutritional disorders (e.g., thymus atrophy due to vitamin or trace element deficiency, and the like), inflammatory diseases (e.g., acute pancreatitis, arthritis, periodontal disease, colitis and the like), ischemic neuropathy, vascular diseases (e.g., arteriosclerosis and the like), respiratory diseases (e.g., interstitial pneumonia, pulmonary fibrosis and the like), articular diseases (e.g., arthritic deformans and the like) and the like].

In addition, an ASK1 activity inhibitory substance can be preferably used as an agent for the prophylaxis or treatment of diabetes or complications thereof (e.g., diabetic nephropathy, diabetic neuropathy etc.), or COPD.

When an ASK1 activity promoting or inhibitory substance is used as the above-mentioned prophylactic or therapeutic agent, it can be formulated according to a conventional means.

For example, an ASK1 activity promoting or inhibitory substance can be used orally as tablets, capsules, elixirs, microcapsules and the like, coated with sugar as required, or can be used non-orally in the form of an injection such as a sterile solution or suspension in water or another pharmaceutically acceptable liquid. For example, a preparation can be produced by blending an ASK1 activity promoting or inhibitory substance with a known physiologically acceptable carrier, a sweetener, a excipient, a vehicle, an antiseptic, a stabilizer, a binder and the like, in a unit dosage form required for generally accepted preparation design. The active ingredient contents in these preparations are intended to ensure that an appropriate dose in the specified range is obtained.

As examples of additives that can be formulated in tablets, capsules and the like, a binder like gelatin, cornstarch, tragacanth and gum arabic, a excipient like crystalline cellulose, a swelling agent like cornstarch, gelatin, alginic acid and the like, a lubricant like magnesium stearate, a sweetener like sucrose, lactose or saccharin, a flavoring agent like peppermint, acamono oil or cherry and the like can be used. When the formulation unit form is a capsule, the above-described type of material can further contain a liquid carrier like an oil or fat. A sterile composition for injection can be formulated according to an ordinary preparation design such as dissolving or suspending an active substance, a naturally produced vegetable oil such as sesame oil or coconut oil, and the like in a vehicle like water for injection. As examples of aqueous solutions for injection, physiological saline, an isotonic solution containing glucose or other auxiliary agent (for example, D-sorbitol, D-mannitol, sodium chloride and the like) and the like can be used, which may be used in combination with an appropriate solubilizer, for example, an alcohol (e.g., ethanol), a polyalcohol (e.g., propylene glycol, polyethylene glycol), a non-ionic surfactant (e.g., polysorbate 80™, HCO-50) and the like. As examples of oily solutions, sesame oil, soybean oil and the like can be used, which may be used in combination with solubilizers benzyl benzoate, benzyl alcohol and the like.

Also, the above-described prophylactic or therapeutic agent may be formulated with, for example, a buffering agent (for example, phosphate buffer solution, sodium acetate buffer solution), a soothing agent (for example, benzalkonium chloride, procaine hydrochloride and the like), a stabilizer (for example, human serum albumin, polyethylene glycol and the like), a preservative (for example, benzyl alcohol, phenol and the like), an antioxidant and the like. The prepared injection solution is normally filled in an appropriate ampoule.

Since the preparation thus obtained is safe and of low toxicity, it can be administered to, for example, a human or another warm-blooded animal (for example, rat, mouse, hamster, rabbit, sheep, goat, swine, bovine, horse, cat, dog, monkey, chimpanzee, bird and the like).

The dosage of the substance for promoting ASK1 activation varies depending on subject of administration, target organ, symptoms, method of administration and the like; in a cancer patient (body weight 60 kg), for example, the usual oral dosage is about 0.1 mg to 100 mg, preferably about 1.0 to 50 mg, more preferably about 1.0 to 20 mg, per day, In the case of non-oral administration, the dosage per administration varies depending on subject of administration, target organ, symptoms, method of administration and the like; in a cancer patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

The dosage of the substance for inhibiting ASK1 activation also varies depending on subject of administration, target organ, symptoms, method of administration and the like; in a diabetes (or complications thereof) or COPD patient (body weight 60 kg), for example, the usual oral dosage is about 0.1 mg to 100 mg, preferably about 1.0 to 50 mg, more preferably about 1.0 to 20 mg, per day. In the case of non-oral administration, the dosage per administration varies depending on subject of administration, target organ, symptoms, method of administration and the like; in a diabetes (or complications thereof) or COPD patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

As one example of the screening method of an ASK1 activity regulating (promoting or inhibiting) substance, using a cell having the ability to produce ASK1s and an anti-phosphorylated ASK1 antibody, a method of measuring autophosphorylation of ASK1s is specifically explained in the following. The screening system can also be used for screening for an ASK1 expression regulating (promoting or inhibiting) substance using an anti-ASK1 antibody.

### [Reagent for screening]

1) medium
   Dulbecco's modified Eagle medium (DMEM) containing 4.5 mg/mL glucose, which is sterilized by filtration through a filter (pore size 0.45 µm) and preserved at 4°C.
2)human ASK1 reference standard
   HeLa cell or HEK293 cell suspended in the above-mentioned 1) medium (supplemented with 10% fatal bovine serum (FBS) and 100 unit/mL penicillin) to 4×10⁴ cells/mL, seeded in a 96-well culture plate at 100 µL/well, and cultured for one day under an atmosphere of 5% CO₂/95% air at 37°C. 3) 20×ATP
   A 300 µM aqueous solution preserved at -20°C.
4) Buffer for cytolysis
   20 mM Tris-HCl (pH 7.5) [containing 150 mM NaCl, 10 mM EDTA, 1% Triton X-100, 1% deoxycholate, 1.5% aprotinin and 1 mM PMSF] preserved at 4°C.
5) Rabbit anti-human ASK1 polyclonal antibody
   Dissolved in PBS (containing 0.02% sodium azide) to 2 mg/mL and preserved at 4°C.
6) Rabbit anti-human phosphorylated ASK1 polyclonal antibody
   Dissolved in PBS (containing 0.02% sodium azide) to 2 mg/mL, and preserved at 4°C.
7) Horseradish peroxidase (HRP) labeled goat anti-rabbit IgG polyclonal antibody
   Dissolved in PBS (containing 0.02% sodium azide) to 2 mg/mL, and preserved at 4°C.
8) Chemiluminescent substrate for peroxidase activity measurement

SuperSignal (trademark) West Femto Maximum Sensitivity Substrate (PIERCE). Preserved at 4°C.

### [Measurement method]

i) HeLa (or HEK293) cells cultured in a 96-well culture plate are washed twice with the medium of the above-mentioned 1), and a fresh medium (90 µl) is added to each well.
ii) A 10⁻³-10⁻¹⁰M test substance solution (5 µl) is added, 20XATP (5 µl) is add, and the mixture is cultivated under a 5% CO₂/95% air atmosphere at 37°C for 24 hr.
iii) The culture medium is collected in a tube, the culture supernatant is removed by centrifugation, washed with a fresh medium, the cytolytic buffer of the above-mentioned 4) is added to lyse the cells, and the supernatant is collected by centrifugation to give a cell lysate.
iv) An anti-ASK1 antibody (1 µL, 2 µg) is added, the cells are incubated at 4°C for 1 hr, protein G-immobilized Sepharose beads are added, immunoprecipitation is performed at 4°C for 30 min and the precipitate is recovered by centrifugation.
v) The precipitate is washed, buffer for electrophoresis [4% SDS, 100 mM tris-hydrochloride (pH 8.8), 0.01% bromophenol blue, 36% glycerol] (30 µL) is added, the mixture is boiled at 100°C for 5 min, loaded on SDS-polyacrylamide gel and electrophoresed at 150 V for 60 min.
vi) The protein is transferred on a PVDF membrane, blocked overnight at 4°C, reacted with anti-ASK1 antibody or anti-phosphorylated ASK1 antibody (1000-fold diluted solution) overnight at 4°C, and further reacted with an HRP-labeled anti-rabbit IgG antibody (1000-fold diluted solution) at room temperature for 60 min.
vii) A chemiluminescent substrate is added and the amount of luminescence is measured using an image analyzer.

In the above-mentioned screening method, when the signal intensity of the band reactive with an anti-phosphorylated ASK1 antibody increases by the addition of a test substance to ASK1 producing cells, the substance can be selected as an ASK1 activity promoting substance. Similarly, when the signal intensity decreases by the addition of a test substance, the substance can be selected as an ASK1 activity inhibitory substance. In contrast, when the signal intensity of the band reactive with an anti-ASK1 antibody increases by the addition of a test substance to ASK1 producing cells, the substance can be selected as an ASK1 expression promoting substance. Similarly, when the signal intensity decreases by the addition of a test substance, the substance can be selected as an ASK1 expression inhibitory substance.

As mentioned above, a substance regulating (promoting or inhibiting) the ASK1 expression is effective for the prophylaxis or treatment of apoptosis or an inflammation-associated disease. In addition, an ASK1 expression inhibitory substance is particularly effective for the prophylaxis or treatment of diabetes or complications thereof, or COPD.

Accordingly, the present invention also provides a method for screening for a substance for the prophylaxis or treatment of apoptosis or an inflammation-associated disease, diabetes or complications thereof, or COPD, which comprises comparing the expression of ASK1s in a cell having an ability to produce ASK1s between in the presence of a test substance and in the absence of the test substance.

The expression level of ASK1 can also be measured at a transcription level by detecting mRNA using a nucleic acid capable of hybridizing to a nucleic acid encoding ASK1 under high stringent conditions (namely, a nucleic acid having the aforementioned base sequence encoding ASK1 or a part thereof or an antisense nucleic acid of the present invention; hereinafter sometimes to be comprehensively referred to as "the nucleic acid of the present invention"). Alternatively, the expression level can also be measured at a translation level by detecting the protein (peptide) using the aforementioned antibody of the present invention.

More specifically, therefore, the present invention provides
(b) a method for screening for a substance for the prophylaxis or treatment of apoptosis or an inflammation-associated disease, diabetes or complications thereof, or COPD, which comprises culturing a cell having the ability to produce ASK1s in the presence of a test substance and in the absence of the test substance, and measuring and comparing the amount of mRNA encoding ASK1s under the both conditions, using the nucleic acid of the present invention, and
(c) a method for screening for a substance for the prophylaxis or treatment of apoptosis or an inflammation-associated disease, diabetes or complications thereof, or COPD, which comprises culturing a cell having the ability to produce ASK1s in the presence of a test substance and in the absence of the test substance, and measuring and comparing the amount of protein (peptide) of ASK1s under the both conditions, using the antibody of the present invention.

In the above-mentioned screening methods (b) and (c), as the cell having the ability to produce ASK1s, the cells similar to those used for the screening method of the above-mentioned (a-2) are preferably used.

For example, a measurement of the mRNA content or protein (peptide) content of ASK1s is specifically conducted as described below.
(i) A drug (for example, TNF-α, IL-1, Fas, anticancer agents and the like), a physicochemical stress (for example, UV, active oxygen, ischemia and the like) or the like is added to a normal or pathologic model non-human warm-blooded animal (for example, mouse, rat, rabbit, sheep, swine, bovine, cat, dog, monkey, bird and the like), and after a given time has elapsed, blood or a particular organ (for example, brain, liver, kidney and the like), or a tissue or cells isolated from the organ are obtained.
   The mRNA of the ASK1 contained in the cells obtained can be quantified by, for example, extracting the mRNA from the cells and the like by an ordinary method, and using a technique, for example, RT-PCR and the like, and can also be quantified by a Northern blot analysis known *per se.* On the other hand, the ASK1 protein content can be quantified by Western blot analysis or various immunoassays to be described in detail in the following.
(ii) a transformant incorporating a nucleic acid encoding ASK1 or active peptide of the present invention is prepared according to the above-mentioned method, ASK1s contained in the transformant or mRNA encoding same is quantified and analyzed in the same manner as in the above-mentioned (i).

A substance that changes the expression level of ASK1s can be screened for by
(i) administering a test substance to a normal or diseased non-human warm-blooded animal model at a given time (30 min - 24 hr, preferably 30 min - 12 hr, more preferably 1 hr - 6 hr) before giving a pharmaceutical agent or physicochemical stress and the like, or a given time (30 min - 3 days, preferably 1 hr - 2 days, more preferably 1 hr - 24 hr) thereafter, or simultaneously with a pharmaceutical agent or a physicochemical stress, and quantifying and analyzing the amount of mRNA encoding the ASK1s or a protein (peptide) of ASK1s contained in the cell isolated from the animal, at a given time (30 min - 3 days, preferably 1 hr - 2 days, more preferably 1 hr - 24 hr) after the administration, or
(ii) adding a test substance to a medium during cultivation of a transformant according to a conventional method and, after culture for a given time (1 day - 7 days, preferably 1 day - 3 days, more preferably 2 days - 3 days), and quantifying and analyzing the amount of mRNA or a protein (peptide) of ASK1s contained in the transformant.

As the test substance, a peptide, a protein, a non-peptide compound, a synthetic compound, a fermentation product and the like can be mentioned, and these substances may be novel substances or publicly known substances.

For the measurement of the amount of ASK1s in the screening method of the above-mentioned (c), specifically, for example,
(i) a method comprising quantifying ASK1 in a sample solution by competitively reacting the antibody of the present invention with the sample solution and labeled ASK1, detecting labeled ASK1 bonded to the antibody, and
(ii) a method comprising quantifying ASK1 in a sample solution by simultaneously or continuously reacting the sample solution with the antibody of the present invention insolubilized on a carrier and a labeled other antibody of the present invention, and measuring the amount (activity) of the labeling agent insolubilized on the carrier, and the like can be mentioned.

In the quantification method of the above-mentioned (ii), two kinds of antibodies desirably recognize different portions of ASK1. For example, when one antibody recognizes the N-terminal portion of ASK1, as the other antibody, one reactive with the C-terminal portion of ASK1 can be used.

As examples of the labeling agent used for the assay using a labeled substance, a radioisotope, an enzyme, a fluorescent substance, a luminescent substance and the like can be used. As examples of the radioisotope, [¹²⁵I], [¹³¹I], [³H], [¹⁴C] and the like can be used. As the above-described enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-(strepto)avidin system can also be used for binding of an antibody or an antigen and a labeling agent.

As the sample solution, when ASK1 is localized in a cell, a cell disrupt solution obtained by suspending the cell in an appropriate buffer, and disrupting the cell by sonication or freeze-thawing and the like is used and, when ASK1 is extracellularly secreted, a cell culture supernatant is used. Where necessary, ASK1 may be separated and purified from a disrupt solution or culture supernatant and quantified. As long as the labeling agent can be detected, an intact cell may be used as a sample.

The quantification of ASK1s using the antibody of the present invention is not subject to limitation, and any method of measurement can be used, as long as it is a measurement method wherein the amount of antibody, antigen or antibody-antigen complex corresponding to the amount of antigen in the sample solution is detected by a chemical or physical means and is calculated from a standard curve generated using standard solutions containing known amounts of antigen. For example, nephelometry, the competitive method, the immunometric method and the sandwich method are preferably used; it is preferable, for example, in terms of sensitivity and specificity, to use the sandwich method described below.

In insolubilizing the antigen or antibody, physical adsorption may be used, and a chemical bond in common use to insolubilize or immobilize a protein or an enzyme or the like, may also be used. As the carrier, insoluble polysaccharides such as agarose, dextran and cellulose, synthetic resins such as polystyrene, polyacrylamide and silicone, glass and the like can be mentioned.

In the sandwich method, the amount of ASK1s in a sample solution can be quantified by reacting the sample solution to an antibody of the present invention insolubilized (primary reaction) and further reacting to another antibody of the present invention labeled (secondary reaction), and thereafter measuring the amount or activity of the labeling agent on the insolubilizing carrier. The primary reaction and the secondary reaction may be conducted in the reverse order, and may be conducted simultaneously or after a time lag. The labeling agent and the method of insolubilization can be based on those described above. Also, in the immunoassay by the sandwich method, the antibody used as the antibody for a solid phase or the antibody for labeling needs not always be one kind; a mixture of two kinds or more of antibodies may be used for the purposes of measurement sensitivity improvement and the like.

The antibody of the present invention can be used for a measurement system other than the sandwich method, for example, the competitive method, the immunometric method or nephelometry and the like.

In the competitive method, ASK1 and labeled ASK1 in a sample solution are competitively reacted with an antibody, a non-reacted labeled antigen (F) and a labeled antigen (B) bonded to the antibody are separated (B/F separation), the amount of label of B or F is measured to quantify ASK1 in the sample solution. For this reaction method, a liquid phase method wherein a soluble antibody is used as the antibody, and polyethylene glycol and a secondary antibody against the aforementioned antibody (primary antibody) and the like are used for B/F separation, and a solid-phase method wherein a solid-phase antibody is used as a primary antibody (direct method), or a soluble primary antibody is used and a solid-phase antibody is used as a secondary antibody (indirect method) are employed.

In the immunometric method, ASK1 in a sample solution and solid-phased ASK1 are competitively reacted with a given amount of a labeled antibody, and the solid phase and the liquid phase are separated, or ASK1 in a sample solution and an excess amount of a labeled antibody are reacted, solid-phased ASK1 is added to bond a non-reacted labeled antibody to the solid phase, and the solid phase is separated from the liquid phase. Then, the label of either phase is measured and the amount of antigen in the sample solution is quantified.

Also, in nephelometry, the amount of insoluble precipitate resulting from an antigen-antibody reaction in the gel or in the solution is measured. Even when the amount of ASK1s in the sample solution is small and only a small amount of precipitate is obtained, laser nephelometry, which utilizes laser scattering, and the like are preferably used.

In applying these individual immunological measurement methods to the quantification method of the present invention, it is unnecessary to set special conditions, procedures and the like. Making ordinary technical considerations for those skilled in the art to the ordinary conditions and procedures in each method, a measurement system for ASK1s can be constructed. For details of these general technical means, compendia, books and the like can be referred to.

For example, edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121(Immunochemical Techniques (Part I: Hybridoma Technology and Monoclonal Antibodies)) (all published by Academic Press) and the like can be referred to. Using the antibody of the present invention as described above, the amount of ASK1 produced in a cell can be quantified with high sensitivity.

According to the screening methods of the above-mentioned (b) and (c), a substance that increased the expression level (mRNA amount or protein (peptide) amount) of ASK1 can be selected as an ASK1 expression promoting substance, and a substance that decreased the expression level can be selected as an ASK1 expression inhibitory substance. The ASK1 expression promoting substance can be used as an agent for the prophylaxis or treatment of diseases associated with insufficient apoptosis induction or inflammatory cytokine production [for example, cancers (e.g., leukemia, esophageal cancer, gastric cancer, colon cancer, rectal cancer, lung cancer, liver cancer, kidney cancer, breast cancer, uterine cancer, ovarian cancer, prostatic cancer, melanoma, myeloma, osteosarcoma, brain tumor and the like), autoimmune diseases (e.g., systemic lupus erythematosus, scleroderma, rheumatoid arthritis, Sjogren's syndrome, multiple sclerosis, insulin-dependent diabetes, psoriasis, ulcerative colitis, idiopathic thrombocytopenic purpura, Crohn's disease, glomerulonephritis and the like), viral infections (e.g., hemorrhagic fever, T-cell leukemia, Kaposi sarcoma, infectious mononucleosis, lymphoma, epipharyngeal cancer, cervical cancer, skin cancer, hepatitis, liver cancer and the like), endocrine diseases (e.g., hyperhormonal disease, hypercytokine disease and the like), hematological diseases (e.g., hemocytosis, B-cell lymphoma, polycythemia and the like), organ hyperplasia (e.g., hermaphroditism, undescended testis, teratoma, nephroblastoma, polycystic kidney, cardiac/aortic malformations, syndactyly and the like), post-angioplastic restenosis, recurrence after cancer resection and the like].

On the other hand, a substance for inhibiting ASK1 expression can be used as a prophylactic or therapeutic agent for a disease associated with abnormal promotion of apoptosis induction or inflammatory cytokine production [for example, viral infections (e.g., AIDS, influenza, fever of unknown origin and the like), endocrine diseases (e.g., hormone deficiency, cytokine deficiency and the like), hematological diseases (e.g., hemocytopenia, renal anemia and the like), organ hypoplasia (e.g., thyroid atrophy, cleft palate and the like), organ graft rejection, graft-versus-host disease, immune deficiency, neurodegenerative diseases (e.g., polyglutamine disease, Alzheimer's disease, Parkinson's disease, amyotrophic lateral sclerosis, prion disease, cerebellar degeneration and the like), ischemic heart diseases (e.g., angina pectoris, myocardial infarction and the like), radiation injuries, ultraviolet injuries (e.g., sunburns and the like), poisoning diseases (e.g., renal tubular cell injury by heavy metals, liver cell injury by alcohol, and the like), nutritional disorders (e.g., thymus atrophy due to vitamin or trace element deficiency, and the like), inflammatory diseases (e.g., acute pancreatitis, arthritis, periodontal disease, colitis and the like), ischemic neuropathy, vascular diseases (e.g., arteriosclerosis and the like), respiratory diseases (e.g., interstitial pneumonia, pulmonary fibrosis and the like), articular diseases (e.g., arthritic deformans and the like) and the like].

In addition, an ASK1 expression inhibitory substance can be preferably used as an agent for the prophylaxis or treatment of diabetes or complications thereof (e.g., diabetic nephropathy, diabetic neuropathy etc.), or COPD.

When the ASK1 expression promoting or inhibitory substance is used as the above-described prophylactic or therapeutic agent, it can be formulated in the same manner as in the aforementioned ASK1 activity promoting or inhibitory substance.

Since a preparation obtained in this way is safe and shows lower toxicity, it can be administered, for example, to human and other warm-blooded animals (e.g., rat, mouse, hamster, rabbit, sheep, goat, swine, bovine, horse, cat, dog, monkey, chimpanzee, bird and the like).

The dosage of a substance for promoting ASK1 expression varies depending on subject of administration, target organ, symptoms, method of administration and the like; in a cancer patient (body weight 60 kg), for example, the usual oral dosage is about 0.1 mg to 100 mg, preferably about 1.0 to 50 mg, more preferably about 1.0 to 20 mg, per day. In the case of non-oral administration, the dosage per administration varies depending on subject of administration, target organ, symptoms, method of administration and the like; in a cancer patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

The dosage of the substance for inhibiting ASK1 expression varies depending on subject of administration, target organ, symptoms, method of administration and the like; in a diabetes (or complications thereof) or COPD patient (body weight 60 kg), for example, the usual oral dosage is about 0.1 mg to 100 mg, preferably about 1.0 to 50 mg, more preferably about 1.0 to 20 mg, per day. In the case of non-oral administration, the dosage per administration varies depending on subject of administration, target organ, symptoms, method of administration and the like; in a diabetes (or complications thereof) or COPD patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

### (2) an agent for the prophylaxis or treatment of a disease associated with abnormal promotion of apoptosis induction or inflammatory cytokine production

As described above, since ASK1 induces apoptosis to cells and enhances the production of inflammatory cytokine, it causes various diseases, for example, viral infections, endocrine diseases, hematological diseases, organ hypoplasia, organ graft rejection, graft-versus-host disease, immune deficiency, neurodegenerative diseases, ischemic heart diseases, radiation injuries, ultraviolet injuries, poisoning diseases, nutritional disorders, inflammation, ischemic neuropathy vascular diseases, respiratory diseases, articular diseases and the like develop if there is an abnormality in ASK1 or a nucleic acid that encodes the same (e.g., gene, mRNA and the like) in the body (emergence of highly active variant), if the expression level is abnormally increases, or if apoptosis induction or inflammatory cytokine production in cells is abnormally promoted due to any other factor.

Therefore, it is possible to suppress the apoptotic death of originally essential cells and inflammatory reactions by:
a) administering the antibody of the present invention to a patient who lacks cells that are essential to the body by apoptosis, or who has an inflammatory disease, due to an increase in ASK1 and the like, to inactivate ASK1, or by: b) reducing the ASK1 content in the patient's body by (i) administering the antisense nucleic acid of the present invention to the patient to introduce (and express) it to the target cell, or (ii) introducing the antisense nucleic acid of the present invention to the isolated target cell to allow its expression, and transferring the cell to the patient.

Since inactivation of ASK1 gene decreases the sensitivity of blood glucose increase due to high-fat diet, and inhibits progression of neuropathy in diabetes model, the in vivo ASK1 activity or amount in patients can be decreased, the disease can be treated, and the progress can be suppressed by applying the treatment of the above-mentioned a) or b) to patients suffering from diabetes and complications thereof.

Moreover, since inactivation of ASK1 gene inhibits changes in the inflammation in the lung due to tobacco smoke exposure, the in vivo ASK1 activity or amount in patients can be decreased, the disease can be treated, and the progress can be suppressed by applying the treatment of the above-mentioned a) or b) to patients suffering from COPD.

Accordingly, a) the antibody of the present invention or b) the antisense nucleic acid of the present invention can be used as a prophylactic or therapeutic agent for (i) a disease in which suppression of apoptosis induction or inflammatory cytokine production is effective for the prophylaxis or therapy thereof, for example, a disease caused by ASK1 overexpression and the like, specifically viral infections (e.g., AIDS, influenza, fever of unknown origin and the like), endocrine diseases (e.g., hormone deficiency, cytokine deficiency and the like), hematological diseases (e.g., hemocytopenia, renal anemia and the like), organ hypoplasia (e.g., thyroid atrophy, cleft palate and the like), organ graft rejection, graft-versus-host disease, immune deficiency, neurodegenerative diseases (e.g., polyglutamine disease, Alzheimer's disease, Parkinson's disease, amyotrophic lateral sclerosis, prion disease, cerebellar degeneration and the like), ischemic heart diseases (e.g., angina pectoris, myocardial infarction and the like), radiation injuries, ultraviolet injuries (e.g., sunburns and the like), poisoning diseases (e.g., renal tubular cell injury by heavy metals, liver cell injury by alcohol, and the like), nutritional disorders (e.g., thymus atrophy due to vitamin or trace element deficiency, and the like), inflammatory diseases (e.g., acute pancreatitis, arthritis, periodontal disease, colitis and the like), ischemic neuropathy, vascular diseases (e.g., arteriosclerosis and the like), respiratory diseases (e.g., interstitial pneumonia, pulmonary fibrosis and the like), articular diseases (e.g., arthritic deformans and the like) and the like (ii) diabetes (particularly type 2 diabetes) or diabetic complications (e.g., diabetic nephropathy, diabetic neuropathy etc.), or (iii) COPD.

When the antibody of the present invention is used as the above-mentioned agent for the prophylaxis or treatment, it can be formulated in the same manner as in the aforementioned ASK1 activity inhibitory substance.

When the antisense nucleic acid of the present invention is used as the above-mentioned agent for the prophylaxis or treatment, the nucleic acid alone may be formulated according to a conventional means or after insertion into a suitable vector, retrovirus vector, adenoviral vector, adeno virus-associated virus vector and the like, according to a conventional means. The nucleic acid can be administered as it is, or together with an auxiliary agent for the promotion of intake, using a gene gun or a catheter such as a hydrogel catheter.

For example, antisense nucleic acid of the present invention can be used orally as tablets, capsules, elixirs, microcapsules and the like, coated with sugar as required, or can be used non-orally in the form of an injection such as a sterile solution or suspension in water or another pharmaceutically acceptable liquid. For example, by blending antisense nucleic acid of the present invention along with a known physiologically acceptable carrier, a sweetener, a excipient, a vehicle, an antiseptic, a stabilizer, a binder and the like, in a unit dosage form required for generally accepted preparation design, such a preparation can be produced. The active ingredient contents in these preparations are intended to ensure that an appropriate dose in the specified range is obtained.

As the additive such as the above-mentioned physiologically acceptable and known carrier, flavor, excipient, vehicle, preservative, stabilizer, binder and the like, additive similar to those used for the aforementioned ASK1 activity inhibitory substance can be given as examples.

Since the preparation thus obtained is safe and of low toxicity, it can be administered to, for example, a human or another warm-blooded animal (for example, rat, mouse, hamster, rabbit, sheep, goat, swine, bovine, horse, cat, dog, monkey, chimpanzee, bird and the like).

The dosage of the antibody of the present invention varies depending on subject of administration, target organ, symptoms, method of administration and the like; in a diabetes (or complications thereof) or COPD patient (body weight 60 kg), for example, the usual oral dosage is about 0.1 mg to 100 mg, preferably about 1.0 to 50 mg, more preferably about 1.0 to 20 mg, per day. In the case of non-oral administration, the dosage per administration varies depending on subject of administration, target organ, symptoms, method of administration and the like; in a diabetes (or complications thereof) or COPD patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

The dosage of the antisense nucleic acid of the present invention varies depending on subject of administration, target organ, symptoms, method of administration and the like; in a type 2 diabetes (or diabetic complications) or COPD patient (body weight 60 kg), for example, the usual oral dosage is about 0.1 mg to 100 mg, preferably about 1.0 to 50 mg, more preferably about 1.0 to 20 mg, per day. In the case of non-oral administration, the dosage per administration varies depending on subject of administration, target organ, symptoms, method of administration and the like; in a type 2 diabetes (or diabetic complications) or COPD patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

As mentioned above, the inhibitory peptide of the present invention can function as an ASK1 (antagonist) inhibitory substance. That is, since the peptide has affinity for but does not activate MAPKK, which is a substrate protein of ASK1, it can competitively inhibit the approach of ASK1 and MAPKK endogenous to the cell and activation (phosphorylation) of MAPKK thereby. Therefore, the partial peptide can be used as an agent for the prophylaxis or treatment of diseases associated with abnormal promotion of apoptosis induction or inflammatory cytokine production, diabetes or complications thereof, or COPD, in the same manner as with the antibody of the present invention and the antisense nucleic acid of the present invention.

When the inhibitory peptide of the present invention is used as the above-mentioned agent for the prophylaxis or treatment, it can be formulated in the same manner as in the aforementioned ASK1 activity inhibitory substance.

Since the preparation thus obtained is safe and of low toxicity, it can be administered to, for example, a human or another warm-blooded animal (for example, rat, mouse, hamster, rabbit, sheep, goat, swine, bovine, horse, cat, dog, monkey, chimpanzee, bird and the like).

The dosage of the inhibitory peptide of the present invention varies depending on subject of administration, target organ, symptoms, method of administration and the like; in a diabetes (or complications thereof) or COPD patient (body weight 60 kg), for example, the usual oral dosage is about 0.1 mg to 100 mg, preferably about 1.0 to 50 mg, more preferably about 1.0 to 20 mg, per day. In the case of non-oral administration, the dosage per administration varies depending on subject of administration, target organ, symptoms, method of administration and the like; in a diabetes (or complications thereof) or COPD patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

### (3) Genetic diagnostic reagent

Since the above-described nucleic acid of the present invention is capable of detecting an abnormality (genetic abnormality) in the DNA or mRNA that encodes ASK1 in a human or another warm-blooded animal (for example, rat, mouse, hamster, rabbit, sheep, goat, swine, bovine, horse, cat, dog, monkey, chimpanzee, bird, and the like) when used as a probe and the like, it is useful as, for example, a genetic diagnostic reagent for damage or mutation of the DNA, a splicing abnormality or decreased expression of mRNA, amplification of the DNA, overexpression of mRNA, and the like. A nucleic acid that comprises a portion of the base sequence that encodes ASK1 is not subject to limitation, as long as it has a necessary length for a probe (for example, about 15 bases or more), and needs not encode a partial peptide of ASK1 (i.e., being in frame).

The above-described genetic diagnosis using the nucleic acid of the present invention can be performed by, for example, Northern hybridization, quantitative RT-PCR, PCR-SSCP method, allele-specific PCR, PCR-SSOP method, DGGE method, RNase protection method, PCR-RFLP method and the like which are known *per se.*

For example, when a reduction in ASK1 expression is detected as a result of Northern hybridization or quantitative RT-PCR of an RNA fraction extracted from cells of a test warm-blooded animal, or if a mutation in the ASK1 gene is detected as a result of an analysis of an RNA fraction or genomic DNA fraction by the PCR-SSCP method, the animal can be diagnosed as suffering from or being likely to suffer from a disease associated with insufficient apoptosis induction or inflammatory cytokine production, for example, cancers (e.g., leukemia, esophageal cancer, gastric cancer, colon cancer, rectal cancer, lung cancer, liver cancer, kidney cancer, breast cancer, uterine cancer, ovarian cancer, prostatic cancer, melanoma, myeloma, osteosarcoma, brain tumor and the like), autoimmune diseases (e.g., systemic lupus erythematosus, scleroderma, rheumatoid arthritis, Sjogren's syndrome, multiple sclerosis, insulin-dependent diabetes, psoriasis, ulcerative colitis, idiopathic thrombocytopenic purpura, Crohn's disease, glomerulonephritis and the like), viral infections (e.g., hemorrhagic fever, T-cell leukemia, Kaposi sarcoma, infectious mononucleosis, lymphoma, epipharyngeal cancer, cervical cancer, skin cancer, hepatitis, liver cancer and the like), endocrine diseases (e.g., hyperhormonal disease, hypercytokine disease and the like), hematological diseases (e.g., hemocytosis, B-cell lymphoma, polycythemia and the like), organ hyperplasia (e.g., hermaphroditism, undescended testis, teratoma, nephroblastoma, polycystic kidney, cardiac/aortic malformations, syndactyly and the like), post-angiectatic restenosis, recurrence after cancer resection and the like.

In contrast, when an excess expression of ASK1 is detected by northern hybridization or quantitative RT-PCR, the animal can be diagnosed to have affected with, or have a high possibility of being affected in the future with,
(i) a disease associated with abnormal promotion of apoptosis induction or inflammatory cytokine production, for example, viral infections (e.g., AIDS, influenza, fever of unknown origin and the like), endocrine diseases (e.g., hormone deficiency, cytokine deficiency and the like), hematological diseases (e.g., hemocytopenia, renal anemia and the like), organ hypoplasia (e.g., thyroid atrophy, cleft palate and the like), organ graft rejection, graft-versus-host disease, immune deficiency, neurodegenerative diseases (e.g., polyglutamine disease, Alzheimer's disease, Parkinson's disease, amyotrophic lateral sclerosis, prion disease, cerebellar degeneration and the like), ischemic heart diseases (e.g., angina pectoris, myocardial infarction and the like), radiation injuries, ultraviolet injuries (e.g., sunburns and the like), poisoning diseases (e.g., renal tubular cell injury by heavy metals, liver cell injury by alcohol, and the like), nutritional disorders (e.g., thymus atrophy due to vitamin or trace element deficiency, and the like), inflammatory diseases (e.g., acute pancreatitis, arthritis, periodontal disease, colitis and the like), ischemic neuropathy, vascular diseases (e.g., arteriosclerosis), respiratory diseases (e.g., interstitial pneumonia, pulmonary fibrosis etc.), articular diseases (e.g., arthritic deformans and the like) and the like, (ii) diabetes or complications thereof (e.g., diabetic nephropathy, diabetic neuropathy etc.), or (iii) COPD.

Since the aforementioned antibody of the present invention can measure the amount of ASK1 or a salt thereof in human or other warm-blooded animals (e.g., rat, mouse, humster, rabbit, sheep, goat, swine, bovine, horse, cat, dog, monkey, chimpanzee, bird and the like), it is useful, for example, as a gene diagnostic reagent for decreased expression, excess expression and the like of the protein.

The above-mentioned gene diagnosis using the nucleic acid of the present invention can be performed by an immunoassay using a biological sample (e.g., blood, plasma, urine, biopsy and the like) collected from a warm-blooded test animal as a cell having the ability to produce ASK1s in the screening method (screening method of (c)) for an ASK1 expression regulating (promoting or inhibiting) substance, which comprises using the aforementioned antibody of the present invention.

As a result of the immunoassay, when ASK1 or a salt thereof in the sample is detected to have decreased, the subject can be diagnosed to have affected with, or have a high possibility of being affected in the future with a disease associated with insufficient apoptosis induction or inflammatory cytokine production, for example, cancers (e.g., leukemia, esophageal cancer, gastric cancer, colon cancer, rectal cancer, lung cancer, liver cancer, kidney cancer, breast cancer, uterine cancer, ovarian cancer, prostatic cancer, melanoma, myeloma, osteosarcoma, brain tumor and the like), autoimmune diseases (e.g., systemic lupus erythematosus, scleroderma, rheumatoid arthritis, Sjogren's syndrome, multiple sclerosis, insulin-dependent diabetes, psoriasis, ulcerative colitis, idiopathic thrombocytopenic purpura, Crohn's disease, glomerulonephritis and the like), viral infections (e.g., hemorrhagic fever, T-cell leukemia, Kaposi sarcoma, infectious mononucleosis, lymphoma, epipharyngeal cancer, cervical cancer, skin cancer, hepatitis, liver cancer and the like), endocrine diseases (e.g., hyperhormonal disease, hypercytokine disease and the like), hematological diseases (e.g., hemocytosis, B-cell lymphoma, polycythemia and the like), organ hyperplasia (e.g., hermaphroditism, undescended testis, teratoma, nephroblastoma, polycystic kidney, cardiac/aortic malformations, syndactyly and the like), post-angiectatic restenosis, recurrence after cancer resection, and the like.

On the other hand, as a result of the immunoassay, when ASK1 or a salt thereof in the sample is detected to have increased, the subject can be diagnosed to have affected with, or have a high possibility of being affected in the future with (i) a disease associated with abnormal promotion of apoptosis induction or inflammatory cytokine production, for example, viral infections (e.g., AIDS, influenza, fever of unknown origin and the like), endocrine diseases (e.g., hormone deficiency, cytokine deficiency and the like), hematological diseases (e.g., hemocytopenia, renal anemia and the like), organ hypoplasia (e.g., thyroid atrophy, cleft palate and the like), organ graft rejection, graft-versus-host disease, immune deficiency, neurodegenerative diseases (e.g., polyglutamine disease, Alzheimer's disease, Parkinson's disease, amyotrophic lateral sclerosis, prion disease, cerebellar degeneration and the like), ischemic heart diseases (e.g., angina pectoris, myocardial infarction and the like), radiation injuries, ultraviolet injuries (e.g., sunburns and the like), poisoning diseases (e.g., renal tubular cell injury by heavy metals, liver cell injury by alcohol, and the like), nutritional disorders (e.g., thymus atrophy due to vitamin or trace element deficiency, and the like), inflammatory diseases (e.g., acute pancreatitis, arthritis, periodontal disease, colitis and the like), ischemic neuropathy, vascular diseases (e.g., arteriosclerosis), respiratory diseases (e.g., interstitial pneumonia, pulmonary fibrosis etc.), articular diseases (e.g., arthritic deformans and the like) and the like, (ii) diabetes or complications thereof (e.g., diabetic nephropathy, diabetic neuropathy etc.), or (iii) COPD.

### (4) Use of ASK1 inhibitory substance

The present invention also provides an agent for the prophylaxis or treatment of (i) type 2 diabetes or diabetic complications (e.g., diabetic nephropathy etc.), or (ii) COPD, which comprises an ASK1 inhibitory substance (as is clear from the above, the term includes the above-mentioned ASK1 expression inhibitory substance and the ASK1 activity inhibitory substance).

Examples of the ASK1 inhibitory substance include, but not limited to, dominant-negative mutant of ASK1 (see, e.g., the above-mentioned patent references 1-3), glutathione S-transferase, neph, 14-3-3, thioredoxin, HSP72 (Mol. Cell Biol., 22(22): 7721-7730, 2002), an ASK1 expression or activity inhibitory substance obtained by the aforementioned various screening methods of the present invention, and the like.

When an ASK1 inhibitory substance is used as the above-mentioned prophylactic or therapeutic agent, it can be formulated in the same manner as in the ASK1 activity inhibitory substance obtained by the aforementioned screening method.

Since the preparation thus obtained is safe and of low toxicity, it can be administered to, for example, a human or another warm-blooded animal (for example, rat, mouse, hamster, rabbit, sheep, goat, swine, bovine, horse, cat, dog, monkey, chimpanzee, bird and the like).

The dosage of the ASK1 inhibitory substance varies depending on subject of administration, target organ, symptoms, method of administration and the like; in a type 2 diabetes (or diabetic complications) or COPD patient (body weight 60 kg), for example, the usual oral dosage is about 0.1 mg to 100 mg, preferably about 1.0 to 50 mg, more preferably about 1.0 to 20 mg, per day. In the case of non-oral administration, the dosage per administration varies depending on subject of administration, target organ, symptoms, method of administration and the like; in a type 2 diabetes (or diabetic nephropathy) or COPD patient (body weight 60 kg), for example, the usual dosage in an injection is conveniently about 0.01 to 30 mg, preferably about 0.1 to 20 mg, more preferably about 0.1 to 10 mg, per day. In the case of other animals, a dosage converted per 60 kg body weight can be administered.

### (5) Animal introduced with a DNA that encodes ASK1 and use therefor

The present invention provides a new use for a non-human mammal having a foreign DNA that encodes ASK1 (hereinafter abbreviated as a foreign DNA of the present invention) or a variant DNA thereof (also abbreviated as foreign variant DNA of the present invention).

The non-human mammal used in the present invention includes:
[1] a non-human mammal having a foreign DNA of the present invention or a variant DNA thereof,
[2] the mammal described in term [1], wherein the non-human mammal is a rodent, and
[3] the mammal described in term [2], wherein the rodent is a mouse or rat.

The non-human mammal having the foreign DNA of the present invention or a variant DNA thereof (hereinafter abbreviated as the DNA transgenic animal of the present invention) can be produced by transferring the desired DNA to a germ cell, including an unfertilized ovum, a fertilized ovum, a sperm and a primordial cell thereof, and the like, preferably in the stage of embryogenesis in the stage of development of the non-human mammal (more preferably, in the single-cell or fertilized ovum cell stage and generally at or prior to the 8-cell stage), by the calcium phosphate method, the electric pulse method, the lipofection method, the aggregation method, the microinjection method, the particle gun method, the DEAE-dextran method and the like. Also, it is possible to transfer the desired foreign DNA of the present invention to a somatic cell, an organ of a living body, a tissue cell and the like by the DNA transfer method, and utilize it for cell culture, tissue culture and the like; furthermore, it is also possible to produce the DNA transgenic animal of the present invention by fusing these cells with the above-described germ cell by a method of cell fusion known *per se.*

As examples of the non-human mammal, bovine, swine, sheep, goat, rabbit, dog, cat, guinea pig, hamster, mouse, rat and the like can be used. Particularly preferred from the viewpoint of preparation of a pathologic animal model system are rodents, which have relatively short ontogenesis and biological cycles, and which permit easy propagation, particularly the mouse (for example, C57BL/6 strain, DBA2 strain and the like as pure strains, B6C3F₁ strain, BDF₁ strain, B6D2F₁ strain, BALB/c strain, ICR strain and the like as cross strains) or the rat (for example, Wistar, SD and the like) and the like.

As the "mammal" in a recombinant vector that can be expressed in a mammal, human and the like can be mentioned, in addition to the above-described non-human mammals.

The foreign DNA of the present invention refers to the DNA that encodes ASK1 once isolated and extracted from a mammal, rather than to the DNA that encodes ASK1 originally possessed by a non-human mammal.

As the variant DNA of the present invention, one having a variation in the base sequence of the original ASK1-coding DNA (for example, mutation and the like), specifically DNA and the like having a base added, deleted, or substituted by another base, and the like can be used, and an abnormal DNA is also included.

The abnormal DNA means a DNA that expresses abnormal ASK1; for example, a DNA that expresses abnormal ASK1 that suppresses the function of normal ASK1 and the like can be used.

The foreign DNA of the present invention may be derived from a mammal of the same species as, or a different species from, the subject animal. In transferring the foreign DNA of the present invention to the subject animal, it is generally advantageous to use the DNA as a DNA construct bound downstream of a promoter capable of allowing the DNA to be expressed in an animal cell. For example, when the DNA that encodes human ASK1 is transferred, by microinjecting a DNA construct (e.g., vector and the like) having the DNA that encodes human ASK1 bound downstream of various promoters capable of expressing a DNA derived from various mammals (for example, rabbit, dog, cat, guinea pig, hamster, rat, mouse and the like) having a DNA highly homologous thereto to a fertilized ovum of a subject mammal, for example, a mouse fertilized ovum, a DNA transgenic mammal that expresses the DNA that encodes ASK1 at a high level can be produced.

As the expression vector for ASK1, an *Escherichia* coli-derived plasmid, a *Bacillus* subtilis-derived plasmid, a yeast-derived plasmid, a bacteriophage such as λ phage, a retrovirus such as Moloney leukemia virus, an animal virus such as vaccinia virus or baculovirus, and the like can be used. Particularly preferably used are an *Escherichia* coli-derived plasmid, a *Bacillus subtilis*-derived plasmid or a yeast-derived plasmid and the like.

As examples of the above-described promoter that regulates DNA expression, (i) promoters for DNAs derived from viruses (e.g., simian virus, cytomegalovirus, Moloney leukemia virus, JC virus, breast cancer virus, poliovirus and the like), (ii) promoters derived from various mammals (human, rabbit, dog, cat, guinea pig, hamster, rat, mouse and the like), for example, promoters for albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen type I and type II, cyclic AMP-dependent protein kinase βI subunit, dystrophin, tartaric acid-resistant alkaline phosphatase, atrial natriuretic factor, endothelial receptor tyrosine kinase (generally abbreviated as Tie2), sodium-potassium adenosine triphosphatase (Na,K-ATPase), neurofilament light chain, metallothionein I and IIA, metalloproteinase 1 tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin basic protein, thyroglobulin, Thy-1, immunoglobulin, H chain variable portion (VNP), serum amyloid P component, myoglobulin, troponin C, smooth muscle α actin, preproenkephalin A, vasopressin and the like, and the like can be used. Particularly preferred are the cytomegalovirus promoter, the human peptide chain elongation factor 1α (EF-1α) promoter, the human and chicken β actin promoters and the like, which can be expressed at high levels systemically.

The above-described vector preferably has a sequence that terminates the transcription of the desired mRNA in the DNA transgenic mammal (generally referred to as terminator); for example, the sequence of each DNA derived from a virus and derived from various mammals can be used, and the SV40 terminator of simian virus and the like can be used preferably.

Besides, for the purpose of expressing the desired foreign DNA at a higher level, the splicing signal and an enhancer region of each DNA, a portion of the intron of a eukaryotic DNA and the like can also be joined upstream of the 5' of the promoter region, between the promoter region and the translation region or downstream of the 3' of the translation region depending on the purpose.

The translation region of the normal ASK1 can be acquired as the entire or a portion of genomic DNA from a DNA from liver, kidney, thyroid cells or fibroblasts of a human or various mammals (for example, rabbit, dog, cat, guinea pig, hamster, rat, mouse and the like), or from commercially available various genomic DNA libraries, or using a complementary DNA prepared from an RNA derived from liver, kidney, thyroid cells, fibroblasts and the like by a publicly known method as the raw material. Also, for the foreign abnormal DNA, it is possible to prepare a translation region by mutating the translation region of normal ASK1 from the above-described cell or tissue by point mutagenesis.

The translation region can be prepared as a DNA construct that can be expressed in a transgenic animal by an ordinary DNA engineering technique wherein it is joined downstream of the aforementioned promoter and, as desired, upstream of the transcription termination site.

Transfer of the foreign DNA of the present invention in the fertilized ovum cell stage is assured so that it will be present in all germ cells and somatic cells of the subject mammal. The presence of the foreign DNA of the present invention in germ cells of the produced animal after the DNA transfer means that all progenies of the produced animal have the foreign DNA of the present invention in all germ cells and somatic cells thereof. Progenies of this kind of animal that have inherited the foreign DNA of the present invention have the foreign DNA of the present invention in all germ cells and somatic cells thereof.

A non-human mammal to which the foreign normal DNA of the present invention is transferred can be propagated over generations as an animal retaining the DNA in an ordinary rearing environment after the stable retention of the foreign DNA is confirmed by mating.

Transfer of the foreign DNA of the present invention in the fertilized ovum cell stage is assured so that it will be present in excess in all germ cells and somatic cells of the subject mammal. The excess presence of the foreign DNA of the present invention in germ cells of the produced animal after the DNA transfer means that all progenies of the produced animal have the foreign DNA of the present invention in excess in all germ cells and somatic cells thereof. Progenies of this kind of animal that have inherited the foreign DNA of the present invention have the foreign DNA of the present invention in all germ cells and somatic cells thereof in excess.

By acquiring a homozygous animal having the introduced DNA in both homologous chromosomes, and mating a male and female of this animal, the animal can be propagated over generations so that all progenies will have the DNA in excess.

A non-human mammal having the foreign DNA of the present invention has the normal DNA of the present invention expressed at a high level therein, possibly finally develops ASK1 hyperfunction by promoting the function of endogenous normal DNA, and can be utilized as a pathologic model animal thereof. For example, using the normal DNA transgenic animal of the present invention, it is possible to elucidate the pathologic mechanism of ASK1 hyperfunction or disease associated with ASK1, and to investigate a therapeutic method for these diseases.

Also, because a mammal to which the foreign normal DNA of the present invention is transferred has a symptom of increased ASK1, it can also be utilized for a screening test for a prophylactic or therapeutic drug for a disease associated with increased function of ASK1, for example, diabetes and complications thereof (e.g., diabetic nephropathy, diabetic neuropathy etc.), respiratory diseases (e.g., COPD, interstitial pneumonia, pulmonary fibrosis etc.), viral infections (e.g., AIDS, influenza, fever of unknown origin and the like), endocrine diseases (e.g., hormone deficiency, cytokine deficiency and the like), hematological diseases (e.g., hemocytopenia, renal anemia and the like), organ hypoplasia (e.g., thyroid atrophy, cleft palate and the like), organ graft rejection, graft-versus-host disease, immune deficiency, neurodegenerative diseases (e.g., polyglutamine disease, Alzheimer's disease, Parkinson's disease, amyotrophic lateral sclerosis, prion disease, cerebellar degeneration and the like), ischemic heart diseases (e.g., angina pectoris, myocardial infarction and the like), radiation injuries, ultraviolet injuries (e.g., sunburns and the like), poisoning diseases (e.g., renal tubular cell injury by heavy metals, liver cell injury by alcohol, and the like), nutritional disorders (e.g., thymus atrophy due to vitamin or trace element deficiency, and the like), inflammatory diseases (e.g., acute pancreatitis, arthritis, periodontal disease, colitis and the like), ischemic neuropathy, vascular diseases (e.g., arteriosclerosis), articular diseases (e.g., arthritic deformans and the like) and the like.

On the other hand, a non-human mammal having the foreign abnormal DNA of the present invention can be propagated over generations in an ordinary rearing environment as an animal having the DNA after the stable inheritance of the foreign DNA is confirmed by mating. Furthermore, the desired foreign DNA can be used as a raw material as incorporated in the aforementioned plasmid. A DNA construct with a promoter can be prepared by an ordinary DNA engineering technique. Transfer of the abnormal DNA of the present invention in the fertilized ovum cell stage is assured so that it will be present in all germ cells and somatic cells of the subject mammal. The presence of the abnormal DNA of the present invention in germ cells of the produced animal after the DNA transfer means that all progenies of the produced animal have the foreign abnormal DNA of the present invention in all germ cells and somatic cells thereof. By acquiring a homozygous animal having the introduced DNA in both homologous chromosomes, and mating a male and female of this animal, the animal can be propagated over generations so that all progenies will have the DNA.

A non-human mammal having the exogenic abnormal DNA of the present invention has the abnormal DNA of the present invention expressed at a high level therein, possibly finally develops ASK1 function inactivation type unresponsiveness (for example, cancers (e.g., leukemia, esophageal cancer, gastric cancer, colon cancer, rectal cancer, lung cancer, liver cancer, kidney cancer, breast cancer, uterine cancer, ovarian cancer, prostatic cancer, melanoma, myeloma, osteosarcoma, brain tumor and the like), autoimmune diseases (e.g., systemic lupus erythematosus, scleroderma, rheumatoid arthritis, Sjogren's syndrome, multiple sclerosis, insulin-dependent diabetes, psoriasis, ulcerative colitis, idiopathic thrombocytopenic purpura, Crohn's disease, glomerulonephritis and the like), viral infections (e.g., hemorrhagic fever, T-cell leukemia, Kaposi sarcoma, infectious mononucleosis, lymphoma, epipharyngeal cancer, cervical cancer, skin cancer, hepatitis, liver cancer and the like), endocrine diseases (e.g., hyperhormonal disease, hypercytokine disease and the like), hematological diseases (e.g., hemocytosis, B-cell lymphoma, polycythemia and the like), organ hyperplasia (e.g., hermaphroditism, undescended testis, teratoma, nephroblastoma, polycystic kidney, cardiac/aortic malformations, syndactyly and the like) and the like) by inhibiting the function of endogenous normal DNA, and can be utilized as a pathologic model animal thereof. For example, using the abnormal DNA transgenic animal of the present invention, it is possible to elucidate the pathologic mechanism of ASK1 function inactivation type unresponsiveness, and to investigate a therapeutic method for this disease.

Regarding the feasibility of specific application, an animal that expresses the abnormal DNA of the present invention at a high level can serve as a model for elucidating the inhibition of the function of normal ASK1 by abnormal ASK1 (dominant negative action) in ASK1 function inactivation type unresponsiveness.

Also, because a mammal to which the foreign abnormal DNA of the present invention is transferred has a symptom of increase in ASK1, it can also be utilized for a screening test for a therapeutic drug for ASK1 function inactivation type unresponsiveness.

Furthermore, using the DNA transgenic animal of the present invention, it is possible to provide an effective and quick screening method for a prophylactic or therapeutic drug for a disease associated with ASK1, including ASK1 function inactivation type unresponsiveness, to develop the prophylactic or therapeutic drug using the above-described test method, quantification method and the like. It is also possible to investigate and develop a gene therapy for a disease associated with ASK1, using the DNA transgenic animal of the present invention or the foreign DNA expression vector of the present invention.

Abbreviations for bases, amino acids and the like used in the present specification and drawings are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.
- DNA:: Deoxyribonucleic acid
- cDNA:: Complementary deoxyribonucleic acid
- A:: Adenine
- T:: Thymine
- G:: Guanine
- C:: Cytosine
- RNA:: Ribonucleic acid
- mRNA:: Messenger ribonucleic acid
- dATP:: Deoxyadenosine triphosphate
- dTTP:: Deoxythymidine triphosphate
- dGTP:: Deoxyguanosine triphosphate
- dCTP:: Deoxycytidine triphosphate
- ATP:: Adenosine triphosphate
- EDT:: Ethylenediaminetetraacetic acid
- SDS:: Sodium dodecyl sulfate
- Gly:: Glycine
- Ala:: Alanine
- Val:: Valine
- Leu:: Leucine
- Ile:: Isoleucine
- Ser:: Serine
- Thr:: Threonine
- Cys:: Cysteine
- Met:: Methionine
- Glu:: Glutamic acid
- Asp:: Aspartic acid
- Lys:: Lysine
- Arg:: Arginine
- His:: Histidine
- Phe:: Phenylalanine
- Tyr:: Tyrosine
- Trp:: Tryptophan
- Pro:: Proline
- Asn:: Asparagine
- Gln:: Glutamine
- pGlu:: Pyroglutamic acid
- *:: Corresponding to stop codon.
- Me:: Methyl group
- Et:: Ethyl group
- Bu:: Butyl group
- Ph:: Phenyl group
- TC:: Thiazolidine-4(R)-carboxamide group

Substituents, protecting groups and reagents frequently mentioned herein are represented by the symbols shown below.
- Tos:: p-Toluenesulfonyl
- CHO:: Formyl
- Bzl:: Benzyl
- Cl₂Bzl:: 2, 6-Dichlorobenzyl
- Bom:: Benzyloxymethyl
- Z:: Benzyloxycarbonyl
- Cl-Z:: 2-Chlorobenzyloxycarbonyl
- Br-Z:: 2-Bromobenzyloxycarbonyl
- Boc:: t-Butoxycarbonyl
- DNP:: Dinitrophenol
- Trt:: Trityl
- Bum:: t-Butoxymethyl
- Fmoc:: N-9-Fluorenylmethoxycarbonyl
- HOBt:: 1-Hydroxybenztriazole
- HOOBt:: 3,4-Dihydro-3-hydroxy-4-oxo-1,2,3-benzotriazine
- HONB:: 1-Hydroxy-5-norbornane-2,3-dicarboximide
- DCC:: N,N'-Dicyclohexylcarbodiimide

The sequence identification numbers in the sequence listing herein show the following sequences.

### [SEQ ID NO:1]

Shows the base sequence of the amino acid-encoding region of human ASK1 cDNA.

### [SEQ ID NO:2]

### Shows the amino acid sequence of human ASK1.

The present invention is hereinafter described in more detail by means of the following Examples, which examples, however, are not to be construed as limiting the scope of the present invention.

### Example 1 Effect of ASK1 gene knockout on diabetic neuropathy in streptozocin-induced diabetic model

7-week-old male ASK1 knockout (KO) mouse (prepared according to the method described in the above-mentioned patent reference 2) and the same week-old male C57BL/6J mouse (CLEA Japan, Inc.) were used. After fasting for 24 hr, the animals were divided into two groups such that the body weight before and after the fasting and variation thereof were almost equivalent, and used as streptozocin (STZ) 125 mg/kg and 135 mg/kg administration groups. Untreated 6-week-old C57BL/6J mouse was taken as a normal control group. STZ (Sigma) was dissolved in a solvent (1:2 mixture of 0.1 M citric acid and physiological saline solution) to concentrations of 12.5 and 13.5 mg/ml immediately before administration, and intraperitoneally administered at a dose of 10 ml/kg in an ether anesthesia. At 8 and 12 weeks after STZ administration, ocular fundus blood samples were collected and plasma glucose concentration was measured using a biochemically automated analyzer (Beckman Coulter, SYNCHRON CX5Δ). The motoneuron conduction velocity of the tail was measured at 7, 10 and 13 weeks after STZ administration by Neuropack2 (NIHON KOHDEN CORPORATION). The data were collectively analyzed for the 125 mg/kg and 135 mg/kg administration groups.

The results are shown in Table 1 and Table 2. In both C57BL/6J mouse and ASK1 KO mouse, administration of STZ increased plasma glucose concentration and the level thereof was equivalent. In the STZ treated C57BL/6J mouse and STZ treated ASK1 KO mouse, at 7 weeks after STZ treatment, the tail motoneuron conduction velocity significantly decreased as compared to the normal control mouse. In the STZ treated C57BL/6J mouse, the tail motoneuron conduction velocity further decreased at 10 and 13 weeks later, but in the STZ treated ASK1 KO mouse, it shifted around the same level as the value at 7 weeks later.

**[Table 1] Glucose concentration (unit: mg/dL) of plasma of each STZ treated mouse**

| mouse | period (weeks) after STZ treatment | |
|---|---|---|
| | 8 | 12 |
| normal control mouse | 178.2 | 209.3 |
| STZ treated C57BL/6J mouse | 653.0 | 713.7 |
| STZ treated ASK1 KO mouse | 665.7 | 690.5 |

| | | |
|---|---|---|
| (n=7-9, average) | | |

**[Table 2]**

| Tail motoneuron conduction velocity (unit: m/s) of each STZ treated mouse | | | |
|---|---|---|---|
| mouse | period (weeks) after STZ treatment | | |
| | 7 | 10 | 13 |
| normal control mouse | 22.6 | 22.0 | 23.2 |
| STZ treated C57BL/6J mouse | 19.8** | 17.0** | 17.0** |
| STZ treated ASK1 KO mouse | 20.1* | 20.1 | 21.7# |

| | | | |
|---|---|---|---|
| (n=6-9, average, *:p<0.05, **:p<0.01 vs. normal mouse, #:p<0.05 vs. STZ treated C57BL/6J mouse, students t-test) | | | |

### Example 2

### Effect of ASK1 gene knockout on blood glucose increase in mouse during high-fat diet loading

5-week-old male ASK1 KO mouse and male C57BL/6J mouse of the same age were used. The body weight was measured, ocular fundus blood samples were collected, and the plasma glucose concentration (Glu) was measured using a biochemical automated analyzer (Beckman, CX5Δ). The mice were divided into two groups of a normal diet group and a high-fat diet group such that the body weight and Glu were almost equivalent. As the normal diet, CE-2 (CLEA Japan, Inc.) was used and as the high-fat diet, #D12451 (45 kcal% fat, Research Diets, New Brunswick, NJ, USA) was used. At 2, 4, 6, 8 and 10 weeks after a high-fat diet loading, the body weight was measured, blood samples were collected and Glu was measured.

The results are shown in Table 3 and Table 4. In C57BL/6J mouse, the tendency of body weight gain was observed from week 6 to week 8 after high-fat diet loading, and a significant increase in Glu was observed from week 2 to week 10. In the ASK1 KO mouse, a significant increase in the body weight was observed from week 6 to week 10 after high-fat diet loading. While a significant increase in Glu was observed from week 2 to week 6 after high-fat diet loading, it was mild as compared to that in the wild-type (C57BL/6J) mouse, where the difference disappeared at weeks 8 and 10.

**[Table 3]**

| shift of body weight (unit: g) in each animal group | | | | | | |
|---|---|---|---|---|---|---|
| treated group | experiment period (weeks) | | | | | |
| | 0 | 2 | 4 | 6 | 8 | 10 |
| C57BL/6J normal diet group | 20.7 | 23.0 | 24.5 | 26.2 | 27.2 | 28.0 |
| C57BL/6J high-fat diet group | 20.6 | 22.7 | 24.9 | 27.0 | 28.2 | 28.8 |
| ASK1 KO normal diet group | 17.9 | 20.9 | 23.0 | 24.5* | 26.2** | 26.6** |
| ASK1 KO high-fat diet group | 18.0 | 21.5 | 23.8 | 25.6 | 28.3 | 29.5 |

| | | | | | | |
|---|---|---|---|---|---|---|
| (n=10-13, average, *:p<0.05, **:p<0.01 vs normal diet group, students t-test) | | | | | | |

**[Table 4]**

| shift of plasma glucose concentration (unit: mg/dL) in each animal group | | | | | | |
|---|---|---|---|---|---|---|
| treated group | experiment period (weeks) | | | | | |
| | 0 | 2 | 4 | 6 | 8 | 10 |
| C57BL/6J normal diet group | 253.6 | 242.7 | 223.4 | 202.6 | 195.6 | 216.1 |
| C57BL/6J high-fat diet group | 269.5 | 275.9* | 303.5** | 255.7** | 241.7** | 254.1** |
| ASK1 KO normal diet group | 216.5 | 232.3 | 202.2 | 196.7 | 234.1 | 159.3 |
| ASK1 KO high-fat diet group | 208.1 | 266.1** | 221.1 | 227.5* | 224.3 | 172.5 |

| | | | | | | |
|---|---|---|---|---|---|---|
| (n=10-13, average, *:p<0.05, **:p<0.01 vs normal diet group, students t-test) | | | | | | |

### Example 3

### Increase in ASK1 expression and activity in kidney tissue of diabetic nephropathy model mouse (db/db mouse)

Male C57BL/KsJ db/db mouse (32-week-old, CLEA Japan, Inc.) with non-insulin dependent diabetes and spontaneous diabetic nephropathy and normal control animal thereof (same-week-old male C57BL/KsJ *db*/+*m* (CLEA Japan, Inc.)) were used. The mouse was sacrificed, the kidney was removed and frozen with liquid nitrogen, and the tissue was ground. 1 ml of homogenization buffer [20 mM Tris-HCl, 150 mM NaCl, 1% Triton X-100, 1% deoxycholate, 3 mM dithiothreitol, 2 mM sodium orthovanadate, 12 mM β-glycerophosphate, 50 mM NaF, protease inhibitor cocktail (Roche)] was added and the mixture was homogenized with a polytron homogenizer. After treating 5 seconds in an ultrasonic disintegrator, the homogenate was centrifuged at 15000 rpm for 15 min, and the supernatant was transferred to a different tube. The supernatant was applied to a 0.45 µm centrifugation filter to remove lipid (15000 rpm, 1 min), and the filtered solution was transferred to a new tube. Protein was quantitatively determined and adjusted with a homogenization buffer to 10 mg protein per tube. An anti-ASK1 antibody (sc-6368, Santa Cruz Biotechnology, US) was added at 2 µg/tube and, after reversal mixing at 4°C for 1 hr, 20 µL of protein-G-Sepharose (Pharmacia Biotech) was added. The mixture was subjected to reversal mixing at 4°C for 30 min to allow immunoprecipitation. After centrifugation at 15000 rpm for 1 min, the precipitate was washed (3 times), 30 µL of LDS sample buffer (50 mM DTT containing, Invitrogen) was added to the tube, and the mixture was boiled at 100°C for 5 min. Using NuPAGE 3-8% Tris-Acetate Gel (Invitrogen), samples (20 µL per lane) were electrophoresed (150 V, 60 min). The protein was transcribed on a PVDF membrane (semi-dry method) and, using block Ace (Dainippon Pharmaceutical Co. Ltd.), blocking was performed overnight at 4°C. An anti-phosphorylated ASK1 antibody (2000-fold diluted) or anti-ASK1 antibody (DAV antibody, 1000-fold diluted) was reacted overnight at 4°C, and an HRP-labeled anti-rabbit IgG antibody (anti-rabbit IgG, HRP-linked F(ab')₂ fragment, 1000-fold diluted, Amersham) was reacted at room temperature for 60 min. As a substrate, Super Signal West Maximum Sensitivity (PIERCE) was used and chemiluminescence was detected by an image analyzer (ATTO CORPORATION). As a positive control, an ASK1 high expression HEK293 cell-derived sample was used.

The results are shown in Fig. 1. In a kidney tissue of 32-week-old C57BL/KsJ db/db mouse, an increase in the ASK1 protein amount was observed as compared to the normal control animal (C57BL/KsJ *db*/+*m* mouse) (Fig. 1A; lanes 1-6). In addition, in the kidney tissue of the C57BL/KsJ db/db mouse, the amount of phosphorylated ASK1 was also increased (Fig. 1B; lanes 1-6).

### Example 4

### Effect of ASK1 gene knockout on airway inflammation in tobacco smoke exposure COPD model

A 6-week-old male ASK1 knockout (KO) mouse (prepared according to the method described in the above-mentioned patent reference 2) and a same-week-old wild-type mouse (C57BL/6J, male, CLEA Japan, Inc.) were subjected to a tobacco smoke exposure experiment. The ASK1 KO and wild-type mice were placed in an acrylic chamber, and mainstream smoke of one cigarette (tobacco brand: Kentucky Research Cigarette 1R1, tobacco mainstream smoke concentration: 16%) was injected into the chamber every 5 min to perform a tobacco smoke exposure. The tobacco smoke exposure was performed for 30 min (6 cigarettes) per day, which was continued for 3 days. As a control group, a same-week-old male mouse reared in a conventional animal room for the same period was used. After 24 hr from the final tobacco smoke exposure, alveoli washing was collected and the airway inflammation was observed. The mice were sacrified by pentobarbital anesthesia, a cannula was inserted into the airway, and the alveoli washing was collected by injection of 0.5 ml of ice-cold PBS (3 times in total). The alveoli washing was centrifuged at 3000 rpm, 4°C for 10 min. The supernatant was collected and used for the measurement (KC ELISA kit, Genzyme) of KC, which is a neutrophil chemotactic factor. The sediment after the centrifugation was resuspended in 0.1 ml of HBSS and used for the measurement of the total cell number and preparative measurement of leukocytes.

The results are shown in Table 5. In the control group reared in a conventional animal room, no significant difference was found between the wild-type mouse and the ASK1 KO mouse for any of the total cell number, macrophage cell number, neutrophil cell number and KC amount in the alveoli washing. Then, the airway inflammation reaction was compared between the wild-type mouse and the ASK1 KO mouse after tobacco smoke exposure. As a result, the total cell number was 12.0 ± 0.9 × 10⁴ cells in the wild-type mouse, which significantly decreased to 7.2 ± 0.4 × 10⁴ cells in the ASK1 KO mouse. The neutrophil number was 6.5 ± 0.7 × 10⁴ cells in the wild-type mouse, and 2.2 ± 0.4 × 10⁴ cells in the ASK1 KO mouse, showing a significant decreasing effect of 66% was observed. In contrast, no significant difference was observed in the macrophage cell number between the wild-type mouse and the ASK1 KO mouse (5.3 ± 0.2 vs 4.8 ± 0.4 × 10⁴ cells, wild-type vs ASK1 KO mouse). Moreover, the KC amount in the alveoli washing was measured. As a result, a significant decrease was found in the ASK1 KO mouse, as evidenced by 57.0 ± 4.3 pg/ml for the wild-type mouse, and 41.6 ± 2.0 pg/ml for the ASK1 KO mouse.

**[Table 5]**

| number of cells in alveoli washing and KC production amount of each animal group | | | | | |
|---|---|---|---|---|---|
| mouse | number of mice | cell number (×10⁴ cells/alveoli washing) | | | KC amount (pg/ml) |
| | | total cell | macrophage | neutrophil | |
| control group | | | | | |
| C57BL/6J mouse | 4 | 2.3±0.2 | 2.3±0.2 | 0 | 16.2±0.6 |
| ASK1 KO mouse | 6 | 2.8±0.8 | 2.8±0.8 | 0 | 16.4±0.6 |

| tobacco smoke exposure group | | | | | |
|---|---|---|---|---|---|
| C57BL/6J mouse | 6 | 12.0±0.9 | 5.3±0.2 | 6.5±0.7 | 57.0±4.3 |
| ASK1 KO mouse | 8 | 7.2±0.4** | 4.8±0.4 | 2.2±0.4** | 41.6±2.0* |

| | | | | | |
|---|---|---|---|---|---|
| (mean ± standard error,*:P<0.05, **:P<0.01 vs wild-type (C57BL/6J) mouse, students t-test) | | | | | |

### Example 5

### Increase in ASK1 expression and activity in lung tissue of tobacco smoke exposure COPD model

A 6-week-old male mouse (C57BL/6J, CLEA Japan, Inc.) was subjected to a tobacco smoke exposure experiment. The mouse was placed in an acrylic chamber, and tobacco (tobacco brand: Kentucky Research Cigarette 2R4F) mainstream smoke (3%) was flown into the chamber using an automatic tobacco smoke developing apparatus (SIBATA SCIENTIFIC TECHNOLOGY LTD.) to perform a 30 min tobacco smoke exposure. At 3, 6 and 9 hr after completion of the tobacco smoke exposure, the mice were sacrificed, and the lung was quickly frozen in liquid nitrogen. The lung tissue was homogenized in 1 ml of an ice-cold homogenate buffer (containing phosphatase inhibitor, protease inhibitor), and the protein in the supernatant after centrifugation was quantified. An anti-ASK1 antibody (N-19, Santa Cruz, 4 µg/tube) was added to a lung homogenate (5 mg protein) at 2 µg/tube, and the mixture was subjected to reversal mixing at 4°C for 1 hr. Then, 20 µL of protein-G-Sepharose (Pharmacia Biotech) was added, and the mixture was subjected to reversal mixing at 4°C for 30 min to allow immunoprecipitation. The immunoprecipitate was washed, an SDS sample buffer was added and the mixture was boiled at 100°C for 5 min. Using NuPAGE 4-12% Bis-Tris Gel (Invitrogen), samples (10 µL per lane) were electrophoresed (150 V, 90 min). The protein was transcribed on a PVDF membrane and, using block Ace (Dainippon Pharmaceutical Co. Ltd.), blocking was performed overnight at 4°C. An anti-phosphorylated ASK1 antibody (Cell Signaling) or anti-ASK1 antibody (Santa Cruz) was reacted overnight at 4°C, and an HRP-labeled anti-rabbit IgG antibody (anti-rabbit IgG, HRP-linked, Amersham) was reacted at room temperature for 60 min. As a substrate, Super Signal West Maximum Sensitivity (PIERCE) was used and chemiluminescence was detected by an image analyzer (FUJIFILM).

The results of western blotting (Fig. 2A) of phosphorylated ASK1 and ASK1 and quantification of band (Fig. 2B) are shown in Fig. 2. Phosphorylation of ASK1 was promoted by tobacco smoke exposure, and 1.8, 2.7 and 2.6 times enhancement was observed at 3, 6 and 9 hr later, respectively, as compared to the pre values. At this time, the expression level of ASK1 also increased in the same time course as the phosphorylated ASK1, where 1.8, 2.3 and 2.6 times enhancement were observed at 3, 6 and 9 hr later, respectively, as compared to the pre values. From the foregoing results, it has been clarified that, in the lung after tobacco smoke exposure stimulation, ASK1 phosphorylation (activation) is also promoted along with the increasing expression levels of ASK1 protein.

### Industrial Applicability

According to the screening method of the present invention, a compound showing a prophylactic or therapeutic effect against apoptosis or an inflammation-associated disease, diabetes or complications thereof, or chronic obstructive pulmonary disease can be quickly and conveniently selected, and the method is useful as a tool for efficacy evaluation and the like in the development of a prophylactic or therapeutic drug for the disease.

This application is based on application No. 2004-131731 (filing date: April 27, 2004) filed in Japan, the contents of which are incorporated hereinto by reference.

## Claims

1. A method for screening for a substance for the prophylaxis or treatment of apoptosis or an inflammation-associated disease, which comprises using a protein having an amino acid sequence the same as or substantially the same as the amino acid sequence shown by SEQ ID NO: 2 or a partial peptide thereof or a salt thereof.

2. A method for screening for a substance for the prophylaxis or treatment of diabetes or complications thereof, which comprises using a protein having an amino acid sequence the same as or substantially the same as the amino acid sequence shown by SEQ ID NO: 2 or a partial peptide thereof or a salt thereof.

3. A method for screening for a substance for the prophylaxis or treatment of a chronic obstructive pulmonary disease, which comprises using a protein having an amino acid sequence the same as or substantially the same as the amino acid sequence shown by SEQ ID NO: 2 or a partial peptide thereof or a salt thereof.

4. The screening method of any one of claims 1 to 3, which comprises comparing the activity of a protein having an amino acid sequence the same as or substantially the same as the amino acid sequence shown by SEQ ID NO: 2 or a partial peptide thereof or a salt thereof, between that in the presence of a test substance and that in the absence of the test substance.

5. The screening method of claim 4, which comprises culturing a cell having the ability to produce said protein or a partial peptide thereof or a salt thereof in the presence of a test substance and in the absence of the test substance, and comparing the activity of the protein or a partial peptide thereof or a salt thereof under the both conditions.

6. The screening method of any one of claims 1 to 3, which comprises using a protein having the amino acid sequence shown by SEQ ID NO: 2 or a partial peptide thereof or a salt thereof.

7. The screening method of claim 2, wherein the complication is diabetic nephropathy or diabetic neuropathy.

8. A method for screening for a substance for the prophylaxis or treatment of apoptosis or an inflammation-associated disease, which comprises comparing, between that in the presence of a test substance and that in the absence of the test substance, the expression of a protein having an amino acid sequence the same as or substantially the same as the amino acid sequence shown by SEQ ID NO: 2 or a partial peptide thereof or a salt thereof, in a cell having the ability to produce the protein or a partial peptide thereof or a salt thereof.

9. A method for screening for a substance for the prophylaxis or treatment of diabetes or complications thereof, which comprises comparing, between that in the presence of a test substance and that in the absence of the test substance, the expression of a protein having an amino acid sequence the same as or substantially the same as the amino acid sequence shown by SEQ ID NO: 2 or a partial peptide thereof or a salt thereof, in a cell having the ability to produce the protein or a partial peptide thereof or a salt thereof.

10. A method for screening for a substance for the prophylaxis or treatment of a chronic obstructive pulmonary disease, which comprises comparing, between that in the presence of a test substance and that in the absence of the test substance, the expression of a protein having an amino acid sequence the same as or substantially the same as the amino acid sequence shown by SEQ ID NO: 2 or a partial peptide thereof or a salt thereof, in a cell having the ability to produce the protein or a partial peptide thereof or a salt thereof.

11. The screening method of any one of claims 8 to 10, which comprises using a nucleic acid having a base sequence encoding a protein having an amino acid sequence the same as or substantially the same as the amino acid sequence shown by SEQ ID NO: 2 or a part thereof, or an antibody against a protein having an amino acid sequence the same as or substantially the same as the amino acid sequence shown by SEQ ID NO: 2 or a partial peptide thereof or a salt thereof.

12. The screening method of any one of claims 8 to 10, wherein the cell has an ability to produce a protein having the amino acid sequence shown by SEQ ID NO: 2 or a partial peptide thereof or a salt thereof.

13. The screening method of claim 9, wherein the complication is diabetic nephropathy or diabetic neuropathy.

14. An agent for the prophylaxis or treatment of apoptosis or an inflammation-associated disease, which comprises an antibody against a protein having an amino acid sequence the same as or substantially the same as the amino acid sequence shown by SEQ ID NO: 2 or a partial peptide thereof or a salt thereof.

15. A method for the prophylaxis or treatment of apoptosis or an inflammation-associated disease in a mammal, which comprises administering, to the mammal, an effective amount of an antibody against a protein having an amino acid sequence the same as or substantially the same as the amino acid sequence shown by SEQ ID NO: 2 or a partial peptide thereof or a salt thereof.

16. Use of an antibody against a protein having an amino acid sequence the same as or substantially the same as the amino acid sequence shown by SEQ ID NO: 2 or a partial peptide thereof or a salt thereof, for the production of an agent for the prophylaxis or treatment of apoptosis or an inflammation-associated disease.

17. An agent for the prophylaxis or treatment of diabetes or complications thereof, which comprises an antibody against a protein having an amino acid sequence the same as or substantially the same as the amino acid sequence shown by SEQ ID NO: 2 or a partial peptide thereof or a salt thereof.

18. A method for the prophylaxis or treatment of diabetes or complications thereof in a mammal, which comprises administering, to the mammal, an effective amount of an antibody against a protein having an amino acid sequence the same as or substantially the same as the amino acid sequence shown by SEQ ID NO: 2 or a partial peptide thereof or a salt thereof.

19. Use of an antibody against a protein having an amino acid sequence the same as or substantially the same as the amino acid sequence shown by SEQ ID NO: 2 or a partial peptide thereof or a salt thereof, for the production of an agent for the prophylaxis or treatment of diabetes or complications thereof.

20. The agent of claim 17, wherein the complications is diabetic nephropathy or diabetic neuropathy.

21. An agent for the prophylaxis or treatment of a chronic obstructive pulmonary disease, which comprises an antibody against a protein having an amino acid sequence the same as or substantially the same as the amino acid sequence shown by SEQ ID NO: 2 or a partial peptide thereof or a salt thereof.

22. A method for the prophylaxis or treatment of a chronic obstructive pulmonary disease in a mammal, which comprises administering, to the mammal, an effective amount of an antibody against a protein having an amino acid sequence the same as or substantially the same as the amino acid sequence shown by SEQ ID NO: 2 or a partial peptide thereof or a salt thereof.

23. Use of an antibody against a protein having an amino acid sequence the same as or substantially the same as the amino acid sequence shown by SEQ ID NO: 2 or a partial peptide thereof or a salt thereof, for the production of an agent for the prophylaxis or treatment of a chronic obstructive pulmonary disease.

24. An agent for the prophylaxis or treatment of type 2 diabetes or diabetic complications, which comprises a nucleic acid having a base sequence complementary to a base sequence encoding a protein having an amino acid sequence the same as or substantially the same as the amino acid sequence shown by SEQ ID NO: 2 or a part thereof.

25. A method for the prophylaxis or treatment of type 2 diabetes or diabetic complications in a mammal, which comprises administering, to the mammal, an effective amount of a nucleic acid having a base sequence complementary to a base sequence encoding a protein having an amino acid sequence the same as or substantially the same as the amino acid sequence shown by SEQ ID NO: 2 or a part thereof.

26. Use of a nucleic acid having a base sequence complementary to a base sequence encoding a protein having an amino acid sequence the same as or substantially the same as the amino acid sequence shown by SEQ ID NO: 2 or a part thereof, for the production of an agent for the prophylaxis or treatment of type 2 diabetes or diabetic complications.

27. The agent of claim 24, wherein the diabetic complication is diabetic nephropathy or diabetic neuropathy.

28. An agent for the prophylaxis or treatment of a chronic obstructive pulmonary disease, which comprises a nucleic acid having a base sequence complementary to a base sequence encoding a protein having an amino acid sequence the same as or substantially the same as the amino acid sequence shown by SEQ ID NO: 2 or a part thereof.

29. A method for the prophylaxis or treatment of a chronic obstructive pulmonary disease in a mammal, which comprises administering, to the mammal, an effective amount of a nucleic acid having a base sequence complementary to a base sequence encoding a protein having an amino acid sequence the same as or substantially the same as the amino acid sequence shown by SEQ ID NO: 2 or a part thereof.

30. Use of a nucleic acid having a base sequence complementary to a base sequence encoding a protein having an amino acid sequence the same as or substantially the same as the amino acid sequence shown by SEQ ID NO: 2 or a part thereof, for the production of an agent for the prophylaxis or treatment of a chronic obstructive pulmonary disease.

31. A reagent for diagnosing apoptosis or an inflammation-associated disease, which comprises an antibody against a protein having an amino acid sequence the same as or substantially the same as the amino acid sequence shown by SEQ ID NO: 2 or a partial peptide thereof or a salt thereof.

32. A method for diagnosing apoptosis or an inflammation-associated disease in a mammal, which comprises using an antibody against a protein having an amino acid sequence the same as or substantially the same as the amino acid sequence shown by SEQ ID NO: 2 or a partial peptide thereof or a salt thereof.

33. Use of an antibody against a protein having an amino acid sequence the same as or substantially the same as the amino acid sequence shown by SEQ ID NO: 2 or a partial peptide thereof or a salt thereof, for the production of a reagent for diagnosing apoptosis or an inflammation-associated disease.

34. A reagent for diagnosing diabetes or complications thereof, which comprises an antibody against a protein having an amino acid sequence the same as or substantially the same as the amino acid sequence shown by SEQ ID NO: 2 or a partial peptide thereof or a salt thereof.

35. A method for diagnosing diabetes or complications thereof in a mammal, which comprises using an antibody against a protein having an amino acid sequence the same as or substantially the same as the amino acid sequence shown by SEQ ID NO: 2 or a partial peptide thereof or a salt thereof.

36. Use of an antibody against a protein having an amino acid sequence the same as or substantially the same as the amino acid sequence shown by SEQ ID NO: 2 or a partial peptide thereof or a salt thereof, for the production of a reagent for diagnosing diabetes or complications thereof.

37. The diagnostic reagent of claim 34, wherein the complication is diabetic nephropathy or diabetic neuropathy.

38. A reagent for diagnosing a chronic obstructive pulmonary disease, which comprises an antibody against a protein having an amino acid sequence the same as or substantially the same as the amino acid sequence shown by SEQ ID NO: 2 or a partial peptide thereof or a salt thereof.

39. A method for diagnosing a chronic obstructive pulmonary disease in a mammal, which comprises using an antibody against a protein having an amino acid sequence the same as or substantially the same as the amino acid sequence shown by SEQ ID NO: 2 or a partial peptide thereof or a salt thereof.

40. Use of an antibody against a protein having an amino acid sequence the same as or substantially the same as the amino acid sequence shown by SEQ ID NO: 2 or a partial peptide thereof or a salt thereof, for the production of a reagent for diagnosing a chronic obstructive pulmonary disease.

41. A reagent for diagnosing apoptosis or an inflammation-associated disease, which comprises a nucleic acid having a base sequence encoding a protein having an amino acid sequence the same as or substantially the same as the amino acid sequence shown by SEQ ID NO: 2 or a part thereof.

42. A method for diagnosing apoptosis or an inflammation-associated disease in a mammal, which comprises administering, to the mammal, an effective amount of a nucleic acid having a base sequence encoding a protein having an amino acid sequence the same as or substantially the same as the amino acid sequence shown by SEQ ID NO: 2 or a part thereof.

43. Use of a nucleic acid having a base sequence encoding a protein having an amino acid sequence the same as or substantially the same as the amino acid sequence shown by SEQ ID NO: 2 or a part thereof, for the production of a reagent for diagnosing apoptosis or an inflammation-associated disease.

44. A reagent for diagnosing diabetes or complications thereof, which comprises a nucleic acid having a base sequence encoding a protein having an amino acid sequence the same as or substantially the same as the amino acid sequence shown by SEQ ID NO: 2 or a part thereof.

45. A method for diagnosing diabetes or complications thereof in a mammal, which comprises administering, to the mammal, an effective amount of a nucleic acid having a base sequence encoding a protein having an amino acid sequence the same as or substantially the same as the amino acid sequence shown by SEQ ID NO: 2 or a part thereof.

46. Use of a nucleic acid having a base sequence encoding a protein having an amino acid sequence the same as or substantially the same as the amino acid sequence shown by SEQ ID NO: 2 or a part thereof, for the production of a reagent for diagnosing diabetes or complications thereof.

47. The reagent of claim 44, wherein the complication is diabetic nephropathy or diabetic neuropathy.

48. A reagent for diagnosing a chronic obstructive pulmonary disease, which comprises a nucleic acid having a base sequence encoding a protein having an amino acid sequence the same as or substantially the same as the amino acid sequence shown by SEQ ID NO: 2 or a part thereof.

49. A method for diagnosing a chronic obstructive pulmonary disease in a mammal, which comprises administering, to the mammal, an effective amount of a nucleic acid having a base sequence encoding a protein having an amino acid sequence the same as or substantially the same as the amino acid sequence shown by SEQ ID NO: 2 or a part thereof.

50. Use of a nucleic acid having a base sequence encoding a protein having an amino acid sequence the same as or substantially the same as the amino acid sequence shown by SEQ ID NO: 2 or a part thereof, for the production of a reagent for diagnosing a chronic obstructive pulmonary disease.

51. An agent for the prophylaxis or treatment of type 2 diabetes or diabetic complications (excluding diabetic neuropathy), which comprises an ASK1 inhibitory substance.

52. A method for the prophylaxis or treatment of type 2 diabetes or diabetic complications (excluding diabetic neuropathy) in a mammal, which comprises administering, to the mammal, an effective amount of an ASK1 inhibitory substance.

53. Use of an ASK1 inhibitory substance for the production of an agent for the prophylaxis or treatment of type 2 diabetes or diabetic complications (excluding diabetic neuropathy).

54. The agent of claim 51, wherein the ASK1 inhibitory substance is one or more compounds selected from the group consisting of a dominant-negative mutant of ASK1, glutathione S-transferase, neph, 14-3-3, thioredoxin and HSP72.

55. The agent of claim 51, wherein the diabetic complication is diabetic nephropathy.

56. An agent for the prophylaxis or treatment of a chronic obstructive pulmonary disease, which comprises an ASK1 inhibitory substance.

57. A method for the prophylaxis or treatment of a chronic obstructive pulmonary disease, which comprises administering, to the mammal, an effective amount of an ASK1 inhibitory substance.

58. Use of an ASK1 inhibitory substance for the production of an agent for the prophylaxis or treatment of a chronic obstructive pulmonary disease.
